# EUROPEAN PATENT APPLICATION

(11) **EP 4 491 728 A1**
(43) Date of publication of application: **15.01.2025**
(21) Application number: 23766980.9
(22) Date of filing: 10.03.2023
(51) Int. Cl.: C12N 15/113, A61K 31/7088, A61K 31/7125, A61K 31/787, A61K 48/00, A61P 31/12, C07H 21/04

(54) **ANTIVIRAL ANTISENSE OLIGOMER**

(30) Priority: 10.03.2022 JP 2022037327
(71) Applicant: Nippon Shinyaku Co., Ltd., Kyoto-shi Kyoto 601-8550 (JP); National University Corporation Gunma University, Maebashi-shi, Gunma 371-8510 (JP)
(72) Inventor: NAKAGAWA, Shinichiro, Tsukuba-shi, Ibaraki 305-0003 (JP); TAGAYA, Mitsuhiro, Tsukuba-shi, Ibaraki 305-0003 (JP); HIMOTO, Takuya, Tsukuba-shi, Ibaraki 305-0003 (JP); KAMITANI, Wataru, Maebashi-shi, Gunma 371-8510 (JP)
(74) Representative: Hoffmann Eitle
(86) International application number: PCT/JP2023/009404
(87) International publication number: WO 2023/171804

(57) **Abstract**

The present specification provides an antisense oligomer, or a pharmaceutically acceptable salt thereof, or a hydrate of the antisense oligomer or the salt having a length of 15 to 30 bases, comprising a base sequence complementary to a base sequence in a target region, wherein the target region comprises a sequence of at least 10 consecutive bases in at least one region selected from the group consisting of a 5' UTR region, a nsp1 region, a nsp10 region, an RNA-dependent RNA polymerase region, an ORF10 region, and a 3' UTR region in the genome RNA of SARS-CoV-2, or a complementary sequence thereof, wherein the antisense oligomer, or the pharmaceutically acceptable salt thereof, or the hydrate of the antisense oligomer or the salt has an antiviral effect on a virus selected from the group consisting of SARS-CoV-2, SARS-CoV-1, and MERS-CoV.

## Description

### Technical Field

The present invention relates to an antisense oligomer having antiviral effects on SARS-CoV-2, SARS-CoV-1, and MERS-CoV, or a pharmaceutically acceptable salt thereof, or a hydrate of the antisense oligomer or the salt (hereinafter, also referred to as "the antisense oligomer or the like"); a pharmaceutical composition comprising the antisense oligomer or the like; or a method for treating and/or preventing a viral infectious disease, comprising a step of administering, to a subject, the antisense oligomer or the like, or the pharmaceutical composition.

### Background Art

Coronavirus disease-2019 (COVID-19) is a novel infectious disease characterized by pneumonia, which caused the WHO to declare a pandemic in March 2020 after confirmation of the first case in Wuhan City, Hubei Province of China in November 2019 (Non Patent Literature 1). The pathogen of COVID-19 is a novel virus, and the causative virus has been identified to be severe acute respiratory syndrome coronavirus 2 (SARS-CoV-2) in January 2020. SARS-CoV-2 is evolutionarily related to SARS-CoV, which is the causative virus of severe acute respiratory syndrome (SARS) that caused epidemics in 2003, and belongs to the same genus *Betacoronavirus* of the family *Coronaviridae* as SARS-CoV (Non Patent Literature 2).

### Citation List

### Non Patent Literature

Non Patent Literature 1: World Health Organization (WHO) (Press release). 11 March 2020
Non Patent Literature 2: Nat Microbiol. 2020 Apr; 5(4): 536-544

### Summary of Invention

Use of existing antiviral agents, and the like have been proposed against SARS-CoV-2, however, the effects thereof have not been clinically proven so far, and there is no established therapy. Also, there are no established therapies for SARS-CoV-1 and MERS-CoV, which belong to the same genus *Betacoronavirus* as SARS-CoV-2 and cause viral infectious disease.

Under these circumstances, a new therapeutic agent having antiviral effects on SARS-CoV-2, SARS-CoV-1, or MERS-CoV, and the like are desired to be provided.

The present invention provides an antisense oligomer targeting a specific region of a genome RNA of SARS-CoV-2, or a pharmaceutically acceptable salt thereof, or a hydrate of the antisense oligomer or the salt, and a pharmaceutical composition or the like comprising the antisense oligomer, or the pharmaceutically acceptable salt thereof, or the hydrate of the antisense oligomer or the salt as follows.
(1) An antisense oligomer, or a pharmaceutically acceptable salt thereof, or a hydrate of the antisense oligomer or the salt having a length of 15 to 30 bases, comprising a base sequence complementary to a base sequence in a target region,
   wherein the target region comprises a sequence of at least 10 consecutive bases in at least one region selected from the group consisting of a 5' UTR region, a nsp1 region, a nsp10 region, an RNA-dependent RNA polymerase region, an ORF10 region, and a 3' UTR region in the genome RNA of SARS-CoV-2, or a complementary sequence thereof,
   wherein the antisense oligomer, or the pharmaceutically acceptable salt thereof, or the hydrate of the antisense oligomer or the salt has an antiviral effect on a virus selected from the group consisting of SARS-CoV-2, SARS-CoV-1, and MERS-CoV.
(2) The antisense oligomer, or the pharmaceutically acceptable salt thereof, or the hydrate of the antisense oligomer or the salt according to (1), wherein the target region is a base sequence selected from the group consisting of positions 43 to 116, 122 to 132, 185 to 208, 242 to 279, 290 to 312, 402 to 425, 455 to 477, 13363 to 13407, 13412 to 13435, 13458 to 13547, 13578 to 13601, 29554 to 29580, 29598 to 29634, 29638 to 29648, 29652 to 29665, 29667 to 29682, 29689 to 29699, 29708 to 29731, 29744 to 29768, and 29787 to 29867 of a base sequence of SEQ ID NO: 1, or a complementary sequence thereof.
(3) The antisense oligomer, or the pharmaceutically acceptable salt thereof, or the hydrate of the antisense oligomer or the salt according to (2), wherein the target region is a base sequence selected from the group consisting of positions 44 to 67, 52 to 75, 55 to 75, 71 to 94, 93 to 116, 185 to 208, 242 to 265, 246 to 269, 250 to 273, 255 to 278, 290 to 312, 402 to 425, 455 to 477, 13363 to 13386, 13384 to 13407, 13412 to 13435, 13461 to 13484, 13466 to 13489, 13470 to 13493, 13475 to 13498, 13479 to 13502, 13488 to 13513, 13502 to 13525, 13515 to 13538, 13578 to 13601, 29554 to 29580, 29598 to 29621, 29611 to 29634, 29708 to 29731, 29744 to 29768, 29787 to 29810, 29792 to 29815, 29797 to 29820, 29817 to 29840, 29822 to 29845, 29827 to 29850, 29832 to 29855, 29837 to 29860, and 29844 to 29867 of the base sequence of SEQ ID NO: 1, or the complementary sequence thereof.
(4) The antisense oligomer, or the pharmaceutically acceptable salt thereof, or the hydrate of the antisense oligomer or the salt according to any one of (1) to (3), wherein the target region comprises a sequence of at least 15 consecutive bases in at least one region selected from the group consisting of the 5' UTR region, the nsp1 region, the nsp10 region, the RNA-dependent RNA polymerase region, the ORF10 region, and the 3' UTR region in the genome RNA of SARS-CoV-2, or the complementary sequence thereof.
(5-1) The antisense oligomer, or the pharmaceutically acceptable salt thereof, or the hydrate of the antisense oligomer or the salt according to any one of (1) to (4), comprising:
   (a) a base sequence selected from the group consisting of SEQ ID NOs: 2 to 40;
   (b) a base sequence obtained by adding, deleting, or substituting one or several bases in the base sequence selected from the group consisting of SEQ ID NOs: 2 to 40; or
   (c) a base sequence having 80% or more sequence identity with the base sequence selected from the group consisting of SEQ ID NOs: 2 to 40,
   wherein the antisense oligomer, or the pharmaceutically acceptable salt thereof, or the hydrate of the antisense oligomer or the salt inhibits a function of the target region.
(5-2) The antisense oligomer, or the pharmaceutically acceptable salt thereof, or the hydrate of the antisense oligomer or the salt according to (5-1), comprising the sequence in (a).
(6) An antisense oligomer, or a pharmaceutically acceptable salt thereof, or a hydrate of the antisense oligomer or the salt, comprising:
   a first antisense oligomer unit having a length of 8 to 20 bases, comprising a base sequence complementary to a base sequence in a first target region, wherein the first target region comprises a sequence of at least 10 consecutive bases in a first region selected from the group consisting of a 5' UTR region, a nsp1 region, a nsp10 region, an RNA-dependent RNA polymerase region, an ORF10 region, and a 3' UTR region in a genome RNA of SARS-CoV-2, or a complementary sequence thereof; and
   a second antisense oligomer unit having a length of 8 to 20 bases, comprising a base sequence complementary to a base sequence in a second target region, wherein the second target region comprises a sequence of at least 10 consecutive bases in a second region selected from the group consisting of the 5' UTR region, the nsp1 region, the nsp10 region, the RNA-dependent RNA polymerase region, the ORF10 region, and the 3' UTR region in the genome RNA of SARS-CoV-2, or a complementary sequence thereof, wherein
      (i) the difference between a position of a base sequence of SEQ ID NO: 1 at an end of the sequence of at least 10 consecutive bases in the first region, or a complementary sequence thereof, and a position of the base sequence of SEQ ID NO: 1 at an end of the sequence of at least 10 consecutive bases in the second region, or a complementary sequence thereof is 500 bases or less,
      (ii) the first and second regions are the 5' UTR and the 3' UTR regions, respectively, or the 3' UTR and the 5' UTR regions, respectively, or
      (iii) a surrounding sequence of the first region and a surrounding sequence of the second region are complementary to each other, and the surrounding sequences base-pair with each other when replicating, transcribing or translating a virus,
   wherein the antisense oligomer, or the pharmaceutically acceptable salt thereof, or the hydrate of the antisense oligomer or the salt has an antiviral effect on a virus selected from the group consisting of SARS-CoV-2, SARS-CoV-1, and MERS-CoV.
(7) The antisense oligomer, or the pharmaceutically acceptable salt thereof, or the hydrate of the antisense oligomer or the salt according to (6), wherein the sequence of at least 10 consecutive bases in the first region and the sequence of at least 10 consecutive bases in the second region are not consecutive or overlapping with each other.
(8-1) The antisense oligomer, or the pharmaceutically acceptable salt thereof, or the hydrate of the antisense oligomer or the salt according to (7), wherein the first and second target regions are each base sequences selected from the group consisting of positions 43 to 89, 98 to 110, 122 to 132, 190 to 202, 242 to 279, 290 to 312, 408 to 420, 455 to 477, 13363 to 13386, 13388 to 13401, 13418 to 13432, 13458 to 13516, 13518 to 13532, 13537 to 13547, 13582 to 13598, 29554 to 29566, 29568 to 29580, 29599 to 29613, 29615 to 29634, 29638 to 29648, 29652 to 29665, 29667 to 29682, 29689 to 29699, 29712 to 29731, 29744 to 29757, 29759 to 29768, and 29787 to 29867 of a base sequence of SEQ ID NO: 1, or complementary sequences thereof.
(8-2) The antisense oligomer, or the pharmaceutically acceptable salt thereof, or the hydrate of the antisense oligomer or the salt according to (8-1), wherein
   the first target region is a base sequence at positions 43 to 53 of the base sequence of SEQ ID NO: 1, or a complementary sequence thereof, and the second target region is a base sequence at positions 64 to 75 of the base sequence of SEQ ID NO: 1, or a complementary sequence thereof,
   the first target region is a base sequence at positions 43 to 54 of the base sequence of SEQ ID NO: 1, or a complementary sequence thereof, and the second target region is a base sequence at positions 58 to 69 of the base sequence of SEQ ID NO: 1, or a complementary sequence thereof,
   the first target region is a base sequence at positions 43 to 54 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 43 to 54 of the base sequence of SEQ ID NO: 1, or a complementary sequence thereof, and the second target region is a base sequence at positions 64 to 75 of the base sequence of SEQ ID NO: 1, or a complementary sequence thereof,
   the first target region is a base sequence at positions 44 to 54 of the base sequence of SEQ ID NO: 1, or a complementary sequence thereof, and the second target region is a base sequence at positions 64 to 75 of the base sequence of SEQ ID NO: 1, or a complementary sequence thereof,
   the first target region is a base sequence at positions 44 to 55 of the base sequence of SEQ ID NO: 1, or a complementary sequence thereof, and the second target region is a base sequence at positions 58 to 69 of the base sequence of SEQ ID NO: 1, or a complementary sequence thereof,
   the first target region is a base sequence at positions 44 to 55 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 44 to 55 of the base sequence of SEQ ID NO: 1, or a complementary sequence thereof, and the second target region is a base sequence at positions 64 to 75 of the base sequence of SEQ ID NO: 1, or a complementary sequence thereof,
   the first target region is a base sequence at positions 45 to 56 of the base sequence of SEQ ID NO: 1, or a complementary sequence thereof, and the second target region is a base sequence at positions 58 to 69 of the base sequence of SEQ ID NO: 1, or a complementary sequence thereof,
   the first target region is a base sequence at positions 45 to 56 of the base sequence of SEQ ID NO: 1, or a complementary sequence thereof, and the second target region is a base sequence at positions 59 to 70 of the base sequence of SEQ ID NO: 1, or a complementary sequence thereof,
   the first target region is a base sequence at positions 45 to 56 of the base sequence of SEQ ID NO: 1, or a complementary sequence thereof, and the second target region is a base sequence at positions 60 to 71 of the base sequence of SEQ ID NO: 1, or a complementary sequence thereof,
   the first target region is a base sequence at positions 45 to 56 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 45 to 56 of the base sequence of SEQ ID NO: 1, or a complementary sequence thereof, and the second target region is a base sequence at positions 62 to 73 of the base sequence of SEQ ID NO: 1, or a complementary sequence thereof,
   the first target region is a base sequence at positions 45 to 56 of the base sequence of SEQ ID NO: 1, or a complementary sequence thereof, and the second target region is a base sequence at positions 63 to 74 of the base sequence of SEQ ID NO: 1, or a complementary sequence thereof,
   the first target region is a base sequence at positions 45 to 56 of the base sequence of SEQ ID NO: 1, or a complementary sequence thereof, and the second target region is a base sequence at positions 64 to 75 of the base sequence of SEQ ID NO: 1, or a complementary sequence thereof,
   the first target region is a base sequence at positions 46 to 57 of the base sequence of SEQ ID NO: 1, or a complementary sequence thereof, and the second target region is a base sequence at positions 64 to 75 of the base sequence of SEQ ID NO: 1, or a complementary sequence thereof,
   the first target region is a base sequence at positions 47 to 58 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 48 to 59 of the base sequence of SEQ ID NO: 1, or a complementary sequence thereof, and the second target region is a base sequence at positions 64 to 75 of the base sequence of SEQ ID NO: 1, or a complementary sequence thereof,
   the first target region is a base sequence at positions 56 to 67 of the base sequence of SEQ ID NO: 1, or a complementary sequence thereof, and the second target region is a base sequence at positions 77 to 88 of the base sequence of SEQ ID NO: 1, or a complementary sequence thereof,
   the first target region is a base sequence at positions 56 to 67 of the base sequence of SEQ ID NO: 1, or a complementary sequence thereof, and the second target region is a base sequence at positions 98 to 109 of the base sequence of SEQ ID NO: 1, or a complementary sequence thereof,
   the first target region is a base sequence at positions 56 to 67 of the base sequence of SEQ ID NO: 1, or a complementary sequence thereof, and the second target region is a base sequence at positions 122 to 132 of the base sequence of SEQ ID NO: 1, or a complementary sequence thereof,
   the first target region is a base sequence at positions 56 to 67 of the base sequence of SEQ ID NO: 1, or a complementary sequence thereof, and the second target region is a base sequence at positions 190 to 201 of the base sequence of SEQ ID NO: 1, or a complementary sequence thereof,
   the first target region is a base sequence at positions 56 to 67 of the base sequence of SEQ ID NO: 1, or a complementary sequence thereof, and the second target region is a base sequence at positions 248 to 259 of the base sequence of SEQ ID NO: 1, or a complementary sequence thereof,
   the first target region is a base sequence at positions 58 to 69 of the base sequence of SEQ ID NO: 1, or a complementary sequence thereof, and the second target region is a base sequence at positions 98 to 109 of the base sequence of SEQ ID NO: 1, or a complementary sequence thereof,
   the first target region is a base sequence at positions 61 to 72 of the base sequence of SEQ ID NO: 1, or a complementary sequence thereof, and the second target region is a base sequence at positions 78 to 89 of the base sequence of SEQ ID NO: 1, or a complementary sequence thereof,
   the first target region is a base sequence at positions 61 to 72 of the base sequence of SEQ ID NO: 1, or a complementary sequence thereof, and the second target region is a base sequence at positions 99 to 110 of the base sequence of SEQ ID NO: 1, or a complementary sequence thereof,
   the first target region is a base sequence at positions 61 to 72 of the base sequence of SEQ ID NO: 1, or a complementary sequence thereof, and the second target region is a base sequence at positions 122 to 132 of the base sequence of SEQ ID NO: 1, or a complementary sequence thereof,
   the first target region is a base sequence at positions 61 to 72 of the base sequence of SEQ ID NO: 1, or a complementary sequence thereof, and the second target region is a base sequence at positions 191 to 202 of the base sequence of SEQ ID NO: 1, or a complementary sequence thereof,
   the first target region is a base sequence at positions 61 to 72 of the base sequence of SEQ ID NO: 1, or a complementary sequence thereof, and the second target region is a base sequence at positions 249 to 260 of the base sequence of SEQ ID NO: 1, or a complementary sequence thereof,
   the first target region is a base sequence at positions 75 to 64 of the base sequence of SEQ ID NO: 1, or a complementary sequence thereof, and the second target region is a base sequence at positions 54 to 43 of the base sequence of SEQ ID NO: 1, or a complementary sequence thereof,
   the first target region is a base sequence at positions 43 to 54 of the base sequence of SEQ ID NO: 1, or a complementary sequence thereof, and the second target region is a base sequence at positions 290 to 301 of the base sequence of SEQ ID NO: 1, or a complementary sequence thereof,
   the first target region is a base sequence at positions 43 to 54 of the base sequence of SEQ ID NO: 1, or a complementary sequence thereof, and the second target region is a base sequence at positions 301 to 312 of the base sequence of SEQ ID NO: 1, or a complementary sequence thereof,
   the first target region is a base sequence at positions 45 to 56 of the base sequence of SEQ ID NO: 1, or a complementary sequence thereof, and the second target region is a base sequence at positions 290 to 301 of the base sequence of SEQ ID NO: 1, or a complementary sequence thereof,
   the first target region is a base sequence at positions 45 to 56 of the base sequence of SEQ ID NO: 1, or a complementary sequence thereof, and the second target region is a base sequence at positions 301 to 312 of the base sequence of SEQ ID NO: 1, or a complementary sequence thereof,
   the first target region is a base sequence at positions 47 to 58 of the base sequence of SEQ ID NO: 1, or a complementary sequence thereof, and the second target region is a base sequence at positions 301 to 312 of the base sequence of SEQ ID NO: 1, or a complementary sequence thereof,
   the first target region is a base sequence at positions 52 to 63 of the base sequence of SEQ ID NO: 1, or a complementary sequence thereof, and the second target region is a base sequence at positions 290 to 301 of the base sequence of SEQ ID NO: 1, or a complementary sequence thereof,
   the first target region is a base sequence at positions 52 to 63 of the base sequence of SEQ ID NO: 1, or a complementary sequence thereof, and the second target region is a base sequence at positions 301 to 312 of the base sequence of SEQ ID NO: 1, or a complementary sequence thereof,
   the first target region is a base sequence at positions 56 to 67 of the base sequence of SEQ ID NO: 1, or a complementary sequence thereof, and the second target region is a base sequence at positions 290 to 301 of the base sequence of SEQ ID NO: 1, or a complementary sequence thereof,
   the first target region is a base sequence at positions 56 to 67 of the base sequence of SEQ ID NO: 1, or a complementary sequence thereof, and the second target region is a base sequence at positions 301 to 312 of the base sequence of SEQ ID NO: 1, or a complementary sequence thereof,
   the first target region is a base sequence at positions 57 to 68 of the base sequence of SEQ ID NO: 1, or a complementary sequence thereof, and the second target region is a base sequence at positions 301 to 312 of the base sequence of SEQ ID NO: 1, or a complementary sequence thereof,
   the first target region is a base sequence at positions 58 to 69 of the base sequence of SEQ ID NO: 1, or a complementary sequence thereof, and the second target region is a base sequence at positions 290 to 301 of the base sequence of SEQ ID NO: 1, or a complementary sequence thereof,
   the first target region is a base sequence at positions 58 to 69 of the base sequence of SEQ ID NO: 1, or a complementary sequence thereof, and the second target region is a base sequence at positions 300 to 311 of the base sequence of SEQ ID NO: 1, or a complementary sequence thereof,
   the first target region is a base sequence at positions 58 to 69 of the base sequence of SEQ ID NO: 1, or a complementary sequence thereof, and the second target region is a base sequence at positions 301 to 312 of the base sequence of SEQ ID NO: 1, or a complementary sequence thereof,
   the first target region is a base sequence at positions 60 to 71 of the base sequence of SEQ ID NO: 1, or a complementary sequence thereof, and the second target region is a base sequence at positions 301 to 312 of the base sequence of SEQ ID NO: 1, or a complementary sequence thereof,
   the first target region is a base sequence at positions 61 to 72 of the base sequence of SEQ ID NO: 1, or a complementary sequence thereof, and the second target region is a base sequence at positions 260 to 271 of the base sequence of SEQ ID NO: 1, or a complementary sequence thereof,
   the first target region is a base sequence at positions 61 to 72 of the base sequence of SEQ ID NO: 1, or a complementary sequence thereof, and the second target region is a base sequence at positions 290 to 301 of the base sequence of SEQ ID NO: 1, or a complementary sequence thereof,
   the first target region is a base sequence at positions 61 to 72 of the base sequence of SEQ ID NO: 1, or a complementary sequence thereof, and the second target region is a base sequence at positions 301 to 312 of the base sequence of SEQ ID NO: 1, or a complementary sequence thereof,
   the first target region is a base sequence at positions 63 to 74 of the base sequence of SEQ ID NO: 1, or a complementary sequence thereof, and the second target region is a base sequence at positions 290 to 301 of the base sequence of SEQ ID NO: 1, or a complementary sequence thereof,
   the first target region is a base sequence at positions 64 to 75 of the base sequence of SEQ ID NO: 1, or a complementary sequence thereof, and the second target region is a base sequence at positions 290 to 301 of the base sequence of SEQ ID NO: 1, or a complementary sequence thereof,
   the first target region is a base sequence at positions 64 to 75 of the base sequence of SEQ ID NO: 1, or a complementary sequence thereof, and the second target region is a base sequence at positions 301 to 312 of the base sequence of SEQ ID NO: 1, or a complementary sequence thereof,
   the first target region is a base sequence at positions 65 to 76 of the base sequence of SEQ ID NO: 1, or a complementary sequence thereof, and the second target region is a base sequence at positions 290 to 301 of the base sequence of SEQ ID NO: 1, or a complementary sequence thereof,
   the first target region is a base sequence at positions 43 to 54 of the base sequence of SEQ ID NO: 1, or a complementary sequence thereof, and the second target region is a base sequence at positions 29822 to 29833 of the base sequence of SEQ ID NO: 1, or a complementary sequence thereof,
   the first target region is a base sequence at positions 43 to 54 of the base sequence of SEQ ID NO: 1, or a complementary sequence thereof, and the second target region is a base sequence at positions 29843 to 29854 of the base sequence of SEQ ID NO: 1, or a complementary sequence thereof,
   the first target region is a base sequence at positions 43 to 54 of the base sequence of SEQ ID NO: 1, or a complementary sequence thereof, and the second target region is a base sequence at positions 29856 to 29867 of the base sequence of SEQ ID NO: 1, or a complementary sequence thereof,
   the first target region is a base sequence at positions 55 to 66 of the base sequence of SEQ ID NO: 1, or a complementary sequence thereof, and the second target region is a base sequence at positions 29822 to 29833 of the base sequence of SEQ ID NO: 1, or a complementary sequence thereof,
   the first target region is a base sequence at positions 55 to 66 of the base sequence of SEQ ID NO: 1, or a complementary sequence thereof, and the second target region is a base sequence at positions 29843 to 29854 of the base sequence of SEQ ID NO: 1, or a complementary sequence thereof,
   the first target region is a base sequence at positions 55 to 66 of the base sequence of SEQ ID NO: 1, or a complementary sequence thereof, and the second target region is a base sequence at positions 29856 to 29867 of the base sequence of SEQ ID NO: 1, or a complementary sequence thereof,
   the first target region is a base sequence at positions 64 to 75 of the base sequence of SEQ ID NO: 1, or a complementary sequence thereof, and the second target region is a base sequence at positions 29720 to 29731 of the base sequence of SEQ ID NO: 1, or a complementary sequence thereof,
   the first target region is a base sequence at positions 64 to 75 of the base sequence of SEQ ID NO: 1, or a complementary sequence thereof, and the second target region is a base sequence at positions 29744 to 29757 of the base sequence of SEQ ID NO: 1, or a complementary sequence thereof,
   the first target region is a base sequence at positions 64 to 75 of the base sequence of SEQ ID NO: 1, or a complementary sequence thereof, and the second target region is a base sequence at positions 29787 to 29798 of the base sequence of SEQ ID NO: 1, or a complementary sequence thereof,
   the first target region is a base sequence at positions 64 to 75 of the base sequence of SEQ ID NO: 1, or a complementary sequence thereof, and the second target region is a base sequence at positions 29799 to 29810 of the base sequence of SEQ ID NO: 1, or a complementary sequence thereof,
   the first target region is a base sequence at positions 64 to 75 of the base sequence of SEQ ID NO: 1, or a complementary sequence thereof, and the second target region is a base sequence at positions 29822 to 29833 of the base sequence of SEQ ID NO: 1, or a complementary sequence thereof,
   the first target region is a base sequence at positions 64 to 75 of the base sequence of SEQ ID NO: 1, or a complementary sequence thereof, and the second target region is a base sequence at positions 29843 to 29854 of the base sequence of SEQ ID NO: 1, or a complementary sequence thereof,
   the first target region is a base sequence at positions 64 to 75 of the base sequence of SEQ ID NO: 1, or a complementary sequence thereof, and the second target region is a base sequence at positions 29856 to 29867 of the base sequence of SEQ ID NO: 1, or a complementary sequence thereof,
   the first target region is a base sequence at positions 75 to 64 of the base sequence of SEQ ID NO: 1, or a complementary sequence thereof, and the second target region is a base sequence at positions 29757 to 29744 of the base sequence of SEQ ID NO: 1, or a complementary sequence thereof,
   the first target region is a base sequence at positions 75 to 64 of the base sequence of SEQ ID NO: 1, or a complementary sequence thereof, and the second target region is a base sequence at positions 29833 to 29822 of the base sequence of SEQ ID NO: 1, or a complementary sequence thereof,
   the first target region is a base sequence at positions 75 to 64 of the base sequence of SEQ ID NO: 1, or a complementary sequence thereof, and the second target region is a base sequence at positions 29854 to 29843 of the base sequence of SEQ ID NO: 1, or a complementary sequence thereof,
   the first target region is a base sequence at positions 99 to 110 of the base sequence of SEQ ID NO: 1, or a complementary sequence thereof, and the second target region is a base sequence at positions 29856 to 29867 of the base sequence of SEQ ID NO: 1, or a complementary sequence thereof,
   the first target region is a base sequence at positions 13390 to 13401 of the base sequence of SEQ ID NO: 1, or a complementary sequence thereof, and the second target region is a base sequence at positions 13466 to 13477 of the base sequence of SEQ ID NO: 1, or a complementary sequence thereof,
   the first target region is a base sequence at positions 13390 to 13401 of the base sequence of SEQ ID NO: 1, or a complementary sequence thereof, and the second target region is a base sequence at positions 13478 to 13489 of the base sequence of SEQ ID NO: 1, or a complementary sequence thereof,
   the first target region is a base sequence at positions 13390 to 13401 of the base sequence of SEQ ID NO: 1, or a complementary sequence thereof, and the second target region is a base sequence at positions 13504 to 13516 of the base sequence of SEQ ID NO: 1, or a complementary sequence thereof,
   the first target region is a base sequence at positions 13474 to 13485 of the base sequence of SEQ ID NO: 1, or a complementary sequence thereof, and the second target region is a base sequence at positions 13488 to 13499 of the base sequence of SEQ ID NO: 1, or a complementary sequence thereof,
   the first target region is a base sequence at positions 13474 to 13485 of the base sequence of SEQ ID NO: 1, or a complementary sequence thereof, and the second target region is a base sequence at positions 13494 to 13505 of the base sequence of SEQ ID NO: 1, or a complementary sequence thereof,
   the first target region is a base sequence at positions 13474 to 13485 of the base sequence of SEQ ID NO: 1, or a complementary sequence thereof, and the second target region is a base sequence at positions 13503 to 13514 of the base sequence of SEQ ID NO: 1, or a complementary sequence thereof,
   the first target region is a base sequence at positions 13474 to 13485 of the base sequence of SEQ ID NO: 1, or a complementary sequence thereof, and the second target region is a base sequence at positions 13520 to 13531 of the base sequence of SEQ ID NO: 1, or a complementary sequence thereof,
   the first target region is a base sequence at positions 13474 to 13485 of the base sequence of SEQ ID NO: 1, or a complementary sequence thereof, and the second target region is a base sequence at positions 13537 to 13547 of the base sequence of SEQ ID NO: 1, or a complementary sequence thereof,
   the first target region is a base sequence at positions 13488 to 13499 of the base sequence of SEQ ID NO: 1, or a complementary sequence thereof, and the second target region is a base sequence at positions 13502 to 13513 of the base sequence of SEQ ID NO: 1, or a complementary sequence thereof,
   the first target region is a base sequence at positions 13488 to 13499 of the base sequence of SEQ ID NO: 1, or a complementary sequence thereof, and the second target region is a base sequence at positions 13503 to 13514 of the base sequence of SEQ ID NO: 1, or a complementary sequence thereof,
   the first target region is a base sequence at positions 13488 to 13499 of the base sequence of SEQ ID NO: 1, or a complementary sequence thereof, and the second target region is a base sequence at positions 13520 to 13531 of the base sequence of SEQ ID NO: 1, or a complementary sequence thereof,
   the first target region is a base sequence at positions 13488 to 13499 of the base sequence of SEQ ID NO: 1, or a complementary sequence thereof, and the second target region is a base sequence at positions 13537 to 13547 of the base sequence of SEQ ID NO: 1, or a complementary sequence thereof,
   the first target region is a base sequence at positions 13491 to 13502 of the base sequence of SEQ ID NO: 1, or a complementary sequence thereof, and the second target region is a base sequence at positions 13537 to 13547 of the base sequence of SEQ ID NO: 1, or a complementary sequence thereof,
   the first target region is a base sequence at positions 13492 to 13503 of the base sequence of SEQ ID NO: 1, or a complementary sequence thereof, and the second target region is a base sequence at positions 13537 to 13547 of the base sequence of SEQ ID NO: 1, or a complementary sequence thereof,
   the first target region is a base sequence at positions 13493 to 13504 of the base sequence of SEQ ID NO: 1, or a complementary sequence thereof, and the second target region is a base sequence at positions 13537 to 13547 of the base sequence of SEQ ID NO: 1, or a complementary sequence thereof,
   the first target region is a base sequence at positions 13494 to 13505 of the base sequence of SEQ ID NO: 1, or a complementary sequence thereof, and the second target region is a base sequence at positions 13520 to 13531 of the base sequence of SEQ ID NO: 1, or a complementary sequence thereof,
   the first target region is a base sequence at positions 13494 to 13505 of the base sequence of SEQ ID NO: 1, or a complementary sequence thereof, and the second target region is a base sequence at positions 13537 to 13547 of the base sequence of SEQ ID NO: 1, or a complementary sequence thereof,
   the first target region is a base sequence at positions 13495 to 13506 of the base sequence of SEQ ID NO: 1, or a complementary sequence thereof, and the second target region is a base sequence at positions 13537 to 13547 of the base sequence of SEQ ID NO: 1, or a complementary sequence thereof,
   the first target region is a base sequence at positions 13496 to 13507 of the base sequence of SEQ ID NO: 1, or a complementary sequence thereof, and the second target region is a base sequence at positions 13537 to 13547 of the base sequence of SEQ ID NO: 1, or a complementary sequence thereof,
   the first target region is a base sequence at positions 13497 to 13508 of the base sequence of SEQ ID NO: 1, or a complementary sequence thereof, and the second target region is a base sequence at positions 13537 to 13547 of the base sequence of SEQ ID NO: 1, or a complementary sequence thereof,
   the first target region is a base sequence at positions 13499 to 13510 of the base sequence of SEQ ID NO: 1, or a complementary sequence thereof, and the second target region is a base sequence at positions 13537 to 13547 of the base sequence of SEQ ID NO: 1, or a complementary sequence thereof,
   the first target region is a base sequence at positions 13501 to 13512 of the base sequence of SEQ ID NO: 1, or a complementary sequence thereof, and the second target region is a base sequence at positions 13537 to 13547 of the base sequence of SEQ ID NO: 1, or a complementary sequence thereof,
   the first target region is a base sequence at positions 13503 to 13514 of the base sequence of SEQ ID NO: 1, or a complementary sequence thereof, and the second target region is a base sequence at positions 13520 to 13531 of the base sequence of SEQ ID NO: 1, or a complementary sequence thereof,
   the first target region is a base sequence at positions 13503 to 13514 of the base sequence of SEQ ID NO: 1, or a complementary sequence thereof, and the second target region is a base sequence at positions 13537 to 13547 of the base sequence of SEQ ID NO: 1, or a complementary sequence thereof,
   the first target region is a base sequence at positions 13520 to 13531 of the base sequence of SEQ ID NO: 1, or a complementary sequence thereof, and the second target region is a base sequence at positions 13537 to 13547 of the base sequence of SEQ ID NO: 1, or a complementary sequence thereof,
   the first target region is a base sequence at positions 29671 to 29682 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 29689 to 29699 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 29689 to 29699 of the base sequence of SEQ ID NO: 1, or a complementary sequence thereof, and the second target region is a base sequence at positions 64 to 75 of the base sequence of SEQ ID NO: 1, or a complementary sequence thereof,
   the first target region is a base sequence at positions 29712 to 29723 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 29720 to 29731 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 29744 to 29755 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 29744 to 29757 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 29744 to 29757 of the base sequence of SEQ ID NO: 1, or a complementary sequence thereof, and the second target region is a base sequence at positions 64 to 75 of the base sequence of SEQ ID NO: 1, or a complementary sequence thereof,
   the first target region is a base sequence at positions 29744 to 29757 of the base sequence of SEQ ID NO: 1, or a complementary sequence thereof, and the second target region is a base sequence at positions 99 to 110 of the base sequence of SEQ ID NO: 1, or a complementary sequence thereof,
   the first target region is a base sequence at positions 29745 to 29756 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 29746 to 29757 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 29746 to 29757 of the base sequence of SEQ ID NO: 1, or a complementary sequence thereof, and the second target region is a base sequence at positions 64 to 75 of the base sequence of SEQ ID NO: 1, or a complementary sequence thereof,
   the first target region is a base sequence at positions 29757 to 29744 of the base sequence of SEQ ID NO: 1, or a complementary sequence thereof, and the second target region is a base sequence at positions 75 to 64 of the base sequence of SEQ ID NO: 1, or a complementary sequence thereof,
   the first target region is a base sequence at positions 29789 to 29800 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 29803 to 29814 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 29804 to 29815 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 29805 to 29816 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 29806 to 29817 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 29807 to 29818 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 29808 to 29819 of the base sequence of SEQ ID NO: 1, or a complementary sequence thereof, and the second target region is a base sequence at positions 59 to 70 of the base sequence of SEQ ID NO: 1, or a complementary sequence thereof,
   the first target region is a base sequence at positions 29808 to 29819 of the base sequence of SEQ ID NO: 1, or a complementary sequence thereof, and the second target region is a base sequence at positions 60 to 71 of the base sequence of SEQ ID NO: 1, or a complementary sequence thereof,
   the first target region is a base sequence at positions 29808 to 29819 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 29809 to 29820 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 29810 to 29821 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 29822 to 29833 of the base sequence of SEQ ID NO: 1, or a complementary sequence thereof, and the second target region is a base sequence at positions 64 to 75 of the base sequence of SEQ ID NO: 1, or a complementary sequence thereof,
   the first target region is a base sequence at positions 29822 to 29833 of the base sequence of SEQ ID NO: 1, or a complementary sequence thereof, and the second target region is a base sequence at positions 99 to 110 of the base sequence of SEQ ID NO: 1, or a complementary sequence thereof,
   the first target region is a base sequence at positions 29823 to 29834 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 29833 to 29822 of the base sequence of SEQ ID NO: 1, or a complementary sequence thereof, and the second target region is a base sequence at positions 75 to 64 of the base sequence of SEQ ID NO: 1, or a complementary sequence thereof,
   the first target region is a base sequence at positions 29843 to 29854 of the base sequence of SEQ ID NO: 1, or a complementary sequence thereof, and the second target region is a base sequence at positions 64 to 75 of the base sequence of SEQ ID NO: 1, or a complementary sequence thereof,
   the first target region is a base sequence at positions 29854 to 29843 of the base sequence of SEQ ID NO: 1, or a complementary sequence thereof, and the second target region is a base sequence at positions 75 to 64 of the base sequence of SEQ ID NO: 1, or a complementary sequence thereof,
   the first target region is a base sequence at positions 29856 to 29867 of the base sequence of SEQ ID NO: 1, or a complementary sequence thereof, and the second target region is a base sequence at positions 78 to 89 of the base sequence of SEQ ID NO: 1, or a complementary sequence thereof,
   the first target region is a base sequence at positions 29856 to 29867 of the base sequence of SEQ ID NO: 1, or a complementary sequence thereof, and the second target region is a base sequence at positions 99 to 110 of the base sequence of SEQ ID NO: 1, or a complementary sequence thereof,
   the first target region is a base sequence at positions 29856 to 29867 of the base sequence of SEQ ID NO: 1, or a complementary sequence thereof, and the second target region is a base sequence at positions 122 to 132 of the base sequence of SEQ ID NO: 1, or a complementary sequence thereof,
   the first target region is a base sequence at positions 29638 to 29648 of the base sequence of SEQ ID NO: 1, or a complementary sequence thereof, and the second target region is a base sequence at positions 29652 to 29665 of the base sequence of SEQ ID NO: 1, or a complementary sequence thereof,
   the first target region is a base sequence at positions 29667 to 29682 of the base sequence of SEQ ID NO: 1, or a complementary sequence thereof, and the second target region is a base sequence at positions 29689 to 29699 of the base sequence of SEQ ID NO: 1, or a complementary sequence thereof,
   the first target region is a base sequence at positions 29712 to 29723 of the base sequence of SEQ ID NO: 1, or a complementary sequence thereof, and the second target region is a base sequence at positions 29744 to 29757 of the base sequence of SEQ ID NO: 1, or a complementary sequence thereof,
   the first target region is a base sequence at positions 29720 to 29731 of the base sequence of SEQ ID NO: 1, or a complementary sequence thereof, and the second target region is a base sequence at positions 29744 to 29757 of the base sequence of SEQ ID NO: 1, or a complementary sequence thereof,
   the first target region is a base sequence at positions 29744 to 29757 of the base sequence of SEQ ID NO: 1, or a complementary sequence thereof, and the second target region is a base sequence at positions 29822 to 29833 of the base sequence of SEQ ID NO: 1, or a complementary sequence thereof,
   the first target region is a base sequence at positions 29744 to 29757 of the base sequence of SEQ ID NO: 1, or a complementary sequence thereof, and the second target region is a base sequence at positions 29843 to 29854 of the base sequence of SEQ ID NO: 1, or a complementary sequence thereof, or
   the first target region is a base sequence at positions 29744 to 29757 of the base sequence of SEQ ID NO: 1, or a complementary sequence thereof, and the second target region is a base sequence at positions 29856 to 29867 of the base sequence of SEQ ID NO: 1, or a complementary sequence thereof (note that, as used herein, the description "positions a to b" for the target region means, without limitation, that a (+) strand of the genome RNA of SARS-CoV-2 can be targeted when a > b, and a (-) strand of the genome RNA of SARS-CoV-2 can be targeted when a < b).
(8-3) The antisense oligomer, or the pharmaceutically acceptable salt thereof, or the hydrate of the antisense oligomer or the salt according to (8-2), wherein
   the first target region is a base sequence at positions 45 to 56 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 43 to 54 of the base sequence of SEQ ID NO: 1, or a complementary sequence thereof, and the second target region is a base sequence at positions 64 to 75 of the base sequence of SEQ ID NO: 1, or a complementary sequence thereof,
   the first target region is a base sequence at positions 13493 to 13504 of the base sequence of SEQ ID NO: 1, or a complementary sequence thereof, and the second target region is a base sequence at positions 13537 to 13547 of the base sequence of SEQ ID NO: 1, or a complementary sequence thereof,
   the first target region is a base sequence at positions 29808 to 29819 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 45 to 56 of the base sequence of SEQ ID NO: 1, or a complementary sequence thereof, and the second target region is a base sequence at positions 62 to 73 of the base sequence of SEQ ID NO: 1, or a complementary sequence thereof,
   the first target region is a base sequence at positions 29689 to 29699 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 29744 to 29757 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 47 to 58 of the base sequence of SEQ ID NO: 1, or a complementary sequence thereof, and the second target region is a base sequence at positions 61 to 72 of the base sequence of SEQ ID NO: 1, or a complementary sequence thereof,
   the first target region is a base sequence at positions 45 to 56 of the base sequence of SEQ ID NO: 1, or a complementary sequence thereof, and the second target region is a base sequence at positions 60 to 71 of the base sequence of SEQ ID NO: 1, or a complementary sequence thereof,
   the first target region is a base sequence at positions 45 to 56 of the base sequence of SEQ ID NO: 1, or a complementary sequence thereof, and the second target region is a base sequence at positions 64 to 75 of the base sequence of SEQ ID NO: 1, or a complementary sequence thereof,
   the first target region is a base sequence at positions 48 to 59 of the base sequence of SEQ ID NO: 1, or a complementary sequence thereof, and the second target region is a base sequence at positions 64 to 75 of the base sequence of SEQ ID NO: 1, or a complementary sequence thereof,
   the first target region is a base sequence at positions 43 to 53 of the base sequence of SEQ ID NO: 1, or a complementary sequence thereof, and the second target region is a base sequence at positions 64 to 75 of the base sequence of SEQ ID NO: 1, or a complementary sequence thereof,
   the first target region is a base sequence at positions 44 to 54 of the base sequence of SEQ ID NO: 1, or a complementary sequence thereof, and the second target region is a base sequence at positions 64 to 75 of the base sequence of SEQ ID NO: 1, or a complementary sequence thereof,
   the first target region is a base sequence at positions 44 to 55 of the base sequence of SEQ ID NO: 1, or a complementary sequence thereof, and the second target region is a base sequence at positions 64 to 75 of the base sequence of SEQ ID NO: 1, or a complementary sequence thereof,
   the first target region is a base sequence at positions 13494 to 13505 of the base sequence of SEQ ID NO: 1, or a complementary sequence thereof, and the second target region is a base sequence at positions 13537 to 13547 of the base sequence of SEQ ID NO: 1, or a complementary sequence thereof,
   the first target region is a base sequence at positions 13495 to 13506 of the base sequence of SEQ ID NO: 1, or a complementary sequence thereof, and the second target region is a base sequence at positions 13537 to 13547 of the base sequence of SEQ ID NO: 1, or a complementary sequence thereof,
   the first target region is a base sequence at positions 58 to 69 of the base sequence of SEQ ID NO: 1, or a complementary sequence thereof, and the second target region is a base sequence at positions 301 to 312 of the base sequence of SEQ ID NO: 1, or a complementary sequence thereof,
   the first target region is a base sequence at positions 57 to 68 of the base sequence of SEQ ID NO: 1, or a complementary sequence thereof, and the second target region is a base sequence at positions 301 to 312 of the base sequence of SEQ ID NO: 1, or a complementary sequence thereof, or
   the first target region is a base sequence at positions 64 to 75 of the base sequence of SEQ ID NO: 1, or a complementary sequence thereof, and the second target region is a base sequence at positions 290 to 301 of the base sequence of SEQ ID NO: 1, or a complementary sequence thereof.
(9) The antisense oligomer, or the pharmaceutically acceptable salt thereof, or the hydrate of the antisense oligomer or the salt according to any one of (8-1) to (8-3), comprising:
   (a) a base sequence selected from the group consisting of SEQ ID NOs: 41 to 173;
   (b) a base sequence obtained by adding, deleting, or substituting one or several bases in the base sequence selected from the group consisting of SEQ ID NOs: 41 to 173; or
   (c) a base sequence having 80% or more sequence identity with the base sequence selected from the group consisting of SEQ ID NOs: 41 to 173,
   wherein the antisense oligomer, or the pharmaceutically acceptable salt thereof, or the hydrate of the antisense oligomer or the salt inhibits a function of the first region and/or the second region.
(10-1) The antisense oligomer, or the pharmaceutically acceptable salt thereof, or the hydrate of the antisense oligomer or the salt according to (9), comprising:
   (a) a base sequence selected from the group consisting of SEQ ID NO: 41, SEQ ID NO: 44, SEQ ID NO: 45, SEQ ID NO: 48, SEQ ID NO: 51, SEQ ID NO: 52, SEQ ID NO: 53, SEQ ID NO: 55, SEQ ID NO: 57, SEQ ID NO: 58, SEQ ID NO: 80, SEQ ID NO: 83, SEQ ID NO: 89, SEQ ID NO: 123, SEQ ID NO: 125, SEQ ID NO: 126, SEQ ID NO: 135, SEQ ID NO: 140, and SEQ ID NO: 155;
   (b) a base sequence obtained by adding, deleting, or substituting one or several bases in the base sequence selected from the group consisting of SEQ ID NO: 41, SEQ ID NO: 44, SEQ ID NO: 45, SEQ ID NO: 48, SEQ ID NO: 51, SEQ ID NO: 52, SEQ ID NO: 53, SEQ ID NO: 55, SEQ ID NO: 57, SEQ ID NO: 58, SEQ ID NO: 80, SEQ ID NO: 83, SEQ ID NO: 89, SEQ ID NO: 123, SEQ ID NO: 125, SEQ ID NO: 126, SEQ ID NO: 135, SEQ ID NO: 140, and SEQ ID NO: 155; or
   (c) a base sequence having 80% or more sequence identity with the base sequence selected from the group consisting of SEQ ID NO: 41, SEQ ID NO: 44, SEQ ID NO: 45, SEQ ID NO: 48, SEQ ID NO: 51, SEQ ID NO: 52, SEQ ID NO: 53, SEQ ID NO: 55, SEQ ID NO: 57, SEQ ID NO: 58, SEQ ID NO: 80, SEQ ID NO: 83, SEQ ID NO: 89, SEQ ID NO: 123, SEQ ID NO: 125, SEQ ID NO: 126, SEQ ID NO: 135, SEQ ID NO: 140, and SEQ ID NO: 155.
(10-2) The antisense oligomer, or the pharmaceutically acceptable salt thereof, or the hydrate of the antisense oligomer or the salt according to (10-1), comprising:
   (a) a base sequence selected from the group consisting of SEQ ID NO: 57, SEQ ID NO: 58, SEQ ID NO: 126, and SEQ ID NO: 155;
   (b) a base sequence obtained by adding, deleting, or substituting one or several bases in the base sequence selected from the group consisting of SEQ ID NO: 57, SEQ ID NO: 58, SEQ ID NO: 126, and SEQ ID NO: 155; or
   (c) a base sequence having 80% or more sequence identity with the base sequence selected from the group consisting of SEQ ID NO: 57, SEQ ID NO: 58, SEQ ID NO: 126, and SEQ ID NO: 155.
(10-3) The antisense oligomer, or the pharmaceutically acceptable salt thereof, or the hydrate of the antisense oligomer or the salt according to (10-1), comprising:
   (a) a base sequence selected from the group consisting of SEQ ID NO: 52, SEQ ID NO: 123, SEQ ID NO: 53, SEQ ID NO: 135, and SEQ ID NO: 155;
   (b) a base sequence obtained by adding, deleting, or substituting one or several bases in the base sequence selected from the group consisting of SEQ ID NO: 52, SEQ ID NO: 123, SEQ ID NO: 53, SEQ ID NO: 135, and SEQ ID NO: 155; or
   (c) a base sequence having 80% or more sequence identity with the base sequence selected from the group consisting of SEQ ID NO: 52, SEQ ID NO: 123, SEQ ID NO: 53, SEQ ID NO: 135, and SEQ ID NO: 155.
(10-4) The antisense oligomer, or the pharmaceutically acceptable salt thereof, or the hydrate of the antisense oligomer or the salt according to (10-3), comprising:
   (a) a base sequence selected from the group consisting of SEQ ID NO: 52, SEQ ID NO: 123, SEQ ID NO: 53, and SEQ ID NO: 135;
   (b) a base sequence obtained by adding, deleting, or substituting one or several bases in the base sequence selected from the group consisting of SEQ ID NO: 52, SEQ ID NO: 123, SEQ ID NO: 53, and SEQ ID NO: 135; or
   (c) a base sequence having 80% or more sequence identity with the base sequence selected from the group consisting of SEQ ID NO: 52, SEQ ID NO: 123, SEQ ID NO: 53, and SEQ ID NO: 135.
(10-5) The antisense oligomer, or the pharmaceutically acceptable salt thereof, or the hydrate of the antisense oligomer or the salt according to (10-1), comprising:
   (a) a base sequence selected from the group consisting of SEQ ID NO: 45, SEQ ID NO: 55, SEQ ID NO: 83, and SEQ ID NO: 140;
   (b) a base sequence obtained by adding, deleting, or substituting one or several bases in the base sequence selected from the group consisting of SEQ ID NO: 45, SEQ ID NO: 55, SEQ ID NO: 83, and SEQ ID NO: 140; or
   (c) a base sequence having 80% or more sequence identity with the base sequence selected from the group consisting of SEQ ID NO: 45, SEQ ID NO: 55, SEQ ID NO: 83, and SEQ ID NO: 140.
(10-6) The antisense oligomer, or the pharmaceutically acceptable salt thereof, or the hydrate of the antisense oligomer or the salt according to (10-1), comprising:
   (a) a base sequence selected from the group consisting of SEQ ID NO: 41, SEQ ID NO: 48, SEQ ID NO: 80, and SEQ ID NO: 125;
   (b) a base sequence obtained by adding, deleting, or substituting one or several bases in the base sequence selected from the group consisting of SEQ ID NO: 41, SEQ ID NO: 48, SEQ ID NO: 80, and SEQ ID NO: 125; or
   (c) a base sequence having 80% or more sequence identity with the base sequence selected from the group consisting of SEQ ID NO: 41, SEQ ID NO: 48, SEQ ID NO: 80, and SEQ ID NO: 125.
(10-7) The antisense oligomer, or the pharmaceutically acceptable salt thereof, or the hydrate of the antisense oligomer or the salt according to (10-1), comprising:
   (a) a base sequence selected from the group consisting of SEQ ID NO: 44, SEQ ID NO: 51, and SEQ ID NO: 89;
   (b) a base sequence obtained by adding, deleting, or substituting one or several bases in the base sequence selected from the group consisting of SEQ ID NO: 44, SEQ ID NO: 51, and SEQ ID NO: 89; or
   (c) a base sequence having 80% or more sequence identity with the base sequence selected from the group consisting of SEQ ID NO: 44, SEQ ID NO: 51, and SEQ ID NO: 89.
(10-8) The antisense oligomer, or the pharmaceutically acceptable salt thereof, or the hydrate of the antisense oligomer or the salt according to any one of (10-1) to (10-7), comprising the sequence described in (a).
(11) The antisense oligomer, or the pharmaceutically acceptable salt thereof, or the hydrate of the antisense oligomer or the salt according to any one of (1) to (10), wherein the virus is SARS-CoV-2 or SARS-CoV-1.
(12) The antisense oligomer, or the pharmaceutically acceptable salt thereof, or the hydrate of the antisense oligomer or the salt according to any one of (1) to (11), wherein the antisense oligomer is a morpholino oligomer.
(13) The antisense oligomer, or the pharmaceutically acceptable salt thereof, or the hydrate of the antisense oligomer or the salt according to (12), wherein the antisense oligomer is a phosphorodiamidate morpholino oligomer.
(14) The antisense oligomer, or the pharmaceutically acceptable salt thereof, or the hydrate of the antisense oligomer or the salt according to any one of (1) to (13), wherein the antisense oligomer has any group represented by the following chemical formulas (1) and (2) at the 5' end: 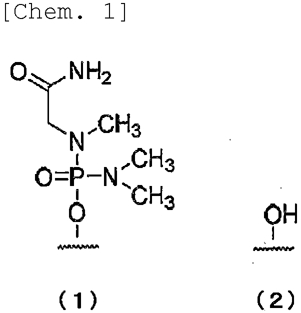
(15) A pharmaceutical composition comprising the antisense oligomer, or the pharmaceutically acceptable salt thereof, or the hydrate of the antisense oligomer or the salt according to any one of (1) to (14).
(16) The pharmaceutical composition according to (15), for treating and/or preventing a viral infectious disease selected from the group consisting of SARS-CoV-2, SARS-CoV-1, and MERS-CoV.
(17) A method for treating and/or preventing a viral infectious disease selected from the group consisting of SARS-CoV-2, SARS-CoV-1, and MERS-CoV, comprising a step of administering, to a subject, an effective amount of the antisense oligomer, or the pharmaceutically acceptable salt thereof, or the hydrate of the antisense oligomer or the salt according to any one of (1) to (14), or the pharmaceutical composition according to (15) or (16) .

The present invention provides an antisense oligomer targeting a base sequence in a specific region of a genome RNA of SARS-CoV-2 or a complementary base sequence thereto, or a pharmaceutically acceptable salt thereof, or a hydrate of the antisense oligomer or the salt, and a composition or the like comprising the antisense oligomer, or the pharmaceutically acceptable salt thereof, or the hydrate of the antisense oligomer or the salt.

According to a preferred embodiment of the present invention, the antisense oligomer of the present invention can provide an antiviral therapeutic drug and/or prophylactic drug with high inhibitory effects on viral growth and/or few side effects. In addition, according to a preferred embodiment of the present invention, the antisense oligomer of the present invention can achieve effects on mutant strains of SARS-CoV-2 and/or known SARS-related coronaviruses (SARSr-CoV) such as SARS-CoV-1 and/or unknown SARS-related coronaviruses.

### Description of Embodiments

### Antisense Oligomer of The Present Invention

In one embodiment, the present invention relates to an antisense oligomer, or a pharmaceutically acceptable salt thereof, or a hydrate of the antisense oligomer or the salt comprising or consisting of a base sequence complementary to a base sequence in a target region, and the target region comprises or consists of: a sequence of at least 10 consecutive bases in at least one region selected from the group consisting of a 5' UTR region, a nsp1 region, a nsp10 region, an RNA-dependent RNA polymerase region, an ORF10 region, and a 3' UTR region in the genome RNA of SARS-CoV-2; or a complementary sequence thereof (hereinafter, the antisense oligomer, and the pharmaceutically acceptable salt thereof, and the hydrate of the antisense oligomer and the salt are also collectively referred to as "first antisense oligomer of the present invention").

As used herein, the "antisense oligomer" means an oligomer comprising a complementary base sequence in the target region.

In one embodiment, the present invention relates to an antisense oligomer, or a pharmaceutically acceptable salt thereof, or a hydrate of the antisense oligomer or the salt, comprising or consisting of: a first antisense oligomer unit that comprises or consists of a base sequence complementary to a base sequence in a first target region, the first target region comprising or consisting of a sequence of at least 10 consecutive bases in a first region selected from the group consisting of the 5' UTR region, the nsp1 region, the nsp10 region, the RNA-dependent RNA polymerase region, the ORF10 region, and the 3' UTR region in the genome RNA of SARS-CoV-2, or a complementary sequence thereof; and a second antisense oligomer unit that comprises or consists of a base sequence complementary to a base sequence in a second target region, the second target region comprising or consisting of a sequence of at least 10 consecutive bases in a second region selected from the group consisting of the 5' UTR region, the nsp1 region, the nsp10 region, the RNA-dependent RNA polymerase region, the ORF10 region, and the 3' UTR region in the genome RNA of SARS-CoV-2, or a complementary sequence thereof (hereinafter, the antisense oligomer, and the pharmaceutically acceptable salt thereof, and the hydrate of the antisense oligomer and the salt are also collectively referred to as "second antisense oligomer of the present invention", and the "first antisense oligomer of the present invention" and "second antisense oligomer of the present invention" are also collectively referred to as "antisense oligomer of the present invention").

As used herein, a base "complementary" to a given base means a base that forms base pairs with the intended base, is not limited to a base that forms Watson-Crick base pairs therewith, but also includes a base that forms wobble base pairs or Hoogsteen base pairs therewith. Herein, the Watson-Crick base pair means a base pair in which a receptor of hydrogen provided from position N3 of the pyrimidine base is at position of N1 of the purine base in the hydrogen bond between adenine and thymine, between adenine and uracil, and between guanine and cytosine, and the wobble base pair means a base pair that forms a hydrogen bond between guanine and uracil, between inosine and uracil, between inosine and adenine, and between inosine and cytosine. The Hoogsteen base pair means a base pair in which a receptor of hydrogen provided from position N3 of the pyrimidine base is at position of N7 of the purine base in the hydrogen bond between adenine and thymine, between adenine and uracil, and between guanine and cytosine.

The term "complementary sequence" or "complementary base sequence" does not have to have 100% complementarity with the intended base sequence, and may comprise, for example, 1, 2, 3, 4, or 5 noncomplementary bases based on the intended base sequence, or may be a base sequence shorter by 1 base, 2 bases, 3 bases, 4 bases, or 5 bases than the intended base sequence. In one embodiment, a base sequence "complementary" to a given base sequence has at least 90%, 91%, 92%, 93%, 94%, 95%, 96%, 97%, 98%, 99%, or 100% complementarity with the intended base sequence. Complementarity can be easily determined by those skilled in the art, and can be calculated, for example, by aligning two sequences, counting the number of bases forming Watson-Crick base pairs or wobble base pairs between these sequences, dividing the number of bases forming the base pairs by the total number of bases in the sequence, and multiplying the resultant by 100.

Examples of a base sequence "complementary" to a given base sequence include a base sequence of an antisense oligomer that can hybridize under stringent conditions, for example, to a nucleic acid comprising the base sequence. As used herein, the term "stringent conditions" may be any of low stringent conditions, moderate stringent conditions, and high stringent conditions. The term "low stringent conditions" is conditions of, for example, 5 × SSC, 5 × Denhardt's solution, 0.5% SDS, 50% formamide at 32°C. The term "moderate stringent conditions" is conditions of, for example, 5 × SSC, 5 × Denhardt's solution, 0.5% SDS, 50% formamide at 42°C, or 5 × SSC, 1% SDS, 50 mM Tris-HCl (pH 7.5), 50% formamide at 42°C. The term "high stringent conditions" is conditions of, for example, 5 × SSC, 5 × Denhardt's solution, 0.5% SDS, 50% formamide at 50°C, or 0.2 × SSC, 0.1% SDS at 65°C. Under these conditions, base sequences with higher sequence identity are expected to be efficiently obtained at higher temperatures. Multiple factors are, however, involved in hybridization stringency including temperature, probe concentration, probe length, ionic strength, time, salt concentration and others, and those skilled in the art may appropriately select these factors to achieve similar stringency.

When commercially available kits are used for hybridization, for example, an AlkPhos Direct Labelling and Detection System (GE Healthcare) may be used. In this case, according to the attached protocol with kit, after incubation with a labeled probe overnight, the membrane can be washed with a primary wash buffer comprising 0.1% (w/v) SDS at 55°C, thereby detecting hybridization. Alternatively, when a probe is labeled with digoxigenin (DIG) using a commercially available reagent (e.g., a PCR Labelling Mix (Roche Diagnostics)) in producing the probe based on a target sequence, hybridization can be detected with a DIG Nucleic Acid Detection Kit (Roche Diagnostics) or the like.

Note that the identity between base sequences may be determined using algorithm BLAST (Basic Local Alignment Search Tool) by Karlin and Altschul (Proc. Natl. Acad. Sci. USA 872264-2268, 1990; Proc. Natl. Acad. Sci. USA 90: 5873, 1993). Programs called BLASTN and BLASTX based on the BLAST algorithm have been developed (Altschul SF, et al.: J. Mol. Biol. 215: 403, 1990). When a base sequence is analyzed using BLASTN, the parameters are, for example, score = 100 and wordlength = 12. When BLAST and Gapped BLAST programs are used, default parameters for each program are used.

The length in bases of the antisense oligomer of the present invention is not limited, and the antisense oligomer may be, for example, 15 bases long or more, 16 bases long or more, 17 bases long or more, 18 bases long or more, 19 bases long or more, 20 bases long or more, 21 bases long or more, 22 bases long or more, 23 bases long or more, 24 bases long or more, 25 bases long or more, 26 bases long or more, 27 bases long or more, 28 bases long or more, 29 bases long or more, or 30 bases long, and may be 30 bases long or less, 29 bases long or less, 28 bases long or less, 27 bases long or less, 26 bases long or less, 25 bases long or less, 24 bases long or less, 23 bases long or less, 22 bases long or less, 21 bases long or less, 20 bases long or less, 19 bases long or less, 18 bases long or less, 17 bases long or less, 16 bases long or less, or 15 bases long. The antisense oligomer of the present invention may consist of 15 to 30 bases, 15 to 25 bases, 16 to 24 bases, 17 to 23 bases, 18 to 22 bases, 19 to 21 bases, and for example, 20 bases.

Examples of the pharmaceutically acceptable salt of the antisense oligomer of the present invention include alkali metal salts such as a sodium salt, a potassium salt, and a lithium salt; alkaline earth metal salts such as a calcium salt, and a magnesium salt; metal salts such as an aluminum salt, an iron salt, a zinc salt, a copper salt, a nickel salt, and a cobalt salt; an ammonium salt; organic amine salts such as a t-octylamine salt, a dibenzylamine salt, a morpholine salt, a glucosamine salt, a phenylglycine alkyl ester salt, an ethylenediamine salt, an N-methylglucamine salt, a guanidine salt, a diethylamine salt, a triethylamine salt, a dicyclohexylamine salt, an N,N'-dibenzylethylenediamine salt, a chloroprocaine salt, a procaine salt, a diethanolamine salt, an N-benzyl-phenethylamine salt, a piperazine salt, a tetramethylammonium salt, and a tris(hydroxymethyl)aminomethane salt; hydrohalide salts such as a hydrofluoride salt, a hydrochloride salt, a hydrobromide salt, and a hydroiodide salt; inorganic acid salts such as a nitrate salt, a perchlorate salt, a sulfate salt, and a phosphate salt; lower alkanesulfonate salts such as a methanesulfonate salt, a trifluoromethanesulfonate salt, and an ethanesulfonate salt; arylsulfonate salts such as a benzenesulfonate salt, and p-toluenesulfonate salt; organic acid salts such as an acetate salt, a malate salt, a fumarate salt, a succinate salt, a citrate salt, a tartrate salt, an oxalate salt, and a maleate salt; and amino acid salts such as a glycine salt, a lysine salt, an arginine salt, an ornithine salt, a glutamate salt, an aspartate salt. These salts may be produced by known methods. Alternatively, the antisense oligomer of the present invention may be in the form of a hydrate thereof.

The antisense oligomer of the present invention may be an oligonucleotide, a morpholino oligomer, or a peptide nucleic acid (PNA) oligomer (hereinafter, also referred to as "antisense oligonucleotide of the present invention", "antisense morpholino oligomer of the present invention", or "antisense peptide nucleic acid oligomer of the present invention", respectively).

The antisense oligonucleotide of the present invention is an antisense oligomer whose constituent unit is a nucleotide, and the nucleotide may be any of a ribonucleotide, a deoxyribonucleotide, or a modified nucleotide.

The modified nucleotide refers to one fully or partly modified in a nucleobase, a sugar moiety and a phosphate-binding region that constitute the ribonucleotide or deoxyribonucleotide.

Examples of the nucleobases can include adenine, guanine, hypoxanthine, cytosine, thymine, uracil, and modified bases thereof. Examples of the modified bases include, but not limited to, pseudouracil, 3-methyluracil, dihydrouracil, 5-alkylcytosine (e.g., 5-methylcytosine), 5-alkyluracil (e.g., 5-ethyluracil), 5-halouracil (e.g., 5-bromouracil), 6-azapyrimidine, 6-alkylpyrimidine (e.g., 6-methyluracil), 2-thiouracil, 4-thiouracil, 4-acetylcytosine, 5-(carboxyhydroxymethyl)uracil, 5-carboxymethylaminomethyl-2-thiouracil, 5-carboxymethylaminomethyl uracil, 1-methyladenine, 1-methylhypoxanthine, 2,2-dimethylguanine, 3-methylcytosine, 2-methyladenine, 2-methylguanine, N6-methyladenine, 7-methylguanine, 5-methoxyaminomethyl-2-thiouracil, 5-methylaminomethyluracil, 5-methylcarbonylmethyluracil, 5-methyloxyuracil, 5-methyl-2-thiouracil, 2-methylthio-N6-isopentenyladenine, uracil-5-oxyacetic acid, 2-thiocytosine, purine, 2,6-diaminopurine, 2-aminopurine, isoguanine, indole, imidazole, and xanthine.

As used herein, thymine "T" and uracil "U" are interchangeable with each other. Neither "T" nor "U" essentially influences the activity of the antisense oligomer of the present invention, and therefore, as used herein, base sequences that are identical except for whether "T" is replaced by "U" are represented by the same SEQ ID NO. Furthermore, as used herein, a sequence comprising a modified base and a sequence not comprising the modified base are represented by the same SEQ ID NO. For example, "cytosine" and "methylcytosine" are interchangeable with each other, and sequences that are identical except for whether "cytosine" is replaced by "methylcytosine" are represented by the same SEQ ID NO.

Examples of the modification of the sugar moiety can include modifications at the 2'-position of ribose, and modifications of other portions of the sugar. Examples of the modification at the 2'-position of ribose include a modification of replacing -OH at the 2'-position of ribose with -OR, -OROR, -R, -R'OR, -SH, -SR, -NH₂, -NHR, -NR₂, -N₃, -CN, -F, -Cl, -Br or -I, for example, -OMe(-O-CH₃) or -O-methoxyethyl (-O-MOE: -O-CH₂CH₂OCH₃). Here, R represents an alkyl or an aryl. R' represents an alkylene.

Examples of the modification for the other portions of the sugar include, but not limited to, replacement of O at the 4'-position of ribose or deoxyribose with S, bridging between 2'- and 4'-positions of the sugar, such as locked nucleic acid (LNA) or 2'-O,4'-C-ethylene-bridged nucleic acids (ENA).

Examples of the modification for the phosphate-binding region can include a modification of replacing phosphodiester bond with a phosphorothioate bond, a phosphorodithioate bond, an alkyl phosphonate bond, a phosphoramidate bond, and a boranophosphate bond (see, e.g., Enya et al.: Bioorganic & Medicinal Chemistry, 2008, 18, 9154-9160) (see, e.g., Japan Domestic RePublication of PCT Application Nos. 2006/129594 and 2006/038608).

As used herein, the alkyl is preferably a straight or branched alkyl having 1 to 6 carbon atoms. Specific examples thereof include methyl, ethyl, n-propyl, isopropyl, n-butyl, isobutyl, sec-butyl, tert-butyl, n-pentyl, isopentyl, neopentyl, tert-pentyl, n-hexyl, and isohexyl. The alkyl may optionally be substituted, and examples of the substituent therefor can include a halogen, an alkoxy, a cyano, and a nitro. The alkyl may be substituted with 1 to 3 substituents.

As used herein, the cycloalkyl is preferably a cycloalkyl having 3 to 12 carbon atoms. Specific examples thereof include cyclopropyl, cyclobutyl, cyclopentyl, cyclohexyl, cycloheptyl, cyclooctyl, cyclodecyl, and cyclododecyl.

As used herein, examples of the halogen can include fluorine, chlorine, bromine, and iodine.

As used herein, examples of the alkoxy include a straight or branched alkoxy having 1 to 6 carbon atoms, such as methoxy, ethoxy, n-propoxy, isopropoxy, n-butoxy, isobutoxy, sec-butoxy, tert-butoxy, n-pentyloxy, isopentyloxy, n-hexyloxy, and isohexyloxy. Among others, an alkoxy having 1 to 3 carbon atoms is preferred.

As used herein, the aryl is preferably an aryl having 6 to 10 carbon atoms. Specific examples thereof can include phenyl, α-naphthyl, and β-naphthyl. Among others, phenyl is preferred. The aryl may optionally be substituted, and examples of the substituent therefor can include an alkyl, a halogen, an alkoxy, a cyano, and nitro. The aryl may be substituted with 1 to 3 substituents.

As used herein, the alkylene is preferably a straight or branched alkylene having 1 to 6 carbon atoms. Specific examples thereof can include methylene, ethylene, trimethylene, tetramethylene, pentamethylene, hexamethylene, 2-(ethyl)trimethylene, and 1-(methyl)tetramethylene.

As used herein, examples of the acyl can include a straight or branched alkanoyl or aroyl. Examples of the alkanoyl include formyl, acetyl, 2-methylacetyl, 2,2-dimethylacetyl, propionyl, butyryl, isobutyryl, pentanoyl, 2,2-dimethylpropionyl, and hexanoyl. Examples of the aroyl can include benzoyl, toluoyl, and naphthoyl. The aroyl may optionally be substituted at substitutable positions, and may be substituted with an alkyl(s).

The antisense oligonucleotide of the present invention may be easily synthesized using various automated synthesizers (e.g., AKTA oligopilot plus 10/100 (GE Healthcare)). Alternatively, the synthesis may also be entrusted to a third-party organization (e.g., Promega Corp. or Takara Co.).

The antisense morpholino oligomer of the present invention is an antisense oligomer whose constituent unit is a group represented by the following general formula: 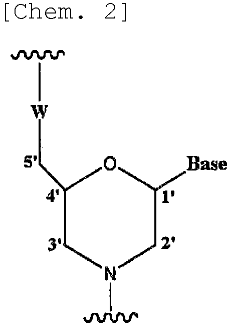
wherein Base represents a nucleobase; and
W represents a group represented by any of the following formulas: 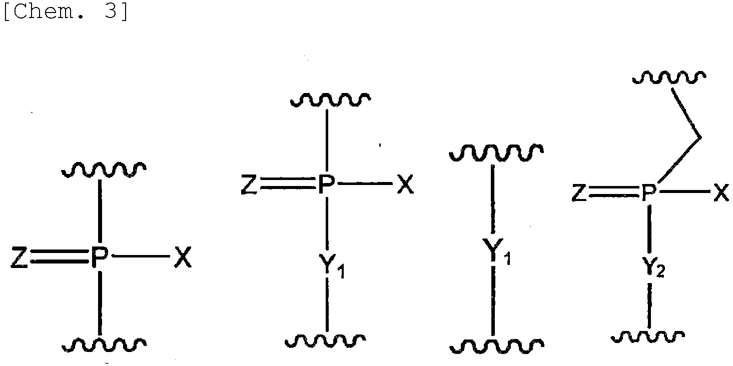
wherein X represents -CH₂R¹, -O-CH₂R¹, -S-CH₂R¹, - NR²R³, or F;
R¹ represents H, or alkyl;
R² and R³ are the same or different, and represent H, alkyl, cycloalkyl, or aryl;
Y₁ represents O, S, CH₂, or NR¹;
Y₂ represents O, S, or NR¹; and
Z represents O or S.

Examples of the morpholino monomer compound used for synthesizing the antisense morpholino oligomer of the present invention include, but not limited to, a morpholino monomer compound (A), a morpholino monomer compound (C), a morpholino monomer compound (T), and morpholino monomer compound (G) shown in Table 1.

**[Table 1]**

| Morpholino monomer compound (A) | Morpholino monomer compound (C) | Morpholino monomer compound (T) | Morpholino monomer compound (G) |
|---|---|---|---|
| 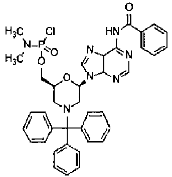 | 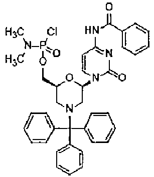 | 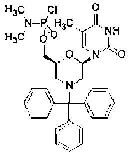 | 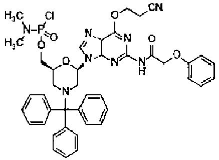 |

In the present invention, the morpholino oligomer is preferably an oligomer whose constituent unit is a group represented by the following formula (phosphorodiamidate morpholino oligomer, (hereinafter referred to as "PMO")): 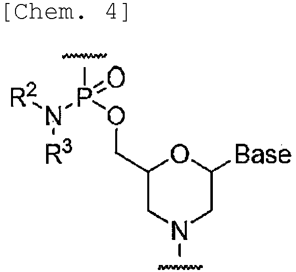 wherein Base, R² and R³ are as defined above.

The morpholino oligomer can be produced according to the methods described in International Publication Nos. 1991/009033, or 2009/064471, for example. In particular, PMO can be produced according to the methods described in International Publication Nos. 2009/064471, or 2013/100190.

In addition, the antisense oligomer of the present invention may have any of the groups represented in the following chemical formula (1) or (2) at its 5'-end.

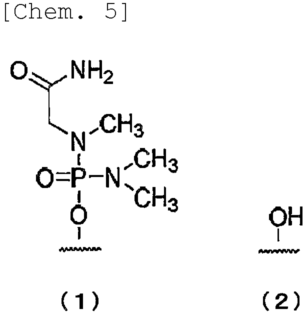

The antisense peptide nucleic acid oligomer of the present invention is an antisense oligomer whose constituent unit is a group represented by the following general formula: 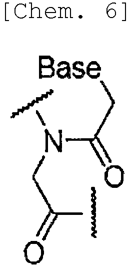 wherein Base is as defined above.

The peptide nucleic acid oligomer can be produced, for example, according to the following references:
1) P. E. Nielsen, M. Egholm, R. H. Berg, O. Buchardt, Science, 254, 1497 (1991) 2) M. Egholm, O. Buchardt, P. E. Nielsen, R. H. Berg, JACS, 114, 1895 (1992) 3) K. L. Dueholm, M. Egholm, C. Behrens, L. Christensen, H. F. Hansen, T. Vulpius, K. H. Petersen, R. H. Berg, P. E. Nielsen, O. Buchardt, J. Org. Chem., 59, 5767 (1994) 4) L. Christensen, R. Fitzpatrick, B. Gildea, K. H. Petersen, H. F. Hansen, T. Koch, M. Egholm, O. Buchardt, P. E. Nielsen, J. Coull, R. H. Berg, J. Pept. Sci., 1, 175 (1995) 5) T. Koch, H. F. Hansen, P. Andersen, T. Larsen, H. G. Batz, K. Otteson, H. Orum, J. Pept. Res., 49, 80 (1997)

In one embodiment, the antisense oligomer of the present invention is a conjugate to which a functional peptide, for example, cell-permeable peptide (CPP) is attached. Known functional peptides or commercially-available functional peptides can be used herein. Examples of the functional peptides that can be used herein include an arginine-rich peptide disclosed in International Publication No. 2008/036127; a peptide targeting organs disclosed in International Publication No. 2009/005793, such as RXR, or RBR; and a peptide comprising amino acid subunits disclosed in International Publication No. 2012/150960. The cell-permeable peptide (CPP) can pass through cell membranes of mammalian cells, and accordingly, it represents a short peptide sequence having about 10 to about 30 amino acids capable of improving cell drug delivery (see, e.g., Hum Mol Genet. 2011 Aug 15; 20(16): 3151-3160; Pharmacology & Therapeutics 154 (2015) 78-86). Known CPPs or commercially-available CPPs can be used herein. Examples of the CPPs that can be used herein include the CPPs listed in Pharmacology & Therapeutics 154 (2015) 78-86, p.80, Table 1, such as TAT(48-60), penetratin, polyarginine, Oct4, WT1-pTj, DPV3, transportan, MAP, VP22, Rep1, KW, KFGF, FGF12, integrin β3 peptide, C105Y, and TP2; and the CPPs listed in Japanese Translation of PCT International Application Publication No. 2017-500856 (International Publication No. 2015/089487), paragraph[0085], Table 1, such as DPV10/6, DPV15b, YM-3, Tat, LR11, C45D18, Lyp-1, Lyp-2, BMV GAG, hLF1-22, C45D18, and LR20. The CPPs are commercially available from Funakoshi, Co., Ltd., for example. Commercially available CPPs such as TAT (Funakoshi, Co., Ltd.), and penetratin (Funakoshi, Co., Ltd.), or known CPPs such as R8 can be used herein. Examples of preferable CPPs that can be used herein include hLIMK, TAT, penetratin, and R8 (see, e.g., International Publication Nos. 2016/187425, 2018/118662, 2018/118599, and 2018/118627, and EBioMedicine 45 (2019) 630-645). The CPP can be directly bound to the antisense oligomer of the present invention, or can be bound via a linker capable of binding the CPP to the antisense oligomer. Known linkers can be used herein. Examples of the linker include those described in Japanese Translation of PCT International Application Publication No. 2017-500856 (International Publication No. 2015/089487), International Publication Nos. 2015/089487, 2009-073809, 2013/075035, 2015/105083, 2014/179620, 2015/006740, and 2017/010575. Examples of preferable linkers that can be used herein include 4-maleimidobutyrate, a linker capable of binding to the functional peptide or antisense oligomer described herein via disulfide bond. The conjugate as used herein can be prepared by a method known to those skilled in the art.

In one embodiment, the antisense oligomer of the present invention has antiviral effects on SARS-CoV-2, SARS-CoV-1, or MERS-CoV, such as SARS-CoV-2 or SARS-CoV-1, for example, SARS-CoV-2. As used herein, SARS-CoV-2 encompasses viruses having a genome RNA sequence consisting of the base sequence of NC_045512.2 (SEQ ID NO: 1) or a mutant strain thereof. SARS-CoV-1 encompasses viruses having a genome RNA sequence consisting of the base sequence of NC_004718.3 or a mutant strain thereof. MERS-CoV encompasses viruses having a genome RNA sequence consisting of the base sequence of NC_019843.3 or a mutant strain thereof.

Here, the mutant strain refers to a descendant having new properties resulting from mutations in which some misreading or recombination occurs during the replication process of viral genes, and the genetic information is partially changed. The mutant strain has some properties that have changed due to the changed genetic information, but the original viral species remains unchanged. Note that in the family *Coronaviridae,* viruses that share 90% or more of the amino acid sequence in the conserved replicase domains are considered to belong to the same species (see, ICTV 9th Report (2011), section Nidrovirales, Coronaviridae (https://talk.ictvonline.org/ictv-reports/ictv_9th_report/positive-sense-rna-viruses-2011/w/posrna_viruses/222/coronaviridae)).

In one embodiment, the antisense oligomer of the present invention can achieve effects not only the known SARS-related coronaviruses (SARSr-CoV), but also unknown SARS-related coronaviruses. As used herein, the SARS-related coronavirus is one of the coronaviruses that cause infection in mammals such as humans or bats, and means a single-stranded plus-strand RNA virus having an envelope, and belonging to the genus *Betacoronavirus* (group 2 coronavirus). The SARS-related coronavirus utilizes an angiotensin-converting enzyme 2 (ACE2) receptor to enter cells.

In one embodiment, the antiviral effect means effects of suppressing viral growth, and/or reducing the infectivity of the virus. Whether the antisense oligomer of the present invention has the antiviral effect can be tested as described in Examples of the present specification. For example, the presence or absence of the antiviral effect can be measured in such a manner that a plasmid that expresses a nucleic acid comprising a sequence in a target region (also referred to as "target sequence") is produced, the obtained plasmid and the antisense oligomer of the present invention are introduced into cell, and whether the amount of the nucleic acid comprising the target sequence expressed by the cells is reduced (e.g., by 5% or more, 10% or more, or 20% or more) or not is examined, compared to the case of introduction of negative control nucleic acid. Alternatively the presence or absence of the antiviral effect can be measured in such a manner that, after introducing the virus and the antisense oligomer of the present invention into cell to culture, whether the amount of the virus in the cell or in cell culture supernatant or the nucleic acid derived therefrom is reduced (e.g., by 5% or more, 10% or more, or 20% or more) or not is examined, compared to the case of introduction of negative control nucleic acid.

In one embodiment, the antisense oligomer of the present invention has a rate of suppressing intracellular virus or a rate of suppressing infectious virus in medium of 5% or more, 10% or more, 20% or more, 30% or more, 40% or more, 50% or more, 60% or more, 70% or more, 80% or more, or 90% or more, when testing as described in Example 2, at the measurement of knockdown activity of gapmer using SARS-CoV-2 virus, and at any concentration of 3, 10, 20, 30, or 50 µM, or has a target sequence identical to that of the antisense oligomer having these activities.

In one embodiment, the antisense oligomer of the present invention targets regions conserved in the sequences of the genome RNA of SARS-CoV-2 and the genome RNA of SARS-CoV-1, and therefore, can achieve antiviral effects on the genus *Betacoronavirus* comprising SARS-CoV-2, SARS-CoV-1, and MERS-CoV.

In one embodiment, the antisense oligomer of the present invention inhibits the function of a target region. As used herein, the phrase "to inhibit the function of a target region" encompasses one or more of inhibiting replication of the genome RNA comprising the target region in which a double strand is formed by the antisense oligomer bound to the target region, inhibiting translation when the target region is to be translated, and inhibiting transcription of the genome RNA comprising the target region. As used herein, the term "sub-genome RNA" refers to an RNA that is synthesized with an RNA-dependent RNA polymerase using a (+) strand genome RNA as a template, and is shorter than a genome RNA synthesized with the RNA-dependent RNA polymerase using a part of a (-) strand RNA as a template, and means an RNA working as an mRNA for viral protein synthesis (translation).

### First Antisense Oligomer of The Present Invention

The first antisense oligomer of the present invention comprises or consists of a base sequence complementary to a base sequence in a target region, and the target region comprises or consists of a sequence of at least 10, for example, at least 11, at least 12, at least 13, at least 14, at least 15, at least 16, at least 17, at least 18, at least 19, or at least 20, for example, 20 consecutive bases in at least one region selected from the group consisting of a 5' UTR region, a nsp1 region, a nsp10 region, an RNA-dependent RNA polymerase region, an ORF10 region, and a 3' UTR region in the genome RNA of SARS-CoV-2, or a complementary sequence thereof.

The sequence of the genome RNA of SARS-CoV-2, and the sequence of each region in the genome RNA can easily be determined with reference to a database (e.g., ncbi). For example, the sequence of the genome RNA of SARS-CoV-2 may be the base sequence of NC_045512.2 (SEQ ID NO: 1). In addition, in the base sequence of SEQ ID NO: 1, the 5' UTR region may be the base sequence of positions 1 to 265 of SEQ ID NO: 1, the nsp1 region may be the base sequence of positions 266 to 805 of SEQ ID NO: 1, the nsp10 region may be the base sequence of positions 13025 to 13441 of SEQ ID NO: 1, the RNA-dependent RNA polymerase region may be the base sequence of positions 13442 to 16236 of SEQ ID NO: 1, the ORF10 region may be the base sequence of positions 29558 to 29674 of SEQ ID NO: 1, and the 3' UTR region may be the base sequence of positions 29675 to 29903 of SEQ ID NO: 1.

The first antisense oligomer of the present invention may target a (+) strand of the genome RNA of SARS-CoV-2, or may target a (-) strand thereof. In a case of targeting a (-) strand, a part of the complementary sequence of the base sequence of SEQ ID NO: 1 can be set to the target region.

In one embodiment, the target region is a base sequence in the regions conserved in the sequences of the genome RNA of SARS-CoV-2 and the genome RNA of SARS-CoV-1, for example, a base sequence selected from the group consisting of positions 43 to 116, 122 to 132, 185 to 208, 242 to 279, 290 to 312, 402 to 425, 455 to 477, 13363 to 13407, 13412 to 13435, 13458 to 13547, 13578 to 13601, 29554 to 29580, 29598 to 29634, 29638 to 29648, 29652 to 29665, 29667 to 29682, 29689 to 29699, 29708 to 29731, 29744 to 29768, and 29787 to 29867 of SEQ ID NO: 1, or a complementary sequence thereof.

In one embodiment, the target region is a base sequence selected from the group consisting of positions 44 to 67, 52 to 75, 55 to 75, 71 to 94, 93 to 116, 185 to 208, 242 to 265, 246 to 269, 250 to 273, 255 to 278, 290 to 312, 402 to 425, 455 to 477, 13363 to 13386, 13384 to 13407, 13412 to 13435, 13461 to 13484, 13466 to 13489, 13470 to 13493, 13475 to 13498, 13479 to 13502, 13488 to 13513, 13502 to 13525, 13515 to 13538, 13578 to 13601, 29554 to 29580, 29598 to 29621, 29611 to 29634, 29708 to 29731, 29744 to 29768, 29787 to 29810, 29792 to 29815, 29797 to 29820, 29817 to 29840, 29822 to 29845, 29827 to 29850, 29832 to 29855, 29837 to 29860, and 29844 to 29867 of SEQ ID NO: 1, or a complementary sequence thereof.

In one embodiment, the first antisense oligomer of the present invention comprises:
(a) a base sequence selected from the group consisting of SEQ ID NOs: 2 to 40;
(b) a base sequence obtained by adding, deleting, or substituting one or several bases in the base sequence selected from the group consisting of SEQ ID NOs: 2 to 40; or
(c) a base sequence having 80%, 85% or more, 86% or more, 87% or more, 88% or more, 89% or more, 90% or more, 910 or more, 92% or more, 93% or more, 94% or more, 95% or more, 96% or more, 97% or more, 98% or more, or 99% or more sequence identity with the base sequence selected from the group consisting of SEQ ID NOs: 2 to 40,
or consists of any of the sequences. In one embodiment, the first antisense oligomer of the present invention comprises or consists of any of the base sequences in (a) above.

As used herein, several in which one or several bases are added, deleted, or substituted means 2, 3, 4, 5, 6, 7, 8, 9, or 10.

The first antisense oligomer of the present invention may comprise only one base sequence complementary to the base sequence of the target region, or may comprise a plurality of, for example, 2, 3, 4, or 5 or more base sequences complementary to the base sequence of each target region.

### Second Antisense Oligomer of The Present Invention

The second antisense oligomer of the present invention comprises or consists of a first antisense oligomer unit and a second antisense oligomer unit. The order of the first antisense oligomer unit and the second antisense oligomer unit is not limited, and for example, the second antisense oligomer of the present invention comprises the first antisense oligomer unit and the second antisense oligomer unit in this order from the 5' end.

In the second antisense oligomer of the present invention, the first antisense oligomer unit comprises a base sequence complementary to the base sequence in the first target region, and the first target region comprises or consists of a sequence of at least 10, for example, at least 11, at least 12, at least 13, at least 14, at least 15, at least 16, at least 17, at least 18, at least 19, or at least 20, for example, 8 to 12 consecutive bases in the first region selected from the group consisting of the 5' UTR region, the nsp1 region, the nsp10 region, the RNA-dependent RNA polymerase region, the ORF10 region, and the 3' UTR region in the genome RNA of SARS-CoV-2, or a complementary sequence thereof. Also, in the second antisense oligomer of the present invention, the second antisense oligomer unit comprises a base sequence complementary to the base sequence in the second target region, and the second target region comprises or consists of a sequence of at least 10, for example, at least 11, at least 12, at least 13, at least 14, at least 15, at least 16, at least 17, at least 18, at least 19, or at least 20, for example, 8 to 12 consecutive bases in the second region selected from the group consisting of the 5' UTR region, the nsp1 region, the nsp10 region, the RNA-dependent RNA polymerase region, the ORF10 region, and the 3' UTR region in the genome RNA of SARS-CoV-2, or a complementary sequence thereof.

The first antisense oligomer unit and the second antisense oligomer unit may be, for example, 8 bases long or more, 9 bases long or more, 10 bases long or more, 11 bases long or more, 12 bases long or more, 13 bases long or more, 14 bases long or more, 15 bases long or more, 16 bases long or more, 17 bases long or more, 18 bases long or more, 19 bases long or more, or 20 bases long, and may be 20 bases long or less, 19 bases long or less, 18 bases long or less, 17 bases long or less, 16 bases long or less, 15 bases long or less, 14 bases long or less, 13 bases long or less, 12 bases long or less, 11 bases long or less, 10 bases long or less, 9 bases long or less, or 8 bases long. The first antisense oligomer unit and the second antisense oligomer unit may be, for example, 8 to 20 bases long, 9 to 18 bases long, or 10 to 16 bases long.

In one embodiment, the first and second regions in the second antisense oligomer of the present invention satisfy any requirement of the following (i) to (iii):
(i) the difference between a position in a base sequence of SEQ ID NO: 1 at an end of a sequence of at least 10 consecutive bases in the first region and a position in the base sequence of SEQ ID NO: 1 at an end of a sequence of at least 10 consecutive bases in the second region is 500 bases or less, 400 bases or less, 300 bases or less, 250 bases or less, 200 bases or less, 150 bases or less, or 100 bases or less;
(ii) the first and second regions are the 5' UTR and the 3' UTR regions, respectively, or the 3' UTR and the 5' UTR regions, respectively; or
(iii) a surrounding sequence of the first region and a surrounding sequence of the second region are complementary to each other, and the surrounding sequences base-pair with each other when replicating, transcribing or translating a virus.

Here, the difference between the position in the base sequence of SEQ ID NO: 1 at the end of a sequence of at least 10 consecutive bases in the first region and the position in the base sequence of SEQ ID NO: 1 at the end of a sequence of at least 10 consecutive bases in the second region in (i) means a distance between the regions in the base sequence of SEQ ID NO: 1. For example, the difference between the positions means the difference between the position at the 3' end of a sequence of at least 10 consecutive bases in the first region and the position at the 5' end of a sequence of at least 10 consecutive bases in the second region in the base sequence of SEQ ID NO: 1, when the first region resides closer to the 5' end than the second region in the base sequence of SEQ ID NO: 1, and the difference between the position at the 5' end of a sequence of at least 10 consecutive bases in the first region and the position at the 3' end of a sequence of at least 10 consecutive bases in the second region in the base sequence of SEQ ID NO: 1, when the first region resides closer to the 3' end than the second region in the base sequence of SEQ ID NO: 1.

In addition, the surrounding sequences of the regions in (iii) mean sequences residing within 500 bases, 400 bases, 300 bases, 250 bases, 200 bases, 150 bases, or 100 bases from the region in the direction of the 5' end or the 3' end in the base sequence of SEQ ID NO: 1. Also, the base pairing with the surrounding sequences in (iii) is as described herein for the base "complementary" to a given base.

In one embodiment, a sequence of at least 10 consecutive bases in the first region and a sequence of at least 10 consecutive bases in the second region in the second antisense oligomer of the present invention are not consecutive or overlapping with each other in the base sequence of SEQ ID NO: 1.

The second antisense oligomer of the present invention may target a (+) strand of the genome RNA of SARS-CoV-2, or may target a (-) strand thereof. In a case of targeting a (-) strand, a part of the complementary sequence of the base sequence of SEQ ID NO: 1 can be set to the target region.

In one embodiment, the first and second target regions are each selected from the group consisting of base sequences at positions 43 to 89, 98 to 110, 122 to 132, 190 to 202, 242 to 279, 290 to 312, 408 to 420, 455 to 477, 13363 to 13386, 13388 to 13401, 13418 to 13432, 13458 to 13516, 13518 to 13532, 13537 to 13547, 13582 to 13598, 29554 to 29566, 29568 to 29580, 29599 to 29613, 29615 to 29634, 29638 to 29648, 29652 to 29665, 29667 to 29682, 29689 to 29699, 29712 to 29731, 29744 to 29757, 29759 to 29768, and 29787 to 29867 of a base sequence of SEQ ID NO: 1, or complementary sequences thereof.

In one embodiment, the first and second target regions are selected from the target sequences listed in Table 2. For example, the first and second target regions may each be a base sequence selected from the group consisting of positions 43 to 53, 43 to 54, 44 to 54, 44 to 55, 45 to 56, 46 to 57, 47 to 58, 48 to 59, 52 to 63, 54 to 43, 55 to 66, 56 to 67, 57 to 68, 58 to 69, 59 to 70, 60 to 71, 61 to 72, 62 to 73, 63 to 74, 63 to 74, 64 to 75, 65 to 76, 75 to 64, 77 to 88, 78 to 89, 98 to 109, 99 to 110, 122 to 132, 190 to 201, 191 to 202, 248 to 259, 249 to 260, 260 to 271, 290 to 301, 300 to 311, 301 to 312, 13390 to 13401, 13466 to 13477, 13474 to 13485, 13478 to 13489, 13488 to 13499, 13491 to 13502, 13492 to 13503, 13493 to 13504, 13494 to 13505, 13495 to 13506, 13496 to 13507, 13497 to 13508, 13499 to 13510, 13501 to 13512, 13502 to 13513, 13503 to 13514, 13504 to 13516, 13520 to 13531, 13537 to 13547, 29638 to 29648, 29652 to 29665, 29667 to 29682, 29671 to 29682, 29689 to 29699, 29712 to 29723, 29720 to 29731, 29744 to 29755, 29744 to 29757, 29745 to 29756, 29746 to 29757, 29757 to 29744, 29787 to 29798, 29789 to 29800, 29799 to 29810, 29803 to 29814, 29804 to 29815, 29805 to 29816, 29806 to 29817, 29807 to 29818, 29808 to 29819, 29809 to 29820, 29810 to 29821, 29822 to 29833, 29823 to 29834, 29833 to 29822, 29843 to 29854, 29854 to 29843, and 29856 to 29867 of a base sequence of SEQ ID NO: 1, or complementary sequences thereof (note that, as used herein, the description "positions a to b" for the target region means, without limitation, that a (+) strand of the genome RNA of SARS-CoV-2 can be targeted when a > b, and a (-) strand of the genome RNA of SARS-CoV-2 can be targeted when a < b).

In one embodiment, the first antisense oligomer unit and the second antisense oligomer unit each comprise:
(a) a base sequence selected from the group consisting of SEQ ID NOs: 174 to 256;
(b) a base sequence obtained by adding, deleting, or substituting one or several bases in the base sequence selected from the group consisting of SEQ ID NOs: 174 to 256; or
(c) a base sequence having 80%, 85% or more, 86% or more, 87% or more, 88% or more, 89% or more, 90% or more, 910 or more, 92% or more, 93% or more, 94% or more, 95% or more, 96% or more, 97% or more, 98% or more, or 99% or more sequence identity with the base sequence selected from the group consisting of SEQ ID NOs: 174 to 256, or consist of any of the sequences. In one embodiment, the first antisense oligomer unit and the second antisense oligomer unit each comprise any of the base sequences in (a) above, or consist of the sequences.

In one embodiment, the first target region is a base sequence at positions 43 to 53 of the base sequence of SEQ ID NO: 1, or a complementary sequence thereof, and the second target region is a base sequence at positions 64 to 75 of the base sequence of SEQ ID NO: 1, or a complementary sequence thereof,
the first target region is a base sequence at positions 43 to 54 of the base sequence of SEQ ID NO: 1, or a complementary sequence thereof, and the second target region is a base sequence at positions 58 to 69 of the base sequence of SEQ ID NO: 1, or a complementary sequence thereof,
the first target region is a base sequence at positions 43 to 54 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 43 to 54 of the base sequence of SEQ ID NO: 1, or a complementary sequence thereof, and the second target region is a base sequence at positions 64 to 75 of the base sequence of SEQ ID NO: 1, or a complementary sequence thereof,
the first target region is a base sequence at positions 44 to 54 of the base sequence of SEQ ID NO: 1, or a complementary sequence thereof, and the second target region is a base sequence at positions 64 to 75 of the base sequence of SEQ ID NO: 1, or a complementary sequence thereof,
the first target region is a base sequence at positions 44 to 55 of the base sequence of SEQ ID NO: 1, or a complementary sequence thereof, and the second target region is a base sequence at positions 58 to 69 of the base sequence of SEQ ID NO: 1, or a complementary sequence thereof,
the first target region is a base sequence at positions 44 to 55 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 44 to 55 of the base sequence of SEQ ID NO: 1, or a complementary sequence thereof, and the second target region is a base sequence at positions 64 to 75 of the base sequence of SEQ ID NO: 1, or a complementary sequence thereof,
the first target region is a base sequence at positions 45 to 56 of the base sequence of SEQ ID NO: 1, or a complementary sequence thereof, and the second target region is a base sequence at positions 58 to 69 of the base sequence of SEQ ID NO: 1, or a complementary sequence thereof,
the first target region is a base sequence at positions 45 to 56 of the base sequence of SEQ ID NO: 1, or a complementary sequence thereof, and the second target region is a base sequence at positions 59 to 70 of the base sequence of SEQ ID NO: 1, or a complementary sequence thereof,
the first target region is a base sequence at positions 45 to 56 of the base sequence of SEQ ID NO: 1, or a complementary sequence thereof, and the second target region is a base sequence at positions 60 to 71 of the base sequence of SEQ ID NO: 1, or a complementary sequence thereof,
the first target region is a base sequence at positions 45 to 56 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 45 to 56 of the base sequence of SEQ ID NO: 1, or a complementary sequence thereof, and the second target region is a base sequence at positions 62 to 73 of the base sequence of SEQ ID NO: 1, or a complementary sequence thereof,
the first target region is a base sequence at positions 45 to 56 of the base sequence of SEQ ID NO: 1, or a complementary sequence thereof, and the second target region is a base sequence at positions 63 to 74 of the base sequence of SEQ ID NO: 1, or a complementary sequence thereof,
the first target region is a base sequence at positions 45 to 56 of the base sequence of SEQ ID NO: 1, or a complementary sequence thereof, and the second target region is a base sequence at positions 64 to 75 of the base sequence of SEQ ID NO: 1, or a complementary sequence thereof,
the first target region is a base sequence at positions 46 to 57 of the base sequence of SEQ ID NO: 1, or a complementary sequence thereof, and the second target region is a base sequence at positions 64 to 75 of the base sequence of SEQ ID NO: 1, or a complementary sequence thereof,
the first target region is a base sequence at positions 47 to 58 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 48 to 59 of the base sequence of SEQ ID NO: 1, or a complementary sequence thereof, and the second target region is a base sequence at positions 64 to 75 of the base sequence of SEQ ID NO: 1, or a complementary sequence thereof,
the first target region is a base sequence at positions 56 to 67 of the base sequence of SEQ ID NO: 1, or a complementary sequence thereof, and the second target region is a base sequence at positions 77 to 88 of the base sequence of SEQ ID NO: 1, or a complementary sequence thereof,
the first target region is a base sequence at positions 56 to 67 of the base sequence of SEQ ID NO: 1, or a complementary sequence thereof, and the second target region is a base sequence at positions 98 to 109 of the base sequence of SEQ ID NO: 1, or a complementary sequence thereof,
the first target region is a base sequence at positions 56 to 67 of the base sequence of SEQ ID NO: 1, or a complementary sequence thereof, and the second target region is a base sequence at positions 122 to 132 of the base sequence of SEQ ID NO: 1, or a complementary sequence thereof,
the first target region is a base sequence at positions 56 to 67 of the base sequence of SEQ ID NO: 1, or a complementary sequence thereof, and the second target region is a base sequence at positions 190 to 201 of the base sequence of SEQ ID NO: 1, or a complementary sequence thereof,
the first target region is a base sequence at positions 56 to 67 of the base sequence of SEQ ID NO: 1, or a complementary sequence thereof, and the second target region is a base sequence at positions 248 to 259 of the base sequence of SEQ ID NO: 1, or a complementary sequence thereof,
the first target region is a base sequence at positions 58 to 69 of the base sequence of SEQ ID NO: 1, or a complementary sequence thereof, and the second target region is a base sequence at positions 98 to 109 of the base sequence of SEQ ID NO: 1, or a complementary sequence thereof,
the first target region is a base sequence at positions 61 to 72 of the base sequence of SEQ ID NO: 1, or a complementary sequence thereof, and the second target region is a base sequence at positions 78 to 89 of the base sequence of SEQ ID NO: 1, or a complementary sequence thereof,
the first target region is a base sequence at positions 61 to 72 of the base sequence of SEQ ID NO: 1, or a complementary sequence thereof, and the second target region is a base sequence at positions 99 to 110 of the base sequence of SEQ ID NO: 1, or a complementary sequence thereof,
the first target region is a base sequence at positions 61 to 72 of the base sequence of SEQ ID NO: 1, or a complementary sequence thereof, and the second target region is a base sequence at positions 122 to 132 of the base sequence of SEQ ID NO: 1, or a complementary sequence thereof,
the first target region is a base sequence at positions 61 to 72 of the base sequence of SEQ ID NO: 1, or a complementary sequence thereof, and the second target region is a base sequence at positions 191 to 202 of the base sequence of SEQ ID NO: 1, or a complementary sequence thereof,
the first target region is a base sequence at positions 61 to 72 of the base sequence of SEQ ID NO: 1, or a complementary sequence thereof, and the second target region is a base sequence at positions 249 to 260 of the base sequence of SEQ ID NO: 1, or a complementary sequence thereof,
the first target region is a base sequence at positions 75 to 64 of the base sequence of SEQ ID NO: 1, or a complementary sequence thereof, and the second target region is a base sequence at positions 54 to 43 of the base sequence of SEQ ID NO: 1, or a complementary sequence thereof,
the first target region is a base sequence at positions 43 to 54 of the base sequence of SEQ ID NO: 1, or a complementary sequence thereof, and the second target region is a base sequence at positions 290 to 301 of the base sequence of SEQ ID NO: 1, or a complementary sequence thereof,
the first target region is a base sequence at positions 43 to 54 of the base sequence of SEQ ID NO: 1, or a complementary sequence thereof, and the second target region is a base sequence at positions 301 to 312 of the base sequence of SEQ ID NO: 1, or a complementary sequence thereof,
the first target region is a base sequence at positions 45 to 56 of the base sequence of SEQ ID NO: 1, or a complementary sequence thereof, and the second target region is a base sequence at positions 290 to 301 of the base sequence of SEQ ID NO: 1, or a complementary sequence thereof,
the first target region is a base sequence at positions 45 to 56 of the base sequence of SEQ ID NO: 1, or a complementary sequence thereof, and the second target region is a base sequence at positions 301 to 312 of the base sequence of SEQ ID NO: 1, or a complementary sequence thereof,
the first target region is a base sequence at positions 47 to 58 of the base sequence of SEQ ID NO: 1, or a complementary sequence thereof, and the second target region is a base sequence at positions 301 to 312 of the base sequence of SEQ ID NO: 1, or a complementary sequence thereof,
the first target region is a base sequence at positions 52 to 63 of the base sequence of SEQ ID NO: 1, or a complementary sequence thereof, and the second target region is a base sequence at positions 290 to 301 of the base sequence of SEQ ID NO: 1, or a complementary sequence thereof,
the first target region is a base sequence at positions 52 to 63 of the base sequence of SEQ ID NO: 1, or a complementary sequence thereof, and the second target region is a base sequence at positions 301 to 312 of the base sequence of SEQ ID NO: 1, or a complementary sequence thereof,
the first target region is a base sequence at positions 56 to 67 of the base sequence of SEQ ID NO: 1, or a complementary sequence thereof, and the second target region is a base sequence at positions 290 to 301 of the base sequence of SEQ ID NO: 1, or a complementary sequence thereof,
the first target region is a base sequence at positions 56 to 67 of the base sequence of SEQ ID NO: 1, or a complementary sequence thereof, and the second target region is a base sequence at positions 301 to 312 of the base sequence of SEQ ID NO: 1, or a complementary sequence thereof,
the first target region is a base sequence at positions 57 to 68 of the base sequence of SEQ ID NO: 1, or a complementary sequence thereof, and the second target region is a base sequence at positions 301 to 312 of the base sequence of SEQ ID NO: 1, or a complementary sequence thereof,
the first target region is a base sequence at positions 58 to 69 of the base sequence of SEQ ID NO: 1, or a complementary sequence thereof, and the second target region is a base sequence at positions 290 to 301 of the base sequence of SEQ ID NO: 1, or a complementary sequence thereof,
the first target region is a base sequence at positions 58 to 69 of the base sequence of SEQ ID NO: 1, or a complementary sequence thereof, and the second target region is a base sequence at positions 300 to 311 of the base sequence of SEQ ID NO: 1, or a complementary sequence thereof,
the first target region is a base sequence at positions 58 to 69 of the base sequence of SEQ ID NO: 1, or a complementary sequence thereof, and the second target region is a base sequence at positions 301 to 312 of the base sequence of SEQ ID NO: 1, or a complementary sequence thereof,
the first target region is a base sequence at positions 60 to 71 of the base sequence of SEQ ID NO: 1, or a complementary sequence thereof, and the second target region is a base sequence at positions 301 to 312 of the base sequence of SEQ ID NO: 1, or a complementary sequence thereof,
the first target region is a base sequence at positions 61 to 72 of the base sequence of SEQ ID NO: 1, or a complementary sequence thereof, and the second target region is a base sequence at positions 260 to 271 of the base sequence of SEQ ID NO: 1, or a complementary sequence thereof,
the first target region is a base sequence at positions 61 to 72 of the base sequence of SEQ ID NO: 1, or a complementary sequence thereof, and the second target region is a base sequence at positions 290 to 301 of the base sequence of SEQ ID NO: 1, or a complementary sequence thereof,
the first target region is a base sequence at positions 61 to 72 of the base sequence of SEQ ID NO: 1, or a complementary sequence thereof, and the second target region is a base sequence at positions 301 to 312 of the base sequence of SEQ ID NO: 1, or a complementary sequence thereof,
the first target region is a base sequence at positions 63 to 74 of the base sequence of SEQ ID NO: 1, or a complementary sequence thereof, and the second target region is a base sequence at positions 290 to 301 of the base sequence of SEQ ID NO: 1, or a complementary sequence thereof,
the first target region is a base sequence at positions 64 to 75 of the base sequence of SEQ ID NO: 1, or a complementary sequence thereof, and the second target region is a base sequence at positions 290 to 301 of the base sequence of SEQ ID NO: 1, or a complementary sequence thereof,
the first target region is a base sequence at positions 64 to 75 of the base sequence of SEQ ID NO: 1, or a complementary sequence thereof, and the second target region is a base sequence at positions 301 to 312 of the base sequence of SEQ ID NO: 1, or a complementary sequence thereof,
the first target region is a base sequence at positions 65 to 76 of the base sequence of SEQ ID NO: 1, or a complementary sequence thereof, and the second target region is a base sequence at positions 290 to 301 of the base sequence of SEQ ID NO: 1, or a complementary sequence thereof,
the first target region is a base sequence at positions 43 to 54 of the base sequence of SEQ ID NO: 1, or a complementary sequence thereof, and the second target region is a base sequence at positions 29822 to 29833 of the base sequence of SEQ ID NO: 1, or a complementary sequence thereof,
the first target region is a base sequence at positions 43 to 54 of the base sequence of SEQ ID NO: 1, or a complementary sequence thereof, and the second target region is a base sequence at positions 29843 to 29854 of the base sequence of SEQ ID NO: 1, or a complementary sequence thereof,
the first target region is a base sequence at positions 43 to 54 of the base sequence of SEQ ID NO: 1, or a complementary sequence thereof, and the second target region is a base sequence at positions 29856 to 29867 of the base sequence of SEQ ID NO: 1, or a complementary sequence thereof,
the first target region is a base sequence at positions 55 to 66 of the base sequence of SEQ ID NO: 1, or a complementary sequence thereof, and the second target region is a base sequence at positions 29822 to 29833 of the base sequence of SEQ ID NO: 1, or a complementary sequence thereof,
the first target region is a base sequence at positions 55 to 66 of the base sequence of SEQ ID NO: 1, or a complementary sequence thereof, and the second target region is a base sequence at positions 29843 to 29854 of the base sequence of SEQ ID NO: 1, or a complementary sequence thereof,
the first target region is a base sequence at positions 55 to 66 of the base sequence of SEQ ID NO: 1, or a complementary sequence thereof, and the second target region is a base sequence at positions 29856 to 29867 of the base sequence of SEQ ID NO: 1, or a complementary sequence thereof,
the first target region is a base sequence at positions 64 to 75 of the base sequence of SEQ ID NO: 1, or a complementary sequence thereof, and the second target region is a base sequence at positions 29720 to 29731 of the base sequence of SEQ ID NO: 1, or a complementary sequence thereof,
the first target region is a base sequence at positions 64 to 75 of the base sequence of SEQ ID NO: 1, or a complementary sequence thereof, and the second target region is a base sequence at positions 29744 to 29757 of the base sequence of SEQ ID NO: 1, or a complementary sequence thereof,
the first target region is a base sequence at positions 64 to 75 of the base sequence of SEQ ID NO: 1, or a complementary sequence thereof, and the second target region is a base sequence at positions 29787 to 29798 of the base sequence of SEQ ID NO: 1, or a complementary sequence thereof,
the first target region is a base sequence at positions 64 to 75 of the base sequence of SEQ ID NO: 1, or a complementary sequence thereof, and the second target region is a base sequence at positions 29799 to 29810 of the base sequence of SEQ ID NO: 1, or a complementary sequence thereof,
the first target region is a base sequence at positions 64 to 75 of the base sequence of SEQ ID NO: 1, or a complementary sequence thereof, and the second target region is a base sequence at positions 29822 to 29833 of the base sequence of SEQ ID NO: 1, or a complementary sequence thereof,
the first target region is a base sequence at positions 64 to 75 of the base sequence of SEQ ID NO: 1, or a complementary sequence thereof, and the second target region is a base sequence at positions 29843 to 29854 of the base sequence of SEQ ID NO: 1, or a complementary sequence thereof,
the first target region is a base sequence at positions 64 to 75 of the base sequence of SEQ ID NO: 1, or a complementary sequence thereof, and the second target region is a base sequence at positions 29856 to 29867 of the base sequence of SEQ ID NO: 1, or a complementary sequence thereof,
the first target region is a base sequence at positions 75 to 64 of the base sequence of SEQ ID NO: 1, or a complementary sequence thereof, and the second target region is a base sequence at positions 29757 to 29744 of the base sequence of SEQ ID NO: 1, or a complementary sequence thereof,
the first target region is a base sequence at positions 75 to 64 of the base sequence of SEQ ID NO: 1, or a complementary sequence thereof, and the second target region is a base sequence at positions 29833 to 29822 of the base sequence of SEQ ID NO: 1, or a complementary sequence thereof,
the first target region is a base sequence at positions 75 to 64 of the base sequence of SEQ ID NO: 1, or a complementary sequence thereof, and the second target region is a base sequence at positions 29854 to 29843 of the base sequence of SEQ ID NO: 1, or a complementary sequence thereof,
the first target region is a base sequence at positions 99 to 110 of the base sequence of SEQ ID NO: 1, or a complementary sequence thereof, and the second target region is a base sequence at positions 29856 to 29867 of the base sequence of SEQ ID NO: 1, or a complementary sequence thereof,
the first target region is a base sequence at positions 13390 to 13401 of the base sequence of SEQ ID NO: 1, or a complementary sequence thereof, and the second target region is a base sequence at positions 13466 to 13477 of the base sequence of SEQ ID NO: 1, or a complementary sequence thereof,
the first target region is a base sequence at positions 13390 to 13401 of the base sequence of SEQ ID NO: 1, or a complementary sequence thereof, and the second target region is a base sequence at positions 13478 to 13489 of the base sequence of SEQ ID NO: 1, or a complementary sequence thereof,
the first target region is a base sequence at positions 13390 to 13401 of the base sequence of SEQ ID NO: 1, or a complementary sequence thereof, and the second target region is a base sequence at positions 13504 to 13516 of the base sequence of SEQ ID NO: 1, or a complementary sequence thereof,
the first target region is a base sequence at positions 13474 to 13485 of the base sequence of SEQ ID NO: 1, or a complementary sequence thereof, and the second target region is a base sequence at positions 13488 to 13499 of the base sequence of SEQ ID NO: 1, or a complementary sequence thereof,
the first target region is a base sequence at positions 13474 to 13485 of the base sequence of SEQ ID NO: 1, or a complementary sequence thereof, and the second target region is a base sequence at positions 13494 to 13505 of the base sequence of SEQ ID NO: 1, or a complementary sequence thereof,
the first target region is a base sequence at positions 13474 to 13485 of the base sequence of SEQ ID NO: 1, or a complementary sequence thereof, and the second target region is a base sequence at positions 13503 to 13514 of the base sequence of SEQ ID NO: 1, or a complementary sequence thereof,
the first target region is a base sequence at positions 13474 to 13485 of the base sequence of SEQ ID NO: 1, or a complementary sequence thereof, and the second target region is a base sequence at positions 13520 to 13531 of the base sequence of SEQ ID NO: 1, or a complementary sequence thereof,
the first target region is a base sequence at positions 13474 to 13485 of the base sequence of SEQ ID NO: 1, or a complementary sequence thereof, and the second target region is a base sequence at positions 13537 to 13547 of the base sequence of SEQ ID NO: 1, or a complementary sequence thereof,
the first target region is a base sequence at positions 13488 to 13499 of the base sequence of SEQ ID NO: 1, or a complementary sequence thereof, and the second target region is a base sequence at positions 13502 to 13513 of the base sequence of SEQ ID NO: 1, or a complementary sequence thereof,
the first target region is a base sequence at positions 13488 to 13499 of the base sequence of SEQ ID NO: 1, or a complementary sequence thereof, and the second target region is a base sequence at positions 13503 to 13514 of the base sequence of SEQ ID NO: 1, or a complementary sequence thereof,
the first target region is a base sequence at positions 13488 to 13499 of the base sequence of SEQ ID NO: 1, or a complementary sequence thereof, and the second target region is a base sequence at positions 13520 to 13531 of the base sequence of SEQ ID NO: 1, or a complementary sequence thereof,
the first target region is a base sequence at positions 13488 to 13499 of the base sequence of SEQ ID NO: 1, or a complementary sequence thereof, and the second target region is a base sequence at positions 13537 to 13547 of the base sequence of SEQ ID NO: 1, or a complementary sequence thereof,
the first target region is a base sequence at positions 13491 to 13502 of the base sequence of SEQ ID NO: 1, or a complementary sequence thereof, and the second target region is a base sequence at positions 13537 to 13547 of the base sequence of SEQ ID NO: 1, or a complementary sequence thereof,
the first target region is a base sequence at positions 13492 to 13503 of the base sequence of SEQ ID NO: 1, or a complementary sequence thereof, and the second target region is a base sequence at positions 13537 to 13547 of the base sequence of SEQ ID NO: 1, or a complementary sequence thereof,
the first target region is a base sequence at positions 13493 to 13504 of the base sequence of SEQ ID NO: 1, or a complementary sequence thereof, and the second target region is a base sequence at positions 13537 to 13547 of the base sequence of SEQ ID NO: 1, or a complementary sequence thereof,
the first target region is a base sequence at positions 13494 to 13505 of the base sequence of SEQ ID NO: 1, or a complementary sequence thereof, and the second target region is a base sequence at positions 13520 to 13531 of the base sequence of SEQ ID NO: 1, or a complementary sequence thereof,
the first target region is a base sequence at positions 13494 to 13505 of the base sequence of SEQ ID NO: 1, or a complementary sequence thereof, and the second target region is a base sequence at positions 13537 to 13547 of the base sequence of SEQ ID NO: 1, or a complementary sequence thereof,
the first target region is a base sequence at positions 13495 to 13506 of the base sequence of SEQ ID NO: 1, or a complementary sequence thereof, and the second target region is a base sequence at positions 13537 to 13547 of the base sequence of SEQ ID NO: 1, or a complementary sequence thereof,
the first target region is a base sequence at positions 13496 to 13507 of the base sequence of SEQ ID NO: 1, or a complementary sequence thereof, and the second target region is a base sequence at positions 13537 to 13547 of the base sequence of SEQ ID NO: 1, or a complementary sequence thereof,
the first target region is a base sequence at positions 13497 to 13508 of the base sequence of SEQ ID NO: 1, or a complementary sequence thereof, and the second target region is a base sequence at positions 13537 to 13547 of the base sequence of SEQ ID NO: 1, or a complementary sequence thereof,
the first target region is a base sequence at positions 13499 to 13510 of the base sequence of SEQ ID NO: 1, or a complementary sequence thereof, and the second target region is a base sequence at positions 13537 to 13547 of the base sequence of SEQ ID NO: 1, or a complementary sequence thereof,
the first target region is a base sequence at positions 13501 to 13512 of the base sequence of SEQ ID NO: 1, or a complementary sequence thereof, and the second target region is a base sequence at positions 13537 to 13547 of the base sequence of SEQ ID NO: 1, or a complementary sequence thereof,
the first target region is a base sequence at positions 13503 to 13514 of the base sequence of SEQ ID NO: 1, or a complementary sequence thereof, and the second target region is a base sequence at positions 13520 to 13531 of the base sequence of SEQ ID NO: 1, or a complementary sequence thereof,
the first target region is a base sequence at positions 13503 to 13514 of the base sequence of SEQ ID NO: 1, or a complementary sequence thereof, and the second target region is a base sequence at positions 13537 to 13547 of the base sequence of SEQ ID NO: 1, or a complementary sequence thereof,
the first target region is a base sequence at positions 13520 to 13531 of the base sequence of SEQ ID NO: 1, or a complementary sequence thereof, and the second target region is a base sequence at positions 13537 to 13547 of the base sequence of SEQ ID NO: 1, or a complementary sequence thereof,
the first target region is a base sequence at positions 29671 to 29682 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 29689 to 29699 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 29689 to 29699 of the base sequence of SEQ ID NO: 1, or a complementary sequence thereof, and the second target region is a base sequence at positions 64 to 75 of the base sequence of SEQ ID NO: 1, or a complementary sequence thereof,
the first target region is a base sequence at positions 29712 to 29723 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 29720 to 29731 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 29744 to 29755 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 29744 to 29757 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 29744 to 29757 of the base sequence of SEQ ID NO: 1, or a complementary sequence thereof, and the second target region is a base sequence at positions 64 to 75 of the base sequence of SEQ ID NO: 1, or a complementary sequence thereof,
the first target region is a base sequence at positions 29744 to 29757 of the base sequence of SEQ ID NO: 1, or a complementary sequence thereof, and the second target region is a base sequence at positions 99 to 110 of the base sequence of SEQ ID NO: 1, or a complementary sequence thereof,
the first target region is a base sequence at positions 29745 to 29756 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 29746 to 29757 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 29746 to 29757 of the base sequence of SEQ ID NO: 1, or a complementary sequence thereof, and the second target region is a base sequence at positions 64 to 75 of the base sequence of SEQ ID NO: 1, or a complementary sequence thereof,
the first target region is a base sequence at positions 29757 to 29744 of the base sequence of SEQ ID NO: 1, or a complementary sequence thereof, and the second target region is a base sequence at positions 75 to 64 of the base sequence of SEQ ID NO: 1, or a complementary sequence thereof,
the first target region is a base sequence at positions 29789 to 29800 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 29803 to 29814 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 29804 to 29815 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 29805 to 29816 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 29806 to 29817 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 29807 to 29818 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 29808 to 29819 of the base sequence of SEQ ID NO: 1, or a complementary sequence thereof, and the second target region is a base sequence at positions 59 to 70 of the base sequence of SEQ ID NO: 1, or a complementary sequence thereof,
the first target region is a base sequence at positions 29808 to 29819 of the base sequence of SEQ ID NO: 1, or a complementary sequence thereof, and the second target region is a base sequence at positions 60 to 71 of the base sequence of SEQ ID NO: 1, or a complementary sequence thereof,
the first target region is a base sequence at positions 29808 to 29819 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 29809 to 29820 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 29810 to 29821 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 29822 to 29833 of the base sequence of SEQ ID NO: 1, or a complementary sequence thereof, and the second target region is a base sequence at positions 64 to 75 of the base sequence of SEQ ID NO: 1, or a complementary sequence thereof,
the first target region is a base sequence at positions 29822 to 29833 of the base sequence of SEQ ID NO: 1, or a complementary sequence thereof, and the second target region is a base sequence at positions 99 to 110 of the base sequence of SEQ ID NO: 1, or a complementary sequence thereof,
the first target region is a base sequence at positions 29823 to 29834 of the base sequence of SEQ ID NO: 1, or a complementary sequence thereof, and the second target region is a base sequence at positions 61 to 72 of the base sequence of SEQ ID NO: 1, or a complementary sequence thereof,
the first target region is a base sequence at positions 29833 to 29822 of the base sequence of SEQ ID NO: 1, or a complementary sequence thereof, and the second target region is a base sequence at positions 75 to 64 of the base sequence of SEQ ID NO: 1, or a complementary sequence thereof,
the first target region is a base sequence at positions 29843 to 29854 of the base sequence of SEQ ID NO: 1, or a complementary sequence thereof, and the second target region is a base sequence at positions 64 to 75 of the base sequence of SEQ ID NO: 1, or a complementary sequence thereof,
the first target region is a base sequence at positions 29854 to 29843 of the base sequence of SEQ ID NO: 1, or a complementary sequence thereof, and the second target region is a base sequence at positions 75 to 64 of the base sequence of SEQ ID NO: 1, or a complementary sequence thereof,
the first target region is a base sequence at positions 29856 to 29867 of the base sequence of SEQ ID NO: 1, or a complementary sequence thereof, and the second target region is a base sequence at positions 78 to 89 of the base sequence of SEQ ID NO: 1, or a complementary sequence thereof,
the first target region is a base sequence at positions 29856 to 29867 of the base sequence of SEQ ID NO: 1, or a complementary sequence thereof, and the second target region is a base sequence at positions 99 to 110 of the base sequence of SEQ ID NO: 1, or a complementary sequence thereof,
the first target region is a base sequence at positions 29856 to 29867 of the base sequence of SEQ ID NO: 1, or a complementary sequence thereof, and the second target region is a base sequence at positions 122 to 132 of the base sequence of SEQ ID NO: 1, or a complementary sequence thereof,
the first target region is a base sequence at positions 29638 to 29648 of the base sequence of SEQ ID NO: 1, or a complementary sequence thereof, and the second target region is a base sequence at positions 29652 to 29665 of the base sequence of SEQ ID NO: 1, or a complementary sequence thereof,
the first target region is a base sequence at positions 29667 to 29682 of the base sequence of SEQ ID NO: 1, or a complementary sequence thereof, and the second target region is a base sequence at positions 29689 to 29699 of the base sequence of SEQ ID NO: 1, or a complementary sequence thereof,
the first target region is a base sequence at positions 29712 to 29723 of the base sequence of SEQ ID NO: 1, or a complementary sequence thereof, and the second target region is a base sequence at positions 29744 to 29757 of the base sequence of SEQ ID NO: 1, or a complementary sequence thereof,
the first target region is a base sequence at positions 29720 to 29731 of the base sequence of SEQ ID NO: 1, or a complementary sequence thereof, and the second target region is a base sequence at positions 29744 to 29757 of the base sequence of SEQ ID NO: 1, or a complementary sequence thereof,
the first target region is a base sequence at positions 29744 to 29757 of the base sequence of SEQ ID NO: 1, or a complementary sequence thereof, and the second target region is a base sequence at positions 29822 to 29833 of the base sequence of SEQ ID NO: 1, or a complementary sequence thereof,
the first target region is a base sequence at positions 29744 to 29757 of the base sequence of SEQ ID NO: 1, or a complementary sequence thereof, and the second target region is a base sequence at positions 29843 to 29854 of the base sequence of SEQ ID NO: 1, or a complementary sequence thereof, or
the first target region is a base sequence at positions 29744 to 29757 of the base sequence of SEQ ID NO: 1, or a complementary sequence thereof, and the second target region is a base sequence at positions 29856 to 29867 of the base sequence of SEQ ID NO: 1, or a complementary sequence thereof.

In one embodiment, the first antisense oligomer unit comprises (a) a base sequence of SEQ ID NO: 174, (b) a base sequence obtained by adding, deleting, or substituting one or several bases in the base sequence of SEQ ID NO: 174, or (c) a base sequence having 80% or more sequence identity with the base sequence of SEQ ID NO: 174, or consists of any of the base sequences, and the second antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences,
the first antisense oligomer unit comprises (a) a base sequence of SEQ ID NO: 176, (b) a base sequence obtained by adding, deleting, or substituting one or several bases in the base sequence of SEQ ID NO: 176, or (c) a base sequence having 80% or more sequence identity with the base sequence of SEQ ID NO: 176, or consists of any of the base sequences, and the second antisense oligomer unit comprises (a) a base sequence of SEQ ID NO: 177, (b) a base sequence obtained by adding, deleting, or substituting one or several bases in the base sequence of SEQ ID NO: 177, or (c) a base sequence having 80% or more sequence identity with the base sequence of SEQ ID NO: 177, or consists of any of the base sequences,
the first antisense oligomer unit comprises (a) a base sequence of SEQ ID NO: 176, (b) a base sequence obtained by adding, deleting, or substituting one or several bases in the base sequence of SEQ ID NO: 176, or (c) a base sequence having 80% or more sequence identity with the base sequence of SEQ ID NO: 176, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 176, (b) a base sequence obtained by adding, deleting, or substituting one or several bases in the base sequence of SEQ ID NO: 176, or (c) a base sequence having 80% or more sequence identity with the base sequence of SEQ ID NO: 176, or consists of any of the base sequences, and the second antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences,
the first antisense oligomer unit comprises (a) a base sequence of SEQ ID NO: 179, (b) a base sequence obtained by adding, deleting, or substituting one or several bases in the base sequence of SEQ ID NO: 179, or (c) a base sequence having 80% or more sequence identity with the base sequence of SEQ ID NO: 179, or consists of any of the base sequences, and the second antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences,
the first antisense oligomer unit comprises (a) a base sequence of SEQ ID NO: 180, (b) a base sequence obtained by adding, deleting, or substituting one or several bases in the base sequence of SEQ ID NO: 180, or (c) a base sequence having 80% or more sequence identity with the base sequence of SEQ ID NO: 180, or consists of any of the base sequences, and the second antisense oligomer unit comprises (a) a base sequence of SEQ ID NO: 177, (b) a base sequence obtained by adding, deleting, or substituting one or several bases in the base sequence of SEQ ID NO: 177, or (c) a base sequence having 80% or more sequence identity with the base sequence of SEQ ID NO: 177, or consists of any of the base sequences,
the first antisense oligomer unit comprises (a) a base sequence of SEQ ID NO: 180, (b) a base sequence obtained by adding, deleting, or substituting one or several bases in the base sequence of SEQ ID NO: 180, or (c) a base sequence having 80% or more sequence identity with the base sequence of SEQ ID NO: 180, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 180, (b) a base sequence obtained by adding, deleting, or substituting one or several bases in the base sequence of SEQ ID NO: 180, or (c) a base sequence having 80% or more sequence identity with the base sequence of SEQ ID NO: 180, or consists of any of the base sequences, and the second antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences,
the first antisense oligomer unit comprises (a) a base sequence of SEQ ID NO: 181, (b) a base sequence obtained by adding, deleting, or substituting one or several bases in the base sequence of SEQ ID NO: 181, or (c) a base sequence having 80% or more sequence identity with the base sequence of SEQ ID NO: 181, or consists of any of the base sequences, and the second antisense oligomer unit comprises (a) a base sequence of SEQ ID NO: 177, (b) a base sequence obtained by adding, deleting, or substituting one or several bases in the base sequence of SEQ ID NO: 177, or (c) a base sequence having 80% or more sequence identity with the base sequence of SEQ ID NO: 177, or consists of any of the base sequences,
the first antisense oligomer unit comprises (a) a base sequence of SEQ ID NO: 181, (b) a base sequence obtained by adding, deleting, or substituting one or several bases in the base sequence of SEQ ID NO: 181, or (c) a base sequence having 80% or more sequence identity with the base sequence of SEQ ID NO: 181, or consists of any of the base sequences, and the second antisense oligomer unit comprises (a) a base sequence of SEQ ID NO: 182, (b) a base sequence obtained by adding, deleting, or substituting one or several bases in the base sequence of SEQ ID NO: 182, or (c) a base sequence having 80% or more sequence identity with the base sequence of SEQ ID NO: 182, or consists of any of the base sequences,
the first antisense oligomer unit comprises (a) a base sequence of SEQ ID NO: 181, (b) a base sequence obtained by adding, deleting, or substituting one or several bases in the base sequence of SEQ ID NO: 181, or (c) a base sequence having 80% or more sequence identity with the base sequence of SEQ ID NO: 181, or consists of any of the base sequences, and the second antisense oligomer unit comprises (a) a base sequence of SEQ ID NO: 183, (b) a base sequence obtained by adding, deleting, or substituting one or several bases in the base sequence of SEQ ID NO: 183, or (c) a base sequence having 80% or more sequence identity with the base sequence of SEQ ID NO: 183, or consists of any of the base sequences,
the first antisense oligomer unit comprises (a) a base sequence of SEQ ID NO: 181, (b) a base sequence obtained by adding, deleting, or substituting one or several bases in the base sequence of SEQ ID NO: 181, or (c) a base sequence having 80% or more sequence identity with the base sequence of SEQ ID NO: 181, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 181, (b) a base sequence obtained by adding, deleting, or substituting one or several bases in the base sequence of SEQ ID NO: 181, or (c) a base sequence having 80% or more sequence identity with the base sequence of SEQ ID NO: 181, or consists of any of the base sequences, and the second antisense oligomer unit comprises (a) a base sequence of SEQ ID NO: 184, (b) a base sequence obtained by adding, deleting, or substituting one or several bases in the base sequence of SEQ ID NO: 184, or (c) a base sequence having 80% or more sequence identity with the base sequence of SEQ ID NO: 184, or consists of any of the base sequences,
the first antisense oligomer unit comprises (a) a base sequence of SEQ ID NO: 181, (b) a base sequence obtained by adding, deleting, or substituting one or several bases in the base sequence of SEQ ID NO: 181, or (c) a base sequence having 80% or more sequence identity with the base sequence of SEQ ID NO: 181, or consists of any of the base sequences, and the second antisense oligomer unit comprises (a) a base sequence of SEQ ID NO: 185, (b) a base sequence obtained by adding, deleting, or substituting one or several bases in the base sequence of SEQ ID NO: 185, or (c) a base sequence having 80% or more sequence identity with the base sequence of SEQ ID NO: 185, or consists of any of the base sequences,
the first antisense oligomer unit comprises (a) a base sequence of SEQ ID NO: 181, (b) a base sequence obtained by adding, deleting, or substituting one or several bases in the base sequence of SEQ ID NO: 181, or (c) a base sequence having 80% or more sequence identity with the base sequence of SEQ ID NO: 181, or consists of any of the base sequences, and the second antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences,
the first antisense oligomer unit comprises (a) a base sequence of SEQ ID NO: 186, (b) a base sequence obtained by adding, deleting, or substituting one or several bases in the base sequence of SEQ ID NO: 186, or (c) a base sequence having 80% or more sequence identity with the base sequence of SEQ ID NO: 186, or consists of any of the base sequences, and the second antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences,
the first antisense oligomer unit comprises (a) a base sequence of SEQ ID NO: 187, (b) a base sequence obtained by adding, deleting, or substituting one or several bases in the base sequence of SEQ ID NO: 187, or (c) a base sequence having 80% or more sequence identity with the base sequence of SEQ ID NO: 187, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 188, (b) a base sequence obtained by adding, deleting, or substituting one or several bases in the base sequence of SEQ ID NO: 188, or (c) a base sequence having 80% or more sequence identity with the base sequence of SEQ ID NO: 188, or consists of any of the base sequences, and the second antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences,
the first antisense oligomer unit comprises (a) a base sequence of SEQ ID NO: 189, (b) a base sequence obtained by adding, deleting, or substituting one or several bases in the base sequence of SEQ ID NO: 189, or (c) a base sequence having 80% or more sequence identity with the base sequence of SEQ ID NO: 189, or consists of any of the base sequences, and the second antisense oligomer unit comprises (a) a base sequence of SEQ ID NO: 190, (b) a base sequence obtained by adding, deleting, or substituting one or several bases in the base sequence of SEQ ID NO: 190, or (c) a base sequence having 80% or more sequence identity with the base sequence of SEQ ID NO: 190, or consists of any of the base sequences,
the first antisense oligomer unit comprises (a) a base sequence of SEQ ID NO: 189, (b) a base sequence obtained by adding, deleting, or substituting one or several bases in the base sequence of SEQ ID NO: 189, or (c) a base sequence having 80% or more sequence identity with the base sequence of SEQ ID NO: 189, or consists of any of the base sequences, and the second antisense oligomer unit comprises (a) a base sequence of SEQ ID NO: 191, (b) a base sequence obtained by adding, deleting, or substituting one or several bases in the base sequence of SEQ ID NO: 191, or (c) a base sequence having 80% or more sequence identity with the base sequence of SEQ ID NO: 191, or consists of any of the base sequences,
the first antisense oligomer unit comprises (a) a base sequence of SEQ ID NO: 189, (b) a base sequence obtained by adding, deleting, or substituting one or several bases in the base sequence of SEQ ID NO: 189, or (c) a base sequence having 80% or more sequence identity with the base sequence of SEQ ID NO: 189, or consists of any of the base sequences, and the second antisense oligomer unit comprises (a) a base sequence of SEQ ID NO: 192, (b) a base sequence obtained by adding, deleting, or substituting one or several bases in the base sequence of SEQ ID NO: 192, or (c) a base sequence having 80% or more sequence identity with the base sequence of SEQ ID NO: 192, or consists of any of the base sequences,
the first antisense oligomer unit comprises (a) a base sequence of SEQ ID NO: 189, (b) a base sequence obtained by adding, deleting, or substituting one or several bases in the base sequence of SEQ ID NO: 189, or (c) a base sequence having 80% or more sequence identity with the base sequence of SEQ ID NO: 189, or consists of any of the base sequences, and the second antisense oligomer unit comprises (a) a base sequence of SEQ ID NO: 193, (b) a base sequence obtained by adding, deleting, or substituting one or several bases in the base sequence of SEQ ID NO: 193, or (c) a base sequence having 80% or more sequence identity with the base sequence of SEQ ID NO: 193, or consists of any of the base sequences,
the first antisense oligomer unit comprises (a) a base sequence of SEQ ID NO: 189, (b) a base sequence obtained by adding, deleting, or substituting one or several bases in the base sequence of SEQ ID NO: 189, or (c) a base sequence having 80% or more sequence identity with the base sequence of SEQ ID NO: 189, or consists of any of the base sequences, and the second antisense oligomer unit comprises (a) a base sequence of SEQ ID NO: 194, (b) a base sequence obtained by adding, deleting, or substituting one or several bases in the base sequence of SEQ ID NO: 194, or (c) a base sequence having 80% or more sequence identity with the base sequence of SEQ ID NO: 194, or consists of any of the base sequences,
the first antisense oligomer unit comprises (a) a base sequence of SEQ ID NO: 177, (b) a base sequence obtained by adding, deleting, or substituting one or several bases in the base sequence of SEQ ID NO: 177, or (c) a base sequence having 80% or more sequence identity with the base sequence of SEQ ID NO: 177, or consists of any of the base sequences, and the second antisense oligomer unit comprises (a) a base sequence of SEQ ID NO: 191, (b) a base sequence obtained by adding, deleting, or substituting one or several bases in the base sequence of SEQ ID NO: 191, or (c) a base sequence having 80% or more sequence identity with the base sequence of SEQ ID NO: 191, or consists of any of the base sequences,
the first antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences, and the second antisense oligomer unit comprises (a) a base sequence of SEQ ID NO: 195, (b) a base sequence obtained by adding, deleting, or substituting one or several bases in the base sequence of SEQ ID NO: 195, or (c) a base sequence having 80% or more sequence identity with the base sequence of SEQ ID NO: 195, or consists of any of the base sequences,
the first antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences, and the second antisense oligomer unit comprises (a) a base sequence of SEQ ID NO: 196, (b) a base sequence obtained by adding, deleting, or substituting one or several bases in the base sequence of SEQ ID NO: 196, or (c) a base sequence having 80% or more sequence identity with the base sequence of SEQ ID NO: 196, or consists of any of the base sequences,
the first antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences, and the second antisense oligomer unit comprises (a) a base sequence of SEQ ID NO: 192, (b) a base sequence obtained by adding, deleting, or substituting one or several bases in the base sequence of SEQ ID NO: 192, or (c) a base sequence having 80% or more sequence identity with the base sequence of SEQ ID NO: 192, or consists of any of the base sequences,
the first antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences, and the second antisense oligomer unit comprises (a) a base sequence of SEQ ID NO: 197, (b) a base sequence obtained by adding, deleting, or substituting one or several bases in the base sequence of SEQ ID NO: 197, or (c) a base sequence having 80% or more sequence identity with the base sequence of SEQ ID NO: 197, or consists of any of the base sequences,
the first antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences, and the second antisense oligomer unit comprises (a) a base sequence of SEQ ID NO: 198, (b) a base sequence obtained by adding, deleting, or substituting one or several bases in the base sequence of SEQ ID NO: 198, or (c) a base sequence having 80% or more sequence identity with the base sequence of SEQ ID NO: 198, or consists of any of the base sequences,
the first antisense oligomer unit comprises (a) a base sequence of SEQ ID NO: 199, (b) a base sequence obtained by adding, deleting, or substituting one or several bases in the base sequence of SEQ ID NO: 199, or (c) a base sequence having 80% or more sequence identity with the base sequence of SEQ ID NO: 199, or consists of any of the base sequences, and the second antisense oligomer unit comprises (a) a base sequence of SEQ ID NO: 200, (b) a base sequence obtained by adding, deleting, or substituting one or several bases in the base sequence of SEQ ID NO: 200, or (c) a base sequence having 80% or more sequence identity with the base sequence of SEQ ID NO: 200, or consists of any of the base sequences,
the first antisense oligomer unit comprises (a) a base sequence of SEQ ID NO: 176, (b) a base sequence obtained by adding, deleting, or substituting one or several bases in the base sequence of SEQ ID NO: 176, or (c) a base sequence having 80% or more sequence identity with the base sequence of SEQ ID NO: 176, or consists of any of the base sequences, and the second antisense oligomer unit comprises (a) a base sequence of SEQ ID NO: 201, (b) a base sequence obtained by adding, deleting, or substituting one or several bases in the base sequence of SEQ ID NO: 201, or (c) a base sequence having 80% or more sequence identity with the base sequence of SEQ ID NO: 201, or consists of any of the base sequences,
the first antisense oligomer unit comprises (a) a base sequence of SEQ ID NO: 176, (b) a base sequence obtained by adding, deleting, or substituting one or several bases in the base sequence of SEQ ID NO: 176, or (c) a base sequence having 80% or more sequence identity with the base sequence of SEQ ID NO: 176, or consists of any of the base sequences, and the second antisense oligomer unit comprises (a) a base sequence of SEQ ID NO: 202, (b) a base sequence obtained by adding, deleting, or substituting one or several bases in the base sequence of SEQ ID NO: 202, or (c) a base sequence having 80% or more sequence identity with the base sequence of SEQ ID NO: 202, or consists of any of the base sequences,
the first antisense oligomer unit comprises (a) a base sequence of SEQ ID NO: 181, (b) a base sequence obtained by adding, deleting, or substituting one or several bases in the base sequence of SEQ ID NO: 181, or (c) a base sequence having 80% or more sequence identity with the base sequence of SEQ ID NO: 181, or consists of any of the base sequences, and the second antisense oligomer unit comprises (a) a base sequence of SEQ ID NO: 201, (b) a base sequence obtained by adding, deleting, or substituting one or several bases in the base sequence of SEQ ID NO: 201, or (c) a base sequence having 80% or more sequence identity with the base sequence of SEQ ID NO: 201, or consists of any of the base sequences,
the first antisense oligomer unit comprises (a) a base sequence of SEQ ID NO: 181, (b) a base sequence obtained by adding, deleting, or substituting one or several bases in the base sequence of SEQ ID NO: 181, or (c) a base sequence having 80% or more sequence identity with the base sequence of SEQ ID NO: 181, or consists of any of the base sequences, and the second antisense oligomer unit comprises (a) a base sequence of SEQ ID NO: 202, (b) a base sequence obtained by adding, deleting, or substituting one or several bases in the base sequence of SEQ ID NO: 202, or (c) a base sequence having 80% or more sequence identity with the base sequence of SEQ ID NO: 202, or consists of any of the base sequences,
the first antisense oligomer unit comprises (a) a base sequence of SEQ ID NO: 187, (b) a base sequence obtained by adding, deleting, or substituting one or several bases in the base sequence of SEQ ID NO: 187, or (c) a base sequence having 80% or more sequence identity with the base sequence of SEQ ID NO: 187, or consists of any of the base sequences, and the second antisense oligomer unit comprises (a) a base sequence of SEQ ID NO: 202, (b) a base sequence obtained by adding, deleting, or substituting one or several bases in the base sequence of SEQ ID NO: 202, or (c) a base sequence having 80% or more sequence identity with the base sequence of SEQ ID NO: 202, or consists of any of the base sequences,
the first antisense oligomer unit comprises (a) a base sequence of SEQ ID NO: 203, (b) a base sequence obtained by adding, deleting, or substituting one or several bases in the base sequence of SEQ ID NO: 203, or (c) a base sequence having 80% or more sequence identity with the base sequence of SEQ ID NO: 203, or consists of any of the base sequences, and the second antisense oligomer unit comprises (a) a base sequence of SEQ ID NO: 201, (b) a base sequence obtained by adding, deleting, or substituting one or several bases in the base sequence of SEQ ID NO: 201, or (c) a base sequence having 80% or more sequence identity with the base sequence of SEQ ID NO: 201, or consists of any of the base sequences,
the first antisense oligomer unit comprises (a) a base sequence of SEQ ID NO: 203, (b) a base sequence obtained by adding, deleting, or substituting one or several bases in the base sequence of SEQ ID NO: 203, or (c) a base sequence having 80% or more sequence identity with the base sequence of SEQ ID NO: 203, or consists of any of the base sequences, and the second antisense oligomer unit comprises (a) a base sequence of SEQ ID NO: 202, (b) a base sequence obtained by adding, deleting, or substituting one or several bases in the base sequence of SEQ ID NO: 202, or (c) a base sequence having 80% or more sequence identity with the base sequence of SEQ ID NO: 202, or consists of any of the base sequences,
the first antisense oligomer unit comprises (a) a base sequence of SEQ ID NO: 189, (b) a base sequence obtained by adding, deleting, or substituting one or several bases in the base sequence of SEQ ID NO: 189, or (c) a base sequence having 80% or more sequence identity with the base sequence of SEQ ID NO: 189, or consists of any of the base sequences, and the second antisense oligomer unit comprises (a) a base sequence of SEQ ID NO: 201, (b) a base sequence obtained by adding, deleting, or substituting one or several bases in the base sequence of SEQ ID NO: 201, or (c) a base sequence having 80% or more sequence identity with the base sequence of SEQ ID NO: 201, or consists of any of the base sequences,
the first antisense oligomer unit comprises (a) a base sequence of SEQ ID NO: 189, (b) a base sequence obtained by adding, deleting, or substituting one or several bases in the base sequence of SEQ ID NO: 189, or (c) a base sequence having 80% or more sequence identity with the base sequence of SEQ ID NO: 189, or consists of any of the base sequences, and the second antisense oligomer unit comprises (a) a base sequence of SEQ ID NO: 202, (b) a base sequence obtained by adding, deleting, or substituting one or several bases in the base sequence of SEQ ID NO: 202, or (c) a base sequence having 80% or more sequence identity with the base sequence of SEQ ID NO: 202, or consists of any of the base sequences,
the first antisense oligomer unit comprises (a) a base sequence of SEQ ID NO: 204, (b) a base sequence obtained by adding, deleting, or substituting one or several bases in the base sequence of SEQ ID NO: 204, or (c) a base sequence having 80% or more sequence identity with the base sequence of SEQ ID NO: 204, or consists of any of the base sequences, and the second antisense oligomer unit comprises (a) a base sequence of SEQ ID NO: 202, (b) a base sequence obtained by adding, deleting, or substituting one or several bases in the base sequence of SEQ ID NO: 202, or (c) a base sequence having 80% or more sequence identity with the base sequence of SEQ ID NO: 202, or consists of any of the base sequences,
the first antisense oligomer unit comprises (a) a base sequence of SEQ ID NO: 177, (b) a base sequence obtained by adding, deleting, or substituting one or several bases in the base sequence of SEQ ID NO: 177, or (c) a base sequence having 80% or more sequence identity with the base sequence of SEQ ID NO: 177, or consists of any of the base sequences, and the second antisense oligomer unit comprises (a) a base sequence of SEQ ID NO: 201, (b) a base sequence obtained by adding, deleting, or substituting one or several bases in the base sequence of SEQ ID NO: 201, or (c) a base sequence having 80% or more sequence identity with the base sequence of SEQ ID NO: 201, or consists of any of the base sequences,
the first antisense oligomer unit comprises (a) a base sequence of SEQ ID NO: 177, (b) a base sequence obtained by adding, deleting, or substituting one or several bases in the base sequence of SEQ ID NO: 177, or (c) a base sequence having 80% or more sequence identity with the base sequence of SEQ ID NO: 177, or consists of any of the base sequences, and the second antisense oligomer unit comprises (a) a base sequence of SEQ ID NO: 205, (b) a base sequence obtained by adding, deleting, or substituting one or several bases in the base sequence of SEQ ID NO: 205, or (c) a base sequence having 80% or more sequence identity with the base sequence of SEQ ID NO: 205, or consists of any of the base sequences,
the first antisense oligomer unit comprises (a) a base sequence of SEQ ID NO: 177, (b) a base sequence obtained by adding, deleting, or substituting one or several bases in the base sequence of SEQ ID NO: 177, or (c) a base sequence having 80% or more sequence identity with the base sequence of SEQ ID NO: 177, or consists of any of the base sequences, and the second antisense oligomer unit comprises (a) a base sequence of SEQ ID NO: 202, (b) a base sequence obtained by adding, deleting, or substituting one or several bases in the base sequence of SEQ ID NO: 202, or (c) a base sequence having 80% or more sequence identity with the base sequence of SEQ ID NO: 202, or consists of any of the base sequences,
the first antisense oligomer unit comprises (a) a base sequence of SEQ ID NO: 183, (b) a base sequence obtained by adding, deleting, or substituting one or several bases in the base sequence of SEQ ID NO: 183, or (c) a base sequence having 80% or more sequence identity with the base sequence of SEQ ID NO: 183, or consists of any of the base sequences, and the second antisense oligomer unit comprises (a) a base sequence of SEQ ID NO: 202, (b) a base sequence obtained by adding, deleting, or substituting one or several bases in the base sequence of SEQ ID NO: 202, or (c) a base sequence having 80% or more sequence identity with the base sequence of SEQ ID NO: 202, or consists of any of the base sequences,
the first antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences, and the second antisense oligomer unit comprises (a) a base sequence of SEQ ID NO: 206, (b) a base sequence obtained by adding, deleting, or substituting one or several bases in the base sequence of SEQ ID NO: 206, or (c) a base sequence having 80% or more sequence identity with the base sequence of SEQ ID NO: 206, or consists of any of the base sequences,
the first antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences, and the second antisense oligomer unit comprises (a) a base sequence of SEQ ID NO: 201, (b) a base sequence obtained by adding, deleting, or substituting one or several bases in the base sequence of SEQ ID NO: 201, or (c) a base sequence having 80% or more sequence identity with the base sequence of SEQ ID NO: 201, or consists of any of the base sequences,
the first antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences, and the second antisense oligomer unit comprises (a) a base sequence of SEQ ID NO: 202, (b) a base sequence obtained by adding, deleting, or substituting one or several bases in the base sequence of SEQ ID NO: 202, or (c) a base sequence having 80% or more sequence identity with the base sequence of SEQ ID NO: 202, or consists of any of the base sequences,
the first antisense oligomer unit comprises (a) a base sequence of SEQ ID NO: 185, (b) a base sequence obtained by adding, deleting, or substituting one or several bases in the base sequence of SEQ ID NO: 185, or (c) a base sequence having 80% or more sequence identity with the base sequence of SEQ ID NO: 185, or consists of any of the base sequences, and the second antisense oligomer unit comprises (a) a base sequence of SEQ ID NO: 201, (b) a base sequence obtained by adding, deleting, or substituting one or several bases in the base sequence of SEQ ID NO: 201, or (c) a base sequence having 80% or more sequence identity with the base sequence of SEQ ID NO: 201, or consists of any of the base sequences,
the first antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences, and the second antisense oligomer unit comprises (a) a base sequence of SEQ ID NO: 201, (b) a base sequence obtained by adding, deleting, or substituting one or several bases in the base sequence of SEQ ID NO: 201, or (c) a base sequence having 80% or more sequence identity with the base sequence of SEQ ID NO: 201, or consists of any of the base sequences,
the first antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences, and the second antisense oligomer unit comprises (a) a base sequence of SEQ ID NO: 202, (b) a base sequence obtained by adding, deleting, or substituting one or several bases in the base sequence of SEQ ID NO: 202, or (c) a base sequence having 80% or more sequence identity with the base sequence of SEQ ID NO: 202, or consists of any of the base sequences,
the first antisense oligomer unit comprises (a) a base sequence of SEQ ID NO: 207, (b) a base sequence obtained by adding, deleting, or substituting one or several bases in the base sequence of SEQ ID NO: 207, or (c) a base sequence having 80% or more sequence identity with the base sequence of SEQ ID NO: 207, or consists of any of the base sequences, and the second antisense oligomer unit comprises (a) a base sequence of SEQ ID NO: 201, (b) a base sequence obtained by adding, deleting, or substituting one or several bases in the base sequence of SEQ ID NO: 201, or (c) a base sequence having 80% or more sequence identity with the base sequence of SEQ ID NO: 201, or consists of any of the base sequences,
the first antisense oligomer unit comprises (a) a base sequence of SEQ ID NO: 176, (b) a base sequence obtained by adding, deleting, or substituting one or several bases in the base sequence of SEQ ID NO: 176, or (c) a base sequence having 80% or more sequence identity with the base sequence of SEQ ID NO: 176, or consists of any of the base sequences, and the second antisense oligomer unit comprises (a) a base sequence of SEQ ID NO: 208, (b) a base sequence obtained by adding, deleting, or substituting one or several bases in the base sequence of SEQ ID NO: 208, or (c) a base sequence having 80% or more sequence identity with the base sequence of SEQ ID NO: 208, or consists of any of the base sequences,
the first antisense oligomer unit comprises (a) a base sequence of SEQ ID NO: 176, (b) a base sequence obtained by adding, deleting, or substituting one or several bases in the base sequence of SEQ ID NO: 176, or (c) a base sequence having 80% or more sequence identity with the base sequence of SEQ ID NO: 176, or consists of any of the base sequences, and the second antisense oligomer unit comprises (a) a base sequence of SEQ ID NO: 209, (b) a base sequence obtained by adding, deleting, or substituting one or several bases in the base sequence of SEQ ID NO: 209, or (c) a base sequence having 80% or more sequence identity with the base sequence of SEQ ID NO: 209, or consists of any of the base sequences,
the first antisense oligomer unit comprises (a) a base sequence of SEQ ID NO: 176, (b) a base sequence obtained by adding, deleting, or substituting one or several bases in the base sequence of SEQ ID NO: 176, or (c) a base sequence having 80% or more sequence identity with the base sequence of SEQ ID NO: 176, or consists of any of the base sequences, and the second antisense oligomer unit comprises (a) a base sequence of SEQ ID NO: 210, (b) a base sequence obtained by adding, deleting, or substituting one or several bases in the base sequence of SEQ ID NO: 210, or (c) a base sequence having 80% or more sequence identity with the base sequence of SEQ ID NO: 210, or consists of any of the base sequences,
the first antisense oligomer unit comprises (a) a base sequence of SEQ ID NO: 211, (b) a base sequence obtained by adding, deleting, or substituting one or several bases in the base sequence of SEQ ID NO: 211, or (c) a base sequence having 80% or more sequence identity with the base sequence of SEQ ID NO: 211, or consists of any of the base sequences, and the second antisense oligomer unit comprises (a) a base sequence of SEQ ID NO: 208, (b) a base sequence obtained by adding, deleting, or substituting one or several bases in the base sequence of SEQ ID NO: 208, or (c) a base sequence having 80% or more sequence identity with the base sequence of SEQ ID NO: 208, or consists of any of the base sequences,
the first antisense oligomer unit comprises (a) a base sequence of SEQ ID NO: 211, (b) a base sequence obtained by adding, deleting, or substituting one or several bases in the base sequence of SEQ ID NO: 211, or (c) a base sequence having 80% or more sequence identity with the base sequence of SEQ ID NO: 211, or consists of any of the base sequences, and the second antisense oligomer unit comprises (a) a base sequence of SEQ ID NO: 209, (b) a base sequence obtained by adding, deleting, or substituting one or several bases in the base sequence of SEQ ID NO: 209, or (c) a base sequence having 80% or more sequence identity with the base sequence of SEQ ID NO: 209, or consists of any of the base sequences,
the first antisense oligomer unit comprises (a) a base sequence of SEQ ID NO: 211, (b) a base sequence obtained by adding, deleting, or substituting one or several bases in the base sequence of SEQ ID NO: 211, or (c) a base sequence having 80% or more sequence identity with the base sequence of SEQ ID NO: 211, or consists of any of the base sequences, and the second antisense oligomer unit comprises (a) a base sequence of SEQ ID NO: 210, (b) a base sequence obtained by adding, deleting, or substituting one or several bases in the base sequence of SEQ ID NO: 210, or (c) a base sequence having 80% or more sequence identity with the base sequence of SEQ ID NO: 210, or consists of any of the base sequences,
the first antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences, and the second antisense oligomer unit comprises (a) a base sequence of SEQ ID NO: 212, (b) a base sequence obtained by adding, deleting, or substituting one or several bases in the base sequence of SEQ ID NO: 212, or (c) a base sequence having 80% or more sequence identity with the base sequence of SEQ ID NO: 212, or consists of any of the base sequences,
the first antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences, and the second antisense oligomer unit comprises (a) a base sequence of SEQ ID NO: 213, (b) a base sequence obtained by adding, deleting, or substituting one or several bases in the base sequence of SEQ ID NO: 213, or (c) a base sequence having 80% or more sequence identity with the base sequence of SEQ ID NO: 213, or consists of any of the base sequences,
the first antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences, and the second antisense oligomer unit comprises (a) a base sequence of SEQ ID NO: 214, (b) a base sequence obtained by adding, deleting, or substituting one or several bases in the base sequence of SEQ ID NO: 214, or (c) a base sequence having 80% or more sequence identity with the base sequence of SEQ ID NO: 214, or consists of any of the base sequences,
the first antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences, and the second antisense oligomer unit comprises (a) a base sequence of SEQ ID NO: 215, (b) a base sequence obtained by adding, deleting, or substituting one or several bases in the base sequence of SEQ ID NO: 215, or (c) a base sequence having 80% or more sequence identity with the base sequence of SEQ ID NO: 215, or consists of any of the base sequences,
the first antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences, and the second antisense oligomer unit comprises (a) a base sequence of SEQ ID NO: 208, (b) a base sequence obtained by adding, deleting, or substituting one or several bases in the base sequence of SEQ ID NO: 208, or (c) a base sequence having 80% or more sequence identity with the base sequence of SEQ ID NO: 208, or consists of any of the base sequences,
the first antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences, and the second antisense oligomer unit comprises (a) a base sequence of SEQ ID NO: 209, (b) a base sequence obtained by adding, deleting, or substituting one or several bases in the base sequence of SEQ ID NO: 209, or (c) a base sequence having 80% or more sequence identity with the base sequence of SEQ ID NO: 209, or consists of any of the base sequences,
the first antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences, and the second antisense oligomer unit comprises (a) a base sequence of SEQ ID NO: 210, (b) a base sequence obtained by adding, deleting, or substituting one or several bases in the base sequence of SEQ ID NO: 210, or (c) a base sequence having 80% or more sequence identity with the base sequence of SEQ ID NO: 210, or consists of any of the base sequences,
the first antisense oligomer unit comprises (a) a base sequence of SEQ ID NO: 199, (b) a base sequence obtained by adding, deleting, or substituting one or several bases in the base sequence of SEQ ID NO: 199, or (c) a base sequence having 80% or more sequence identity with the base sequence of SEQ ID NO: 199, or consists of any of the base sequences, and the second antisense oligomer unit comprises (a) a base sequence of SEQ ID NO: 216, (b) a base sequence obtained by adding, deleting, or substituting one or several bases in the base sequence of SEQ ID NO: 216, or (c) a base sequence having 80% or more sequence identity with the base sequence of SEQ ID NO: 216, or consists of any of the base sequences,
the first antisense oligomer unit comprises (a) a base sequence of SEQ ID NO: 199, (b) a base sequence obtained by adding, deleting, or substituting one or several bases in the base sequence of SEQ ID NO: 199, or (c) a base sequence having 80% or more sequence identity with the base sequence of SEQ ID NO: 199, or consists of any of the base sequences, and the second antisense oligomer unit comprises (a) a base sequence of SEQ ID NO: 217, (b) a base sequence obtained by adding, deleting, or substituting one or several bases in the base sequence of SEQ ID NO: 217, or (c) a base sequence having 80% or more sequence identity with the base sequence of SEQ ID NO: 217, or consists of any of the base sequences,
the first antisense oligomer unit comprises (a) a base sequence of SEQ ID NO: 199, (b) a base sequence obtained by adding, deleting, or substituting one or several bases in the base sequence of SEQ ID NO: 199, or (c) a base sequence having 80% or more sequence identity with the base sequence of SEQ ID NO: 199, or consists of any of the base sequences, and the second antisense oligomer unit comprises (a) a base sequence of SEQ ID NO: 218, (b) a base sequence obtained by adding, deleting, or substituting one or several bases in the base sequence of SEQ ID NO: 218, or (c) a base sequence having 80% or more sequence identity with the base sequence of SEQ ID NO: 218, or consists of any of the base sequences,
the first antisense oligomer unit comprises (a) a base sequence of SEQ ID NO: 196, (b) a base sequence obtained by adding, deleting, or substituting one or several bases in the base sequence of SEQ ID NO: 196, or (c) a base sequence having 80% or more sequence identity with the base sequence of SEQ ID NO: 196, or consists of any of the base sequences, and the second antisense oligomer unit comprises (a) a base sequence of SEQ ID NO: 210, (b) a base sequence obtained by adding, deleting, or substituting one or several bases in the base sequence of SEQ ID NO: 210, or (c) a base sequence having 80% or more sequence identity with the base sequence of SEQ ID NO: 210, or consists of any of the base sequences,
the first antisense oligomer unit comprises (a) a base sequence of SEQ ID NO: 219, (b) a base sequence obtained by adding, deleting, or substituting one or several bases in the base sequence of SEQ ID NO: 219, or (c) a base sequence having 80% or more sequence identity with the base sequence of SEQ ID NO: 219, or consists of any of the base sequences, and the second antisense oligomer unit comprises (a) a base sequence of SEQ ID NO: 220, (b) a base sequence obtained by adding, deleting, or substituting one or several bases in the base sequence of SEQ ID NO: 220, or (c) a base sequence having 80% or more sequence identity with the base sequence of SEQ ID NO: 220, or consists of any of the base sequences,
the first antisense oligomer unit comprises (a) a base sequence of SEQ ID NO: 219, (b) a base sequence obtained by adding, deleting, or substituting one or several bases in the base sequence of SEQ ID NO: 219, or (c) a base sequence having 80% or more sequence identity with the base sequence of SEQ ID NO: 219, or consists of any of the base sequences, and the second antisense oligomer unit comprises (a) a base sequence of SEQ ID NO: 221, (b) a base sequence obtained by adding, deleting, or substituting one or several bases in the base sequence of SEQ ID NO: 221, or (c) a base sequence having 80% or more sequence identity with the base sequence of SEQ ID NO: 221, or consists of any of the base sequences,
the first antisense oligomer unit comprises (a) a base sequence of SEQ ID NO: 219, (b) a base sequence obtained by adding, deleting, or substituting one or several bases in the base sequence of SEQ ID NO: 219, or (c) a base sequence having 80% or more sequence identity with the base sequence of SEQ ID NO: 219, or consists of any of the base sequences, and the second antisense oligomer unit comprises (a) a base sequence of SEQ ID NO: 222, (b) a base sequence obtained by adding, deleting, or substituting one or several bases in the base sequence of SEQ ID NO: 222, or (c) a base sequence having 80% or more sequence identity with the base sequence of SEQ ID NO: 222, or consists of any of the base sequences,
the first antisense oligomer unit comprises (a) a base sequence of SEQ ID NO: 223, (b) a base sequence obtained by adding, deleting, or substituting one or several bases in the base sequence of SEQ ID NO: 223, or (c) a base sequence having 80% or more sequence identity with the base sequence of SEQ ID NO: 223, or consists of any of the base sequences, and the second antisense oligomer unit comprises (a) a base sequence of SEQ ID NO: 224, (b) a base sequence obtained by adding, deleting, or substituting one or several bases in the base sequence of SEQ ID NO: 224, or (c) a base sequence having 80% or more sequence identity with the base sequence of SEQ ID NO: 224, or consists of any of the base sequences,
the first antisense oligomer unit comprises (a) a base sequence of SEQ ID NO: 223, (b) a base sequence obtained by adding, deleting, or substituting one or several bases in the base sequence of SEQ ID NO: 223, or (c) a base sequence having 80% or more sequence identity with the base sequence of SEQ ID NO: 223, or consists of any of the base sequences, and the second antisense oligomer unit comprises (a) a base sequence of SEQ ID NO: 225, (b) a base sequence obtained by adding, deleting, or substituting one or several bases in the base sequence of SEQ ID NO: 225, or (c) a base sequence having 80% or more sequence identity with the base sequence of SEQ ID NO: 225, or consists of any of the base sequences,
the first antisense oligomer unit comprises (a) a base sequence of SEQ ID NO: 223, (b) a base sequence obtained by adding, deleting, or substituting one or several bases in the base sequence of SEQ ID NO: 223, or (c) a base sequence having 80% or more sequence identity with the base sequence of SEQ ID NO: 223, or consists of any of the base sequences, and the second antisense oligomer unit comprises (a) a base sequence of SEQ ID NO: 226, (b) a base sequence obtained by adding, deleting, or substituting one or several bases in the base sequence of SEQ ID NO: 226, or (c) a base sequence having 80% or more sequence identity with the base sequence of SEQ ID NO: 226, or consists of any of the base sequences,
the first antisense oligomer unit comprises (a) a base sequence of SEQ ID NO: 223, (b) a base sequence obtained by adding, deleting, or substituting one or several bases in the base sequence of SEQ ID NO: 223, or (c) a base sequence having 80% or more sequence identity with the base sequence of SEQ ID NO: 223, or consists of any of the base sequences, and the second antisense oligomer unit comprises (a) a base sequence of SEQ ID NO: 227, (b) a base sequence obtained by adding, deleting, or substituting one or several bases in the base sequence of SEQ ID NO: 227, or (c) a base sequence having 80% or more sequence identity with the base sequence of SEQ ID NO: 227, or consists of any of the base sequences,
the first antisense oligomer unit comprises (a) a base sequence of SEQ ID NO: 223, (b) a base sequence obtained by adding, deleting, or substituting one or several bases in the base sequence of SEQ ID NO: 223, or (c) a base sequence having 80% or more sequence identity with the base sequence of SEQ ID NO: 223, or consists of any of the base sequences, and the second antisense oligomer unit comprises (a) a base sequence of SEQ ID NO: 228, (b) a base sequence obtained by adding, deleting, or substituting one or several bases in the base sequence of SEQ ID NO: 228, or (c) a base sequence having 80% or more sequence identity with the base sequence of SEQ ID NO: 228, or consists of any of the base sequences,
the first antisense oligomer unit comprises (a) a base sequence of SEQ ID NO: 224, (b) a base sequence obtained by adding, deleting, or substituting one or several bases in the base sequence of SEQ ID NO: 224, or (c) a base sequence having 80% or more sequence identity with the base sequence of SEQ ID NO: 224, or consists of any of the base sequences, and the second antisense oligomer unit comprises (a) a base sequence of SEQ ID NO: 229, (b) a base sequence obtained by adding, deleting, or substituting one or several bases in the base sequence of SEQ ID NO: 229, or (c) a base sequence having 80% or more sequence identity with the base sequence of SEQ ID NO: 229, or consists of any of the base sequences,
the first antisense oligomer unit comprises (a) a base sequence of SEQ ID NO: 224, (b) a base sequence obtained by adding, deleting, or substituting one or several bases in the base sequence of SEQ ID NO: 224, or (c) a base sequence having 80% or more sequence identity with the base sequence of SEQ ID NO: 224, or consists of any of the base sequences, and the second antisense oligomer unit comprises (a) a base sequence of SEQ ID NO: 226, (b) a base sequence obtained by adding, deleting, or substituting one or several bases in the base sequence of SEQ ID NO: 226, or (c) a base sequence having 80% or more sequence identity with the base sequence of SEQ ID NO: 226, or consists of any of the base sequences,
the first antisense oligomer unit comprises (a) a base sequence of SEQ ID NO: 224, (b) a base sequence obtained by adding, deleting, or substituting one or several bases in the base sequence of SEQ ID NO: 224, or (c) a base sequence having 80% or more sequence identity with the base sequence of SEQ ID NO: 224, or consists of any of the base sequences, and the second antisense oligomer unit comprises (a) a base sequence of SEQ ID NO: 227, (b) a base sequence obtained by adding, deleting, or substituting one or several bases in the base sequence of SEQ ID NO: 227, or (c) a base sequence having 80% or more sequence identity with the base sequence of SEQ ID NO: 227, or consists of any of the base sequences,
the first antisense oligomer unit comprises (a) a base sequence of SEQ ID NO: 224, (b) a base sequence obtained by adding, deleting, or substituting one or several bases in the base sequence of SEQ ID NO: 224, or (c) a base sequence having 80% or more sequence identity with the base sequence of SEQ ID NO: 224, or consists of any of the base sequences, and the second antisense oligomer unit comprises (a) a base sequence of SEQ ID NO: 228, (b) a base sequence obtained by adding, deleting, or substituting one or several bases in the base sequence of SEQ ID NO: 228, or (c) a base sequence having 80% or more sequence identity with the base sequence of SEQ ID NO: 228, or consists of any of the base sequences,
the first antisense oligomer unit comprises (a) a base sequence of SEQ ID NO: 230, (b) a base sequence obtained by adding, deleting, or substituting one or several bases in the base sequence of SEQ ID NO: 230, or (c) a base sequence having 80% or more sequence identity with the base sequence of SEQ ID NO: 230, or consists of any of the base sequences, and the second antisense oligomer unit comprises (a) a base sequence of SEQ ID NO: 228, (b) a base sequence obtained by adding, deleting, or substituting one or several bases in the base sequence of SEQ ID NO: 228, or (c) a base sequence having 80% or more sequence identity with the base sequence of SEQ ID NO: 228, or consists of any of the base sequences,
the first antisense oligomer unit comprises (a) a base sequence of SEQ ID NO: 231, (b) a base sequence obtained by adding, deleting, or substituting one or several bases in the base sequence of SEQ ID NO: 231, or (c) a base sequence having 80% or more sequence identity with the base sequence of SEQ ID NO: 231, or consists of any of the base sequences, and the second antisense oligomer unit comprises (a) a base sequence of SEQ ID NO: 228, (b) a base sequence obtained by adding, deleting, or substituting one or several bases in the base sequence of SEQ ID NO: 228, or (c) a base sequence having 80% or more sequence identity with the base sequence of SEQ ID NO: 228, or consists of any of the base sequences,
the first antisense oligomer unit comprises (a) a base sequence of SEQ ID NO: 232, (b) a base sequence obtained by adding, deleting, or substituting one or several bases in the base sequence of SEQ ID NO: 232, or (c) a base sequence having 80% or more sequence identity with the base sequence of SEQ ID NO: 232, or consists of any of the base sequences, and the second antisense oligomer unit comprises (a) a base sequence of SEQ ID NO: 228, (b) a base sequence obtained by adding, deleting, or substituting one or several bases in the base sequence of SEQ ID NO: 228, or (c) a base sequence having 80% or more sequence identity with the base sequence of SEQ ID NO: 228, or consists of any of the base sequences,
the first antisense oligomer unit comprises (a) a base sequence of SEQ ID NO: 225, (b) a base sequence obtained by adding, deleting, or substituting one or several bases in the base sequence of SEQ ID NO: 225, or (c) a base sequence having 80% or more sequence identity with the base sequence of SEQ ID NO: 225, or consists of any of the base sequences, and the second antisense oligomer unit comprises (a) a base sequence of SEQ ID NO: 227, (b) a base sequence obtained by adding, deleting, or substituting one or several bases in the base sequence of SEQ ID NO: 227, or (c) a base sequence having 80% or more sequence identity with the base sequence of SEQ ID NO: 227, or consists of any of the base sequences,
the first antisense oligomer unit comprises (a) a base sequence of SEQ ID NO: 225, (b) a base sequence obtained by adding, deleting, or substituting one or several bases in the base sequence of SEQ ID NO: 225, or (c) a base sequence having 80% or more sequence identity with the base sequence of SEQ ID NO: 225, or consists of any of the base sequences, and the second antisense oligomer unit comprises (a) a base sequence of SEQ ID NO: 228, (b) a base sequence obtained by adding, deleting, or substituting one or several bases in the base sequence of SEQ ID NO: 228, or (c) a base sequence having 80% or more sequence identity with the base sequence of SEQ ID NO: 228, or consists of any of the base sequences,
the first antisense oligomer unit comprises (a) a base sequence of SEQ ID NO: 233, (b) a base sequence obtained by adding, deleting, or substituting one or several bases in the base sequence of SEQ ID NO: 233, or (c) a base sequence having 80% or more sequence identity with the base sequence of SEQ ID NO: 233, or consists of any of the base sequences, and the second antisense oligomer unit comprises (a) a base sequence of SEQ ID NO: 228, (b) a base sequence obtained by adding, deleting, or substituting one or several bases in the base sequence of SEQ ID NO: 228, or (c) a base sequence having 80% or more sequence identity with the base sequence of SEQ ID NO: 228, or consists of any of the base sequences,
the first antisense oligomer unit comprises (a) a base sequence of SEQ ID NO: 234, (b) a base sequence obtained by adding, deleting, or substituting one or several bases in the base sequence of SEQ ID NO: 234, or (c) a base sequence having 80% or more sequence identity with the base sequence of SEQ ID NO: 234, or consists of any of the base sequences, and the second antisense oligomer unit comprises (a) a base sequence of SEQ ID NO: 228, (b) a base sequence obtained by adding, deleting, or substituting one or several bases in the base sequence of SEQ ID NO: 228, or (c) a base sequence having 80% or more sequence identity with the base sequence of SEQ ID NO: 228, or consists of any of the base sequences,
the first antisense oligomer unit comprises (a) a base sequence of SEQ ID NO: 235, (b) a base sequence obtained by adding, deleting, or substituting one or several bases in the base sequence of SEQ ID NO: 235, or (c) a base sequence having 80% or more sequence identity with the base sequence of SEQ ID NO: 235, or consists of any of the base sequences, and the second antisense oligomer unit comprises (a) a base sequence of SEQ ID NO: 228, (b) a base sequence obtained by adding, deleting, or substituting one or several bases in the base sequence of SEQ ID NO: 228, or (c) a base sequence having 80% or more sequence identity with the base sequence of SEQ ID NO: 228, or consists of any of the base sequences,
the first antisense oligomer unit comprises (a) a base sequence of SEQ ID NO: 236, (b) a base sequence obtained by adding, deleting, or substituting one or several bases in the base sequence of SEQ ID NO: 236, or (c) a base sequence having 80% or more sequence identity with the base sequence of SEQ ID NO: 236, or consists of any of the base sequences, and the second antisense oligomer unit comprises (a) a base sequence of SEQ ID NO: 228, (b) a base sequence obtained by adding, deleting, or substituting one or several bases in the base sequence of SEQ ID NO: 228, or (c) a base sequence having 80% or more sequence identity with the base sequence of SEQ ID NO: 228, or consists of any of the base sequences,
the first antisense oligomer unit comprises (a) a base sequence of SEQ ID NO: 237, (b) a base sequence obtained by adding, deleting, or substituting one or several bases in the base sequence of SEQ ID NO: 237, or (c) a base sequence having 80% or more sequence identity with the base sequence of SEQ ID NO: 237, or consists of any of the base sequences, and the second antisense oligomer unit comprises (a) a base sequence of SEQ ID NO: 228, (b) a base sequence obtained by adding, deleting, or substituting one or several bases in the base sequence of SEQ ID NO: 228, or (c) a base sequence having 80% or more sequence identity with the base sequence of SEQ ID NO: 228, or consists of any of the base sequences,
the first antisense oligomer unit comprises (a) a base sequence of SEQ ID NO: 226, (b) a base sequence obtained by adding, deleting, or substituting one or several bases in the base sequence of SEQ ID NO: 226, or (c) a base sequence having 80% or more sequence identity with the base sequence of SEQ ID NO: 226, or consists of any of the base sequences, and the second antisense oligomer unit comprises (a) a base sequence of SEQ ID NO: 227, (b) a base sequence obtained by adding, deleting, or substituting one or several bases in the base sequence of SEQ ID NO: 227, or (c) a base sequence having 80% or more sequence identity with the base sequence of SEQ ID NO: 227, or consists of any of the base sequences,
the first antisense oligomer unit comprises (a) a base sequence of SEQ ID NO: 226, (b) a base sequence obtained by adding, deleting, or substituting one or several bases in the base sequence of SEQ ID NO: 226, or (c) a base sequence having 80% or more sequence identity with the base sequence of SEQ ID NO: 226, or consists of any of the base sequences, and the second antisense oligomer unit comprises (a) a base sequence of SEQ ID NO: 228, (b) a base sequence obtained by adding, deleting, or substituting one or several bases in the base sequence of SEQ ID NO: 228, or (c) a base sequence having 80% or more sequence identity with the base sequence of SEQ ID NO: 228, or consists of any of the base sequences,
the first antisense oligomer unit comprises (a) a base sequence of SEQ ID NO: 227, (b) a base sequence obtained by adding, deleting, or substituting one or several bases in the base sequence of SEQ ID NO: 227, or (c) a base sequence having 80% or more sequence identity with the base sequence of SEQ ID NO: 227, or consists of any of the base sequences, and the second antisense oligomer unit comprises (a) a base sequence of SEQ ID NO: 228, (b) a base sequence obtained by adding, deleting, or substituting one or several bases in the base sequence of SEQ ID NO: 228, or (c) a base sequence having 80% or more sequence identity with the base sequence of SEQ ID NO: 228, or consists of any of the base sequences,
the first antisense oligomer unit comprises (a) a base sequence of SEQ ID NO: 238, (b) a base sequence obtained by adding, deleting, or substituting one or several bases in the base sequence of SEQ ID NO: 238, or (c) a base sequence having 80% or more sequence identity with the base sequence of SEQ ID NO: 238, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 239, (b) a base sequence obtained by adding, deleting, or substituting one or several bases in the base sequence of SEQ ID NO: 239, or (c) a base sequence having 80% or more sequence identity with the base sequence of SEQ ID NO: 239, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 239, (b) a base sequence obtained by adding, deleting, or substituting one or several bases in the base sequence of SEQ ID NO: 239, or (c) a base sequence having 80% or more sequence identity with the base sequence of SEQ ID NO: 239, or consists of any of the base sequences, and the second antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences,
the first antisense oligomer unit comprises (a) a base sequence of SEQ ID NO: 240, (b) a base sequence obtained by adding, deleting, or substituting one or several bases in the base sequence of SEQ ID NO: 240, or (c) a base sequence having 80% or more sequence identity with the base sequence of SEQ ID NO: 240, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 212, (b) a base sequence obtained by adding, deleting, or substituting one or several bases in the base sequence of SEQ ID NO: 212, or (c) a base sequence having 80% or more sequence identity with the base sequence of SEQ ID NO: 212, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 241, (b) a base sequence obtained by adding, deleting, or substituting one or several bases in the base sequence of SEQ ID NO: 241, or (c) a base sequence having 80% or more sequence identity with the base sequence of SEQ ID NO: 241, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 213, (b) a base sequence obtained by adding, deleting, or substituting one or several bases in the base sequence of SEQ ID NO: 213, or (c) a base sequence having 80% or more sequence identity with the base sequence of SEQ ID NO: 213, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 213, (b) a base sequence obtained by adding, deleting, or substituting one or several bases in the base sequence of SEQ ID NO: 213, or (c) a base sequence having 80% or more sequence identity with the base sequence of SEQ ID NO: 213, or consists of any of the base sequences, and the second antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences,
the first antisense oligomer unit comprises (a) a base sequence of SEQ ID NO: 213, (b) a base sequence obtained by adding, deleting, or substituting one or several bases in the base sequence of SEQ ID NO: 213, or (c) a base sequence having 80% or more sequence identity with the base sequence of SEQ ID NO: 213, or consists of any of the base sequences, and the second antisense oligomer unit comprises (a) a base sequence of SEQ ID NO: 196, (b) a base sequence obtained by adding, deleting, or substituting one or several bases in the base sequence of SEQ ID NO: 196, or (c) a base sequence having 80% or more sequence identity with the base sequence of SEQ ID NO: 196, or consists of any of the base sequences,
the first antisense oligomer unit comprises (a) a base sequence of SEQ ID NO: 242, (b) a base sequence obtained by adding, deleting, or substituting one or several bases in the base sequence of SEQ ID NO: 242, or (c) a base sequence having 80% or more sequence identity with the base sequence of SEQ ID NO: 242, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 243, (b) a base sequence obtained by adding, deleting, or substituting one or several bases in the base sequence of SEQ ID NO: 243, or (c) a base sequence having 80% or more sequence identity with the base sequence of SEQ ID NO: 243, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 243, (b) a base sequence obtained by adding, deleting, or substituting one or several bases in the base sequence of SEQ ID NO: 243, or (c) a base sequence having 80% or more sequence identity with the base sequence of SEQ ID NO: 243, or consists of any of the base sequences, and the second antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences,
the first antisense oligomer unit comprises (a) a base sequence of SEQ ID NO: 216, (b) a base sequence obtained by adding, deleting, or substituting one or several bases in the base sequence of SEQ ID NO: 216, or (c) a base sequence having 80% or more sequence identity with the base sequence of SEQ ID NO: 216, or consists of any of the base sequences, and the second antisense oligomer unit comprises (a) a base sequence of SEQ ID NO: 199, (b) a base sequence obtained by adding, deleting, or substituting one or several bases in the base sequence of SEQ ID NO: 199, or (c) a base sequence having 80% or more sequence identity with the base sequence of SEQ ID NO: 199, or consists of any of the base sequences,
the first antisense oligomer unit comprises (a) a base sequence of SEQ ID NO: 244, (b) a base sequence obtained by adding, deleting, or substituting one or several bases in the base sequence of SEQ ID NO: 244, or (c) a base sequence having 80% or more sequence identity with the base sequence of SEQ ID NO: 244, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 245, (b) a base sequence obtained by adding, deleting, or substituting one or several bases in the base sequence of SEQ ID NO: 245, or (c) a base sequence having 80% or more sequence identity with the base sequence of SEQ ID NO: 245, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 246, (b) a base sequence obtained by adding, deleting, or substituting one or several bases in the base sequence of SEQ ID NO: 246, or (c) a base sequence having 80% or more sequence identity with the base sequence of SEQ ID NO: 246, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 247, (b) a base sequence obtained by adding, deleting, or substituting one or several bases in the base sequence of SEQ ID NO: 247, or (c) a base sequence having 80% or more sequence identity with the base sequence of SEQ ID NO: 247, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 248, (b) a base sequence obtained by adding, deleting, or substituting one or several bases in the base sequence of SEQ ID NO: 248, or (c) a base sequence having 80% or more sequence identity with the base sequence of SEQ ID NO: 248, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 249, (b) a base sequence obtained by adding, deleting, or substituting one or several bases in the base sequence of SEQ ID NO: 249, or (c) a base sequence having 80% or more sequence identity with the base sequence of SEQ ID NO: 249, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 250, (b) a base sequence obtained by adding, deleting, or substituting one or several bases in the base sequence of SEQ ID NO: 250, or (c) a base sequence having 80% or more sequence identity with the base sequence of SEQ ID NO: 250, or consists of any of the base sequences, and the second antisense oligomer unit comprises (a) a base sequence of SEQ ID NO: 182, (b) a base sequence obtained by adding, deleting, or substituting one or several bases in the base sequence of SEQ ID NO: 182, or (c) a base sequence having 80% or more sequence identity with the base sequence of SEQ ID NO: 182, or consists of any of the base sequences,
the first antisense oligomer unit comprises (a) a base sequence of SEQ ID NO: 250, (b) a base sequence obtained by adding, deleting, or substituting one or several bases in the base sequence of SEQ ID NO: 250, or (c) a base sequence having 80% or more sequence identity with the base sequence of SEQ ID NO: 250, or consists of any of the base sequences, and the second antisense oligomer unit comprises (a) a base sequence of SEQ ID NO: 183, (b) a base sequence obtained by adding, deleting, or substituting one or several bases in the base sequence of SEQ ID NO: 183, or (c) a base sequence having 80% or more sequence identity with the base sequence of SEQ ID NO: 183, or consists of any of the base sequences,
the first antisense oligomer unit comprises (a) a base sequence of SEQ ID NO: 250, (b) a base sequence obtained by adding, deleting, or substituting one or several bases in the base sequence of SEQ ID NO: 250, or (c) a base sequence having 80% or more sequence identity with the base sequence of SEQ ID NO: 250, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 251, (b) a base sequence obtained by adding, deleting, or substituting one or several bases in the base sequence of SEQ ID NO: 251, or (c) a base sequence having 80% or more sequence identity with the base sequence of SEQ ID NO: 251, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 252, (b) a base sequence obtained by adding, deleting, or substituting one or several bases in the base sequence of SEQ ID NO: 252, or (c) a base sequence having 80% or more sequence identity with the base sequence of SEQ ID NO: 252, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 208, (b) a base sequence obtained by adding, deleting, or substituting one or several bases in the base sequence of SEQ ID NO: 208, or (c) a base sequence having 80% or more sequence identity with the base sequence of SEQ ID NO: 208, or consists of any of the base sequences, and the second antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences,
the first antisense oligomer unit comprises (a) a base sequence of SEQ ID NO: 208, (b) a base sequence obtained by adding, deleting, or substituting one or several bases in the base sequence of SEQ ID NO: 208, or (c) a base sequence having 80% or more sequence identity with the base sequence of SEQ ID NO: 208, or consists of any of the base sequences, and the second antisense oligomer unit comprises (a) a base sequence of SEQ ID NO: 196, (b) a base sequence obtained by adding, deleting, or substituting one or several bases in the base sequence of SEQ ID NO: 196, or (c) a base sequence having 80% or more sequence identity with the base sequence of SEQ ID NO: 196, or consists of any of the base sequences,
the first antisense oligomer unit comprises (a) a base sequence of SEQ ID NO: 253, (b) a base sequence obtained by adding, deleting, or substituting one or several bases in the base sequence of SEQ ID NO: 253, or (c) a base sequence having 80% or more sequence identity with the base sequence of SEQ ID NO: 253, or consists of any of the base sequences, and the second antisense oligomer unit comprises (a) a base sequence of SEQ ID NO: 178, (b) a base sequence obtained by adding, deleting, or substituting one or several bases in the base sequence of SEQ ID NO: 178, or (c) a base sequence having 80% or more sequence identity with the base sequence of SEQ ID NO: 178, or consists of any of the base sequences,
the first antisense oligomer unit comprises (a) a base sequence of SEQ ID NO: 217, (b) a base sequence obtained by adding, deleting, or substituting one or several bases in the base sequence of SEQ ID NO: 217, or (c) a base sequence having 80% or more sequence identity with the base sequence of SEQ ID NO: 217, or consists of any of the base sequences, and the second antisense oligomer unit comprises (a) a base sequence of SEQ ID NO: 199, (b) a base sequence obtained by adding, deleting, or substituting one or several bases in the base sequence of SEQ ID NO: 199, or (c) a base sequence having 80% or more sequence identity with the base sequence of SEQ ID NO: 199, or consists of any of the base sequences,
the first antisense oligomer unit comprises (a) a base sequence of SEQ ID NO: 209, (b) a base sequence obtained by adding, deleting, or substituting one or several bases in the base sequence of SEQ ID NO: 209, or (c) a base sequence having 80% or more sequence identity with the base sequence of SEQ ID NO: 209, or consists of any of the base sequences, and the second antisense oligomer unit comprises (a) a base sequence of SEQ ID NO: 175, (b) a base sequence obtained by adding, deleting, or substituting one or several bases in the base sequence of SEQ ID NO: 175, or (c) a base sequence having 80% or more sequence identity with the base sequence of SEQ ID NO: 175, or consists of any of the base sequences,
the first antisense oligomer unit comprises (a) a base sequence of SEQ ID NO: 218, (b) a base sequence obtained by adding, deleting, or substituting one or several bases in the base sequence of SEQ ID NO: 218, or (c) a base sequence having 80% or more sequence identity with the base sequence of SEQ ID NO: 218, or consists of any of the base sequences, and the second antisense oligomer unit comprises (a) a base sequence of SEQ ID NO: 199, (b) a base sequence obtained by adding, deleting, or substituting one or several bases in the base sequence of SEQ ID NO: 199, or (c) a base sequence having 80% or more sequence identity with the base sequence of SEQ ID NO: 199, or consists of any of the base sequences,
the first antisense oligomer unit comprises (a) a base sequence of SEQ ID NO: 210, (b) a base sequence obtained by adding, deleting, or substituting one or several bases in the base sequence of SEQ ID NO: 210, or (c) a base sequence having 80% or more sequence identity with the base sequence of SEQ ID NO: 210, or consists of any of the base sequences, and the second antisense oligomer unit comprises (a) a base sequence of SEQ ID NO: 195, (b) a base sequence obtained by adding, deleting, or substituting one or several bases in the base sequence of SEQ ID NO: 195, or (c) a base sequence having 80% or more sequence identity with the base sequence of SEQ ID NO: 195, or consists of any of the base sequences,
the first antisense oligomer unit comprises (a) a base sequence of SEQ ID NO: 210, (b) a base sequence obtained by adding, deleting, or substituting one or several bases in the base sequence of SEQ ID NO: 210, or (c) a base sequence having 80% or more sequence identity with the base sequence of SEQ ID NO: 210, or consists of any of the base sequences, and the second antisense oligomer unit comprises (a) a base sequence of SEQ ID NO: 196, (b) a base sequence obtained by adding, deleting, or substituting one or several bases in the base sequence of SEQ ID NO: 196, or (c) a base sequence having 80% or more sequence identity with the base sequence of SEQ ID NO: 196, or consists of any of the base sequences,
the first antisense oligomer unit comprises (a) a base sequence of SEQ ID NO: 210, (b) a base sequence obtained by adding, deleting, or substituting one or several bases in the base sequence of SEQ ID NO: 210, or (c) a base sequence having 80% or more sequence identity with the base sequence of SEQ ID NO: 210, or consists of any of the base sequences, and the second antisense oligomer unit comprises (a) a base sequence of SEQ ID NO: 192, (b) a base sequence obtained by adding, deleting, or substituting one or several bases in the base sequence of SEQ ID NO: 192, or (c) a base sequence having 80% or more sequence identity with the base sequence of SEQ ID NO: 192, or consists of any of the base sequences,
the first antisense oligomer unit comprises (a) a base sequence of SEQ ID NO: 254, (b) a base sequence obtained by adding, deleting, or substituting one or several bases in the base sequence of SEQ ID NO: 254, or (c) a base sequence having 80% or more sequence identity with the base sequence of SEQ ID NO: 254, or consists of any of the base sequences, and the second antisense oligomer unit comprises (a) a base sequence of SEQ ID NO: 255, (b) a base sequence obtained by adding, deleting, or substituting one or several bases in the base sequence of SEQ ID NO: 255, or (c) a base sequence having 80% or more sequence identity with the base sequence of SEQ ID NO: 255, or consists of any of the base sequences,
the first antisense oligomer unit comprises (a) a base sequence of SEQ ID NO: 256, (b) a base sequence obtained by adding, deleting, or substituting one or several bases in the base sequence of SEQ ID NO: 256, or (c) a base sequence having 80% or more sequence identity with the base sequence of SEQ ID NO: 256, or consists of any of the base sequences, and the second antisense oligomer unit comprises (a) a base sequence of SEQ ID NO: 239, (b) a base sequence obtained by adding, deleting, or substituting one or several bases in the base sequence of SEQ ID NO: 239, or (c) a base sequence having 80% or more sequence identity with the base sequence of SEQ ID NO: 239, or consists of any of the base sequences,
the first antisense oligomer unit comprises (a) a base sequence of SEQ ID NO: 240, (b) a base sequence obtained by adding, deleting, or substituting one or several bases in the base sequence of SEQ ID NO: 240, or (c) a base sequence having 80% or more sequence identity with the base sequence of SEQ ID NO: 240, or consists of any of the base sequences, and the second antisense oligomer unit comprises (a) a base sequence of SEQ ID NO: 213, (b) a base sequence obtained by adding, deleting, or substituting one or several bases in the base sequence of SEQ ID NO: 213, or (c) a base sequence having 80% or more sequence identity with the base sequence of SEQ ID NO: 213, or consists of any of the base sequences,
the first antisense oligomer unit comprises (a) a base sequence of SEQ ID NO: 212, (b) a base sequence obtained by adding, deleting, or substituting one or several bases in the base sequence of SEQ ID NO: 212, or (c) a base sequence having 80% or more sequence identity with the base sequence of SEQ ID NO: 212, or consists of any of the base sequences, and the second antisense oligomer unit comprises (a) a base sequence of SEQ ID NO: 213, (b) a base sequence obtained by adding, deleting, or substituting one or several bases in the base sequence of SEQ ID NO: 213, or (c) a base sequence having 80% or more sequence identity with the base sequence of SEQ ID NO: 213, or consists of any of the base sequences,
the first antisense oligomer unit comprises (a) a base sequence of SEQ ID NO: 213, (b) a base sequence obtained by adding, deleting, or substituting one or several bases in the base sequence of SEQ ID NO: 213, or (c) a base sequence having 80% or more sequence identity with the base sequence of SEQ ID NO: 213, or consists of any of the base sequences, and the second antisense oligomer unit comprises (a) a base sequence of SEQ ID NO: 208, (b) a base sequence obtained by adding, deleting, or substituting one or several bases in the base sequence of SEQ ID NO: 208, or (c) a base sequence having 80% or more sequence identity with the base sequence of SEQ ID NO: 208, or consists of any of the base sequences,
the first antisense oligomer unit comprises (a) a base sequence of SEQ ID NO: 213, (b) a base sequence obtained by adding, deleting, or substituting one or several bases in the base sequence of SEQ ID NO: 213, or (c) a base sequence having 80% or more sequence identity with the base sequence of SEQ ID NO: 213, or consists of any of the base sequences, and the second antisense oligomer unit comprises (a) a base sequence of SEQ ID NO: 209, (b) a base sequence obtained by adding, deleting, or substituting one or several bases in the base sequence of SEQ ID NO: 209, or (c) a base sequence having 80% or more sequence identity with the base sequence of SEQ ID NO: 209, or consists of any of the base sequences, or
the first antisense oligomer unit comprises (a) a base sequence of SEQ ID NO: 213, (b) a base sequence obtained by adding, deleting, or substituting one or several bases in the base sequence of SEQ ID NO: 213, or (c) a base sequence having 80% or more sequence identity with the base sequence of SEQ ID NO: 213, or consists of any of the base sequences, and the second antisense oligomer unit comprises (a) a base sequence of SEQ ID NO: 210, (b) a base sequence obtained by adding, deleting, or substituting one or several bases in the base sequence of SEQ ID NO: 210, or (c) a base sequence having 80% or more sequence identity with the base sequence of SEQ ID NO: 210, or consists of any of the base sequences.

The first antisense oligomer unit and the second antisense oligomer unit may comprise or consist of a base sequence having 85% or more, 86% or more, 87% or more, 88% or more, 89% or more, 90% or more, 910 or more, 92% or more, 93% or more, 94% or more, 95% or more, 96% or more, 97% or more, 98% or more, or 99% or more sequence identity with each base sequence in (c) above.

In one embodiment, the first antisense oligomer unit and the second antisense oligomer unit comprise any of the base sequences in (a) above, or consist of the base sequences.

In one embodiment, the second antisense oligomer of the present invention comprises: (a) a base sequence selected from the group consisting of SEQ ID NOs: 41 to 173;
(b) a base sequence obtained by adding, deleting, or substituting one or several bases in the base sequence selected from the group consisting of SEQ ID NOs: 41 to 173; or
(c) a base sequence having 80% or more, 85% or more, 86% or more, 87% or more, 88% or more, 89% or more, 90% or more, 910 or more, 92% or more, 93% or more, 94% or more, 95% or more, 96% or more, 97% or more, 98% or more, or 99% or more sequence identity with the base sequence selected from the group consisting of SEQ ID NOs: 41 to 173,
or consists of any of the sequences. In one embodiment, the second antisense oligomer of the present invention comprises any of the base sequences in (a) above, or consists of the base sequences.

In one embodiment, the second antisense oligomer of the present invention comprises: (a) a base sequence selected from the group consisting of SEQ ID NO: 41, SEQ ID NO: 44, SEQ ID NO: 45, SEQ ID NO: 48, SEQ ID NO: 51, SEQ ID NO: 52, SEQ ID NO: 53, SEQ ID NO: 55, SEQ ID NO: 57, SEQ ID NO: 58, SEQ ID NO: 80, SEQ ID NO: 83, SEQ ID NO: 89, SEQ ID NO: 123, SEQ ID NO: 125, SEQ ID NO: 126, SEQ ID NO: 135, SEQ ID NO: 140, and SEQ ID NO: 155;
(b) a base sequence obtained by adding, deleting, or substituting one or several bases in the base sequence selected from the group consisting of SEQ ID NO: 41, SEQ ID NO: 44, SEQ ID NO: 45, SEQ ID NO: 48, SEQ ID NO: 51, SEQ ID NO: 52, SEQ ID NO: 53, SEQ ID NO: 55, SEQ ID NO: 57, SEQ ID NO: 58, SEQ ID NO: 80, SEQ ID NO: 83, SEQ ID NO: 89, SEQ ID NO: 123, SEQ ID NO: 125, SEQ ID NO: 126, SEQ ID NO: 135, SEQ ID NO: 140, and SEQ ID NO: 155; or
(c) a base sequence having 80% or more, 85% or more, 86% or more, 87% or more, 88% or more, 89% or more, 90% or more, 910 or more, 92% or more, 93% or more, 94% or more, 95% or more, 96% or more, 97% or more, 98% or more, or 99% or more sequence identity with the base sequence selected from the group consisting of SEQ ID NO: 41, SEQ ID NO: 44, SEQ ID NO: 45, SEQ ID NO: 48, SEQ ID NO: 51, SEQ ID NO: 52, SEQ ID NO: 53, SEQ ID NO: 55, SEQ ID NO: 57, SEQ ID NO: 58, SEQ ID NO: 80, SEQ ID NO: 83, SEQ ID NO: 89, SEQ ID NO: 123, SEQ ID NO: 125, SEQ ID NO: 126, SEQ ID NO: 135, SEQ ID NO: 140, and SEQ ID NO: 155, or consists of any of the base sequences. In one embodiment, the second antisense oligomer of the present invention comprises any of the base sequences in (a) above, or consist of the base sequences.

### Pharmaceutical Composition

In one embodiment, the present invention relates to a pharmaceutical composition comprising one or two or more of the antisense oligomers of the present invention, or the pharmaceutically acceptable salt thereof, or the hydrate of the antisense oligomer or the salt. When the antisense oligomer of the present invention is to be administered to a subject, the pharmaceutical composition of the present invention may comprise a carrier to promote delivery of the antisense oligomer. Such a carrier is not particularly limited as far as it is pharmaceutically acceptable, and examples thereof can include cationic carriers such as cationic liposomes, and cationic polymers, and carriers using viral envelope. Examples of the cationic liposomes can include liposomes composed of 2-O-(2-diethylaminoethyl)carbamoyl-1,3-O-dioleoylglycerol and phospholipids as the essential constituents (hereinafter referred to as "liposome A"), Oligofectamine (registered trademark) (manufactured by Invitrogen Corp.), Lipofectin (registered trademark) (manufactured by Invitrogen Corp.), Lipofectamine (registered trademark) (manufactured by Invitrogen Corp.), Lipofectamine 2000 (registered trademark) (manufactured by Invitrogen Corp.), DMRIE-C (registered trademark) (manufactured by Invitrogen Corp.), GeneSilencer (registered trademark) (manufactured by Gene Therapy Systems), TransMessenger (registered trademark) (manufactured by QIAGEN, Inc.), TransIT TKO (registered trademark) (manufactured by Mirus Bio LLC), and Nucleofector II (Lonza). Examples of the cationic polymers can include JetSI (registered trademark) (manufactured by Qbiogene, Inc.), and Jet-PEI (registered trademark) (polyethylenimine, manufactured by Qbiogene, Inc.). Examples of the carriers using viral envelope can include GenomeOne (registered trademark) (HVJ-E liposome, manufactured by ISHIHARA SANGYO KAISHA, LTD.). Alternatively, the medical devices described in Japanese Patent No. 2924179, and the cationic carriers described in Japanese Domestic Re-Publication of PCT Application Nos. 2006/129594 and 2008/096690 may be used as well.

In one embodiment, the antisense oligomer of the present invention may be in the form of a complex (conjugate) with a lipid or the like in the pharmaceutical composition for promoting delivery of the antisense oligomer. For example, as described in Bijsterbosch, M.K. et al., (2000) Nucleic Acid Res., 28, 2717-2725, the antisense oligomer may be in the form of a conjugate with cholesterol.

The pharmaceutical composition of the present invention may comprise pharmaceutically acceptable additives in addition to the antisense oligomer, or the pharmaceutically acceptable salt thereof, or the hydrate of the antisense oligomer or the salt and optionally the carrier described above. Examples of such additives can include emulsification aids (e.g., fatty acids having 6 to 22 carbon atoms and their pharmaceutically acceptable salts, albumin and dextran), stabilizers (e.g., cholesterol, phosphatidic acid, mannitol, and sorbitol), isotonizing agents (e.g., sodium chloride, glucose, maltose, lactose, sucrose, and trehalose), and pH controlling agents (e.g., hydrochloric acid, sulfuric acid, phosphoric acid, acetic acid, sodium hydroxide, potassium hydroxide, and triethanolamine). One or two or more of these additives can be used. The content of the additive in the composition of the present invention is appropriately 90 wt% or less, preferably 70 wt% or less, and more preferably 50 wt% or less.

The preparation method of the pharmaceutical composition of the present invention is not limited, and the preparation may be conducted by, for example, adding the antisense oligomer of the present invention to a dispersion of the carrier, and appropriately stirring the resultant. Also, the additive may be added in an appropriate step either before or after the addition of the antisense oligomer of the present invention. An aqueous solvent which may be used in adding the antisense oligomer of the present invention is not particularly limited as long as it is pharmaceutically acceptable, and examples thereof can include injectable water, injectable distilled water, an electrolyte fluid such as physiological saline, a buffer solution, and a sugar solution such as a glucose solution, or a maltose solution. Those skilled in the art can appropriately choose conditions for pH and temperature to be employed in this case.

The pharmaceutical composition of the present invention may be prepared into, for example, a liquid form or its lyophilized preparation. The lyophilized preparation can be prepared by lyophilizing the composition of the present invention in a liquid form in a conventional manner. The lyophilization can be performed, for example, by appropriately sterilizing the composition of the present invention in a liquid form, dispensing an aliquot into a vial container, performing preliminary freezing for 2 hours at conditions in a range of about -40°C to -20°C, performing a primary drying in a range of about 0°C to 10°C under reduced pressure, and then performing a secondary drying in a range of about 15°C to 25°C under reduced pressure. In general, the lyophilized preparation of the composition of the present invention can be obtained by replacing the content of the vial with nitrogen gas and capping the resultant.

The lyophilized preparation of the pharmaceutical composition of the present invention can be used in general upon reconstitution by adding an optional suitable solution (reconstitution liquid). Examples of such a reconstitution liquid can include injectable water, physiological saline and other general infusion fluids. A volume of the reconstitution liquid may vary depending on the intended use and the like, is not particularly limited, and is suitably 0.5-fold to 2-fold greater than the volume prior to the lyophilization or no more than 500 mL.

A dose of the composition according to the present invention to be administered can be adjusted by taking the following factors into account: the type of the antisense oligomer according to the present invention, or the pharmaceutically acceptable salt thereof, or the hydrate of the antisense oligomer or the salt contained; the dosage form of the composition; patients' conditions including age, body weight, and the like; administration route; and the characteristics and symptoms of the disease. A single dose calculated as the amount of the antisense oligomer of the present invention to be administered can be 0.1 mg to 2000 mg or 1 mg to 200 mg per kg body weight, preferably 2 mg to 100 mg per kg body weight, more preferably 5 mg to 40 mg per kg body weight, and further preferably 10 mg to 20 mg per kg body weight. The number and frequency of administration are not limited, and for example, the administration can be performed only once, or another administration or more may be performed a few days later (e.g., the next day to within a week) for a total of several administrations (e.g., a total of two administrations). The frequency of administration may be once per 1 to 3 days, once per 4 to 6 days, once per week, or once per 2 to 3 weeks, when administering several times. This numerical range may vary occasionally depending on the type of the target disease, the administration form and the target molecule. Therefore, a dose or frequency of administration equal to or lower than these ranges may be sufficient in some occasion and conversely, a dose or frequency of administration equal to or higher than these ranges may be required occasionally.

The administration form for the composition according to the present invention is not particularly limited as long as it is pharmaceutically acceptable form for administration, and can be chosen depending upon method of treatment. Examples thereof include intratracheal administration, pulmonary administration, nasal administration, intravenous administration, intraarterial administration, intramuscular administration, subcutaneous administration, oral administration, tissue administration, and transdermal administration. Also, dosage forms which are available for the composition of the present invention are not particularly limited, and examples thereof can include inhalations, various injections, oral agents, drips, ointments, and lotions.

The administration form for the pharmaceutical composition according to the present invention is preferably intratracheal administration, and the dosage form which are available for the composition of the present invention is preferably inhalations, and in particular, for example, inhalation liquids (e.g., administered with a nebulizer), powder inhalations (e.g., administered with a dry powder inhaler (DPI)), and aerosols, and preferably inhalation liquids.

Examples of the subject to be given the antisense oligomer or the pharmaceutical composition of the present invention include mammals, including primates such as a human, experimental animals such as a rat, a mouse, and a brown rat, and domestic animals such as a pig, a cow, a horse, and sheep, and the subject is preferably a human.

The pharmaceutical composition of the present invention can be used in combination with other drugs, for example, therapeutic drugs for SARS-related coronaviruses such as SARS-CoV-2. Examples of other drugs include anti-inflammatory drugs (e.g., dexamethasone, baricitinib, and tocilizumab), antiviral drugs (e.g., remdesivir, molnupiravir, nirmatrelvir/ritonavir, and ensitrelvir fumaric acid), and neutralizing antibody drugs (casirivimab/imdevimab, sotrovimab, and tixagevimab/cilgavimab). The pharmaceutical composition of the present invention and other drugs can be administered concurrently or separately with an interval (e.g., 1 to several hours, 1 to several days, or 1 to several weeks) when using in combination. In the case of concurrent administration, the pharmaceutical composition of the present invention and the other drugs can be administered as one medication comprising thereof, or as separate medications.

### Method for Treating and/or Preventing Viral Infectious Disease

In one embodiment, the present invention relates to a method for treating and/or preventing a viral infectious disease selected from the group consisting of SARS-CoV-2, SARS-CoV-1, and MERS-CoV, comprising a step of administering, to a subject, the antisense oligomer, or the pharmaceutically acceptable salt thereof, or the hydrate of the antisense oligomer or the salt, or the pharmaceutical composition of the present invention. The pharmaceutical composition, and the dose, the administration route and the like thereof in the present embodiment are the same as those described herein.

As used herein, treatment of a viral infectious disease encompasses one or more of relief, improvement, and remission of the disease caused by the above-mentioned virus or symptoms thereof (e.g., one or more of dyspnea, fever, dry cough, headache, chill, and muscular pain). As used herein, prevention of viral infectious diseases encompasses reduction of risks of developing diseases caused by the above-mentioned virus or symptoms thereof.

In one embodiment, the present invention relates to the antisense oligomer, or the pharmaceutically acceptable salt thereof, or the hydrate of the antisense oligomer or the salt of the present invention for use in treating and/or preventing a viral infectious disease selected from the group consisting of SARS-CoV-2. SARS-CoV-1, and MERS-CoV. In one embodiment, the present invention relates to use of the antisense oligomer, or the pharmaceutically acceptable salt thereof, or the hydrate of the antisense oligomer or the salt of the present invention in manufacturing medicament for use in treating and/or preventing a viral infectious disease selected from the group consisting of SARS-CoV-2, SARS-CoV-1, and MERS-CoV.

### Examples

### <Example 1: Identification of Target Sequence>

By comparing the sequences of the genome RNA of SARS-CoV-2 (reference sequence; NC_045512.2) (SEQ ID NO: 1) and the genome RNA of SARS-CoV-1 (reference sequence; NC_004718.3), regions that are conserved between the two species were identified. As the regions conserved between the two species, base sequences at positions of 43 to 89, 98 to 110, 122 to 132, 190 to 202, 242 to 279, 290 to 312, 408 to 420, 455 to 477, 13363 to 13386, 13388 to 13401, 13418 to 13432, 13458 to 13502, 13504 to 13516, 13518 to 13532, 13537 to 13547, 13582 to 13598, 29554 to 29566, 29568 to 29580, 29599 to 29613, 29615 to 29634, 29638 to 29648, 29652 to 29665, 29667 to 29682, 29689 to 29699, 29712 to 29731, 29744 to 29757, 29759 to 29768, and 29787 to 29867 of SEQ ID NO: 1 were found. Test substances PMO Nos. P1 to P32, P36 to P41, PG10+P16F, PG10+P16L, PG10+PG13, CRN-3 to CRN-51, CRN-65 to CRN-72, CRN-75 to CRN-84, CRN-86 to CRN-97, CRN-100 to CRN-111, CRN-119 to CRN-142, CRN-150 to CRN-155, CRN-158 to CRN-163, and CRN-174 to CRN-177 (SEQ ID NOs: 2 to 173) were prepared by targeting sequences comprising the conserved regions or being comprised in the conserved regions.

The test substances PMO without CRN in the PMO No. were purchased from Gene Tools, LLC, and those with CRN in the PMO No. were chemically synthesized according to the method described in International Publication No. WO 2015/137409. Each PMO is phosphorodiamidate morpholino oligomer, and the PMO without CRN in the PMO No. has a group represented by the following (1) at the 5' end. The PMO with CRN in the PMO No. has a group represented by the following (2) at the 5' end.

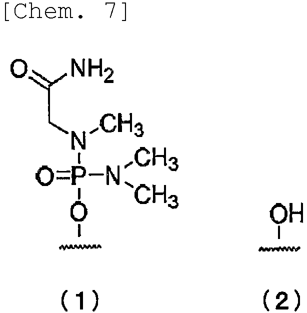

Target sequences and sequences of the test substances PMO (SEQ ID NOs: 2 to 173), and the like are shown in the following Table 2.

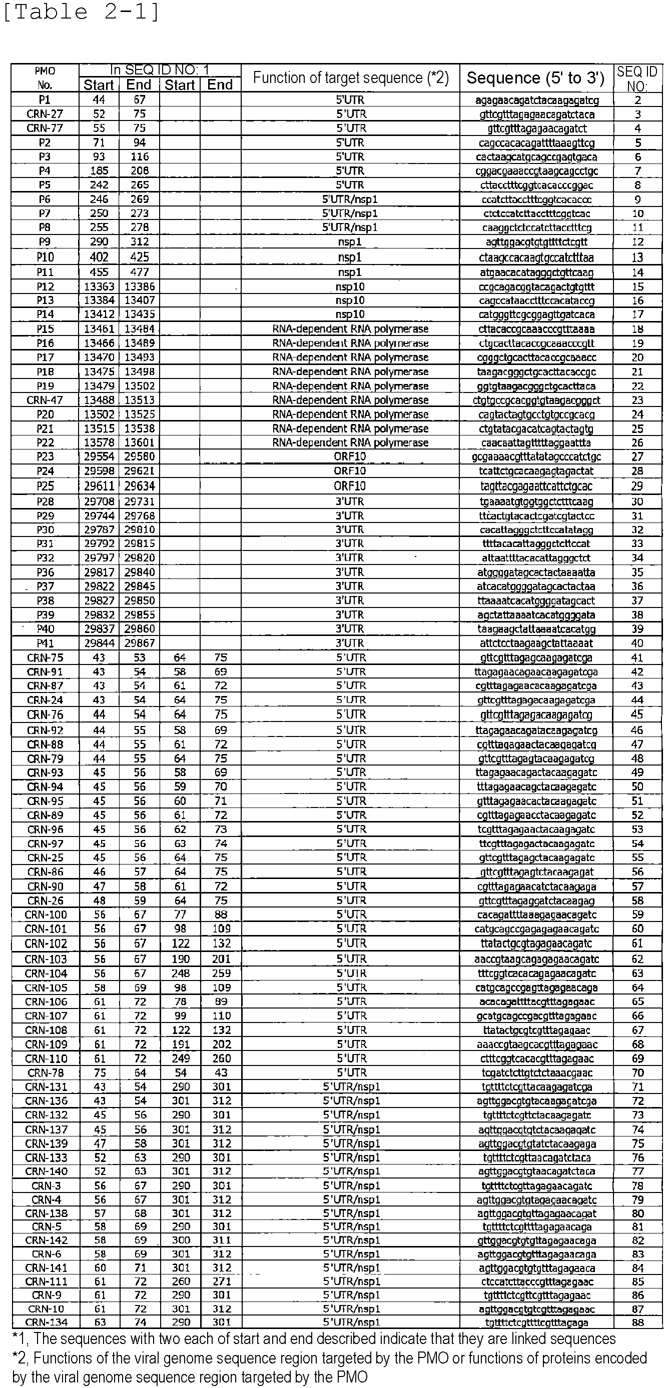

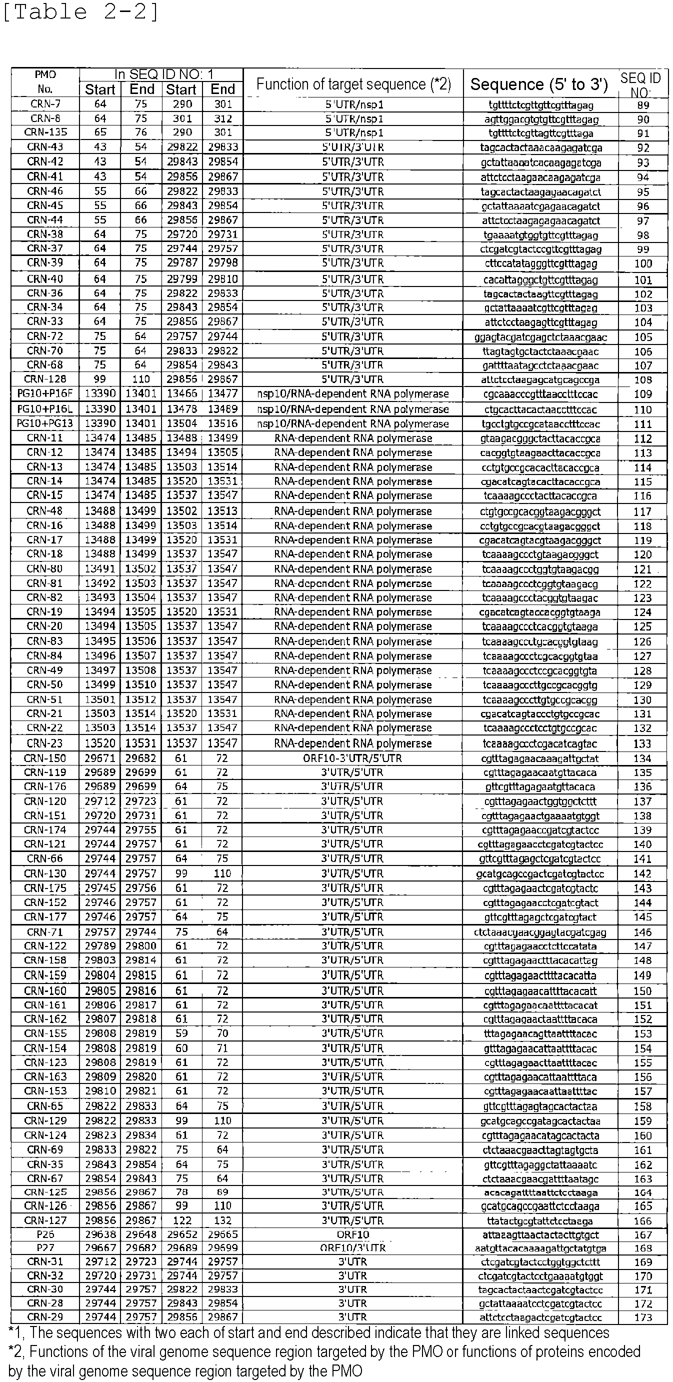

Note that those with a plurality of target sequences described in Table 2 means that the PMO is a linked PMO in which the target sequence of the PMO spans a plurality of regions.

As for the linked PMO, the sequence of each unit is shown in the following Table 3.

**[Table 3-1]**

| PMO No. | Position of target sequence in SEQ ID NO: 1 | | | | Sequence (5' to 3') | SEQ ID NO: | SEQ ID NO: when linking |
|---|---|---|---|---|---|---|---|
| | Start | End | Start | End | | | |
| CRN-75 | 43 | 53 | | | caagagatcga | 174 | 41 |
| | | | 64 | 75 | gttcgtttagag | 175 | 41 |
| CRN-91 | 43 | 54 | | | acaagagatcga | 176 | 42 |
| | | | 58 | 69 | ttagagaacaga | 177 | 42 |
| CRN-87 | 43 | 54 | | | acaagagatcga | 176 | 43 |
| | | | 61 | 72 | cgtttagagaac | 178 | 43 |
| CRN-24 | 43 | 54 | | | acaagagatcga | 176 | 44 |
| | | | 64 | 75 | gttcgtttagag | 175 | 44 |
| CRN-76 | 44 | 54 | | | acaagagatcg | 179 | 45 |
| | | | 64 | 75 | gttcgtttagag | 175 | 45 |
| CRN-92 | 44 | 55 | | | tacaagagatcg | 180 | 46 |
| | | | 58 | 69 | ttagagaacaga | 177 | 46 |
| CRN-88 | 44 | 55 | | | tacaagagatcg | 180 | 47 |
| | | | 61 | 72 | cgtttagagaac | 178 | 47 |
| CRN-79 | 44 | 55 | | | tacaagagatcg | 180 | 48 |
| | | | 64 | 75 | gttcgtttagag | 175 | 48 |
| CRN-93 | 45 | 56 | | | ctacaagagatc | 181 | 49 |
| | | | 58 | 69 | ttagagaacaga | 177 | 49 |
| CRN-94 | 45 | 56 | | | ctacaagagatc | 181 | 50 |
| | | | 59 | 70 | tttagagaacag | 182 | 50 |
| CRN-95 | 45 | 56 | | | ctacaagagatc | 181 | 51 |
| | | | 60 | 71 | gtttagagaaca | 183 | 51 |
| CRN-89 | 45 | 56 | | | ctacaagagatc | 181 | 52 |
| | | | 61 | 72 | cgtttagagaac | 178 | 52 |
| CRN-96 | 45 | 56 | | | ctacaagagatc | 181 | 53 |
| | | | 62 | 73 | tcgtttagagaa | 184 | 53 |
| CRN-97 | 45 | 56 | | | ctacaagagatc | 181 | 54 |
| | | | 63 | 74 | ttcgtttagaga | 185 | 54 |
| CRN-25 | 45 | 56 | | | ctacaagagatc | 181 | 55 |
| | | | 64 | 75 | gttcgtttagag | 175 | 55 |
| CRN-86 | 46 | 57 | | | tctacaagagat | 186 | 56 |
| | | | 64 | 75 | gttcgtttagag | 175 | 56 |
| CRN-90 | 47 | 58 | | | atctacaagaga | 187 | 57 |
| | | | 61 | 72 | cgtttagagaac | 178 | 57 |
| CRN-26 | 48 | 59 | | | gatctacaagag | 188 | 58 |
| | | | 64 | 75 | gttcgtttagag | 175 | 58 |
| CRN-100 | 56 | 67 | | | agagaacagatc | 189 | 59 |
| | | | 77 | 88 | cacagattttaa | 190 | 59 |
| CRN-101 | 56 | 67 | | | agagaacagatc | 189 | 60 |
| | | | 98 | 109 | catgcagccgag | 191 | 60 |
| CRN-102 | 56 | 67 | | | agagaacagatc | 189 | 61 |
| | | | 122 | 132 | ttatactgcgt | 192 | 61 |
| CRN-103 | 56 | 67 | | | agagaacagatc | 189 | 62 |
| | | | 190 | 201 | aaccgtaagcag | 193 | 62 |
| CRN-104 | 56 | 67 | | | agagaacagatc | 189 | 63 |
| | | | 248 | 259 | tttcggtcacac | 194 | 63 |
| CRN-105 | 58 | 69 | | | ttagagaacaga | 177 | 64 |
| | | | 98 | 109 | catgcagccgag | 191 | 64 |
| CRN-106 | 61 | 72 | | | cgtttagagaac | 178 | 65 |
| | | | 78 | 89 | acacagatttta | 195 | 65 |
| CRN-107 | 61 | 72 | | | cgtttagagaac | 178 | 66 |
| | | | 99 | 110 | gcatgcagccga | 196 | 66 |
| CRN-108 | 61 | 72 | | | cgtttagagaac | 178 | 67 |
| | | | 122 | 132 | ttatactgcgt | 192 | 67 |
| CRN-109 | 61 | 72 | | | cgtttagagaac | 178 | 68 |
| | | | 191 | 202 | aaaccgtaagca | 197 | 68 |
| CRN-110 | 61 | 72 | | | cgtttagagaac | 178 | 69 |
| | | | 249 | 260 | ctttcggtcaca | 198 | 69 |
| CRN-78 | 75 | 64 | | | ctctaaacgaac | 199 | 70 |
| | | | 54 | 43 | tcgatctcttgt | 200 | 70 |
| CRN-131 | 43 | 54 | | | acaagagatcga | 176 | 71 |
| | | | 290 | 301 | tgttttctcgtt | 201 | 71 |
| CRN-136 | 43 | 54 | | | acaagagatcga | 176 | 72 |
| | | | 301 | 312 | agttggacgtgt | 202 | 72 |
| CRN-132 | 45 | 56 | | | ctacaagagatc | 181 | 73 |
| | | | 290 | 301 | tgttttctcgtt | 201 | 73 |
| CRN-137 | 45 | 56 | | | ctacaagagatc | 181 | 74 |
| | | | 301 | 312 | agttggacgtgt | 202 | 74 |
| CRN-139 | 47 | 58 | | | atctacaagaga | 187 | 75 |
| | | | 301 | 312 | agttggacgtgt | 202 | 75 |
| CRN-133 | 52 | 63 | | | aacagatctaca | 203 | 76 |
| | | | 290 | 301 | tgttttctcgtt | 201 | 76 |
| CRN-140 | 52 | 63 | | | aacagatctaca | 203 | 77 |
| | | | 301 | 312 | agttggacgtgt | 202 | 77 |
| CRN-3 | 56 | 67 | | | agagaacagatc | 189 | 78 |
| | | | 290 | 301 | tgttttctcgtt | 201 | 78 |
| CRN-4 | 56 | 67 | | | agagaacagatc | 189 | 79 |
| | | | 301 | 312 | agttggacgtgt | 202 | 79 |
| CRN-138 | 57 | 68 | | | tagagaacagat | 204 | 80 |
| | | | 301 | 312 | agttggacgtgt | 202 | 80 |
| CRN-5 | 58 | 69 | | | ttagagaacaga | 177 | 81 |
| | | | 290 | 301 | tgttttctcgtt | 201 | 81 |
| CRN-142 | 58 | 69 | | | ttagagaacaga | 177 | 82 |
| | | | 300 | 311 | gttggacgtgtg | 205 | 82 |
| CRN-6 | 58 | 69 | | | ttagagaacaga | 177 | 83 |
| | | | 301 | 312 | agttggacgtgt | 202 | 83 |
| CRN-141 | 60 | 71 | | | gtttagagaaca | 183 | 84 |
| | | | 301 | 312 | agttggacgtgt | 202 | 84 |
| CRN-111 | 61 | 72 | | | cgtttagagaac | 178 | 85 |
| | | | 260 | 271 | ctccatcttacc | 206 | 85 |
| CRN-9 | 61 | 72 | | | cgtttagagaac | 178 | 86 |
| | | | 290 | 301 | tgttttctcgtt | 201 | 86 |
| CRN-10 | 61 | 72 | | | cgtttagagaac | 178 | 87 |
| | | | 301 | 312 | agttggacgtgt | 202 | 87 |
| CRN-134 | 63 | 74 | | | ttcgtttagaga | 185 | 88 |
| | | | 290 | 301 | tgttttctcgtt | 201 | 88 |
| CRN-7 | 64 | 75 | | | gttcgtttagag | 175 | 89 |
| | | | 290 | 301 | tgttttctcgtt | 201 | 89 |
| CRN-8 | 64 | 75 | | | gttcgtttagag | 175 | 90 |
| | | | 301 | 312 | agttggacgtgt | 202 | 90 91 |
| CRN-135 | 65 | 76 | | | agttcgtttaga | 207 | |
| | | | 290 | 301 | tgttttctcgtt | 201 | 91 |
| CRN-43 | 43 | 54 | | | acaagagatcga | 176 | 92 |
| | | | 29822 | 29833 | tagcactactaa | 208 | 92 |
| CRN-42 | 43 | 54 | | | acaagagatcga | 176 | 93 |
| | | | 29843 | 29854 | gctattaaaatc | 209 | 93 |
| CRN-41 | 43 | 54 | | | acaagagatcga | 176 | 94 |
| | | | 29856 | 29867 | attctcctaaga | 210 | 94 |
| CRN-46 | 55 | 66 | | | gagaacagatct | 211 | 95 |
| | | | 29822 | 29833 | tagcactactaa | 208 | 95 |
| CRN-45 | 55 | 66 | | | gagaacagatct | 211 | 96 |
| | | | 29843 | 29854 | gctattaaaatc | 209 | 96 |
| CRN-44 | 55 | 66 | | | gagaacagatct | 211 | 97 |
| | | | 29856 | 29867 | attctcctaaga | 210 | 97 |
| CRN-38 | 64 | 75 | | | gttcgtttagag | 175 | 98 |
| | | | 29720 | 29731 | tgaaaatgtggt | 212 | 98 |
| CRN-37 | 64 | 75 | | | gttcgtttagag | 175 | 99 |
| | | | 29744 | 29757 | ctcgatcgtactcc | 213 | 99 |
| CRN-39 | 64 | 75 | | | gttcgtttagag | 175 | 100 |
| | | | 29787 | 29798 | cttccatatagg | 214 | 100 |
| CRN-40 | 64 | 75 | | | gttcgtttagag | 175 | 101 |
| | | | 29799 | 29810 | cacattagggct | 215 | 101 |
| CRN-36 | 64 | 75 | | | gttcgtttagag | 175 | 102 |
| | | | 29822 | 29833 | tagcactactaa | 208 | 102 |
| CRN-34 | 64 | 75 | | | gttcgtttagag | 175 | 103 |
| | | | 29843 | 29854 | gctattaaaatc | 209 | 103 |
| CRN-33 | 64 | 75 | | | gttcgtttagag | 175 | 104 |
| | | | 29856 | 29867 | attctcctaaga | 210 | 104 |
| CRN-72 | 75 | 64 | | | ctctaaacgaac | 199 | 105 |
| | | | 29757 | 29744 | ggagtacgatcgag | 216 | 105 |
| CRN-70 | 75 | 64 | | | ctctaaacgaac | 199 | 106 |
| | | | 29833 | 29822 | ttagtagtgcta | 217 | 106 |
| CRN-68 | 75 | 64 | | | ctctaaacgaac | 199 | 107 |
| | | | 29854 | 29843 | gattttaatagc | 218 | 107 |
| CRN-128 | 99 | 110 | | | gcatgcagccga | 196 | 108 |
| | | | 29856 | 29867 | attctcctaaga | 210 | 108 |
| PG10+P16F | 13390 | 13401 | | | taacctttccac | 219 | 109 |
| | | | 13466 | 13477 | cgcaaacccgtt | 220 | 109 |
| PG10+P16L | 13390 | 13401 | | | taacctttccac | 219 | 110 |
| | | | 13478 | 13489 | ctgcacttacac | 221 | 110 |
| PG10+PG13 | 13390 | 13401 | | | taacctttccac | 219 | 111 |
| | | | 13504 | 13516 | tgcctgtgccgca | 222 | 111 |
| CRN-11 | 13474 | 13485 | | | acttacaccgca | 223 | 112 |
| | | | 13488 | 13499 | gtaagacgggct | 224 | 112 |
| CRN-12 | 13474 | 13485 | | | acttacaccgca | 223 | 113 |
| | | | 13494 | 13505 | cacggtgtaaga | 225 | 113 |
| CRN-13 | 13474 | 13485 | | | acttacaccgca | 223 | 114 |
| | | | 13503 | 13514 | cctgtgccgcac | 226 | 114 |
| CRN-14 | 13474 | 13485 | | | acttacaccgca | 223 | 115 |
| | | | 13520 | 13531 | cgacatcagtac | 227 | 115 |
| CRN-15 | 13474 | 13485 | | | acttacaccgca | 223 | 116 |
| | | | 13537 | 13547 | tcaaaagccct | 228 | 116 |
| CRN-48 | 13488 | 13499 | | | gtaagacgggct | 224 | 117 |
| | | | 13502 | 13513 | ctgtgccgcacg | 229 | 117 |
| CRN-16 | 13488 | 13499 | | | gtaagacgggct | 224 | 118 |
| | | | 13503 | 13514 | cctgtgccgcac | 226 | 118 |
| CRN-17 | 13488 | 13499 | | | gtaagacgggct | 224 | 119 |
| | | | 13520 | 13531 | cgacatcagtac | 227 | 119 |
| CRN-18 | 13488 | 13499 | | | gtaagacgggct | 224 | 120 |
| | | | 13537 | 13547 | tcaaaagccct | 228 | 120 |
| CRN-80 | 13491 | 13502 | | | ggtgtaagacgg | 230 | 121 |
| | | | 13537 | 13547 | tcaaaagccct | 228 | 121 |
| CRN-81 | 13492 | 13503 | | | cggtgtaagacg | 231 | 122 |
| | | | 13537 | 13547 | tcaaaagccct | 228 | 122 |
| CRN-82 | 13493 | 13504 | | | acggtgtaagac | 232 | 123 |
| | | | 13537 | 13547 | tcaaaagccct | 228 | 123 |
| CRN-19 | 13494 | 13505 | | | cacggtgtaaga | 225 | 124 |
| | | | 13520 | 13531 | cgacatcagtac | 227 | 124 |
| CRN-20 | 13494 | 13505 | | | cacggtgtaaga | 225 | 125 |
| | | | 13537 | 13547 | tcaaaagccct | 228 | 125 |
| CRN-83 | 13495 | 13506 | | | gcacggtgtaag | 233 | 126 |
| | | | 13537 | 13547 | tcaaaagccct | 228 | 126 |
| CRN-84 | 13496 | 13507 | | | cgcacggtgtaa | 234 | 127 |
| | | | 13537 | 13547 | tcaaaagccct | 228 | 127 |
| CRN-49 | 13497 | 13508 | | | ccgcacggtgta | 235 | 128 |
| | | | 13537 | 13547 | tcaaaagccct | 228 | 128 |
| CRN-50 | 13499 | 13510 | | | tgccgcacggtg | 236 | 129 |
| | | | 13537 | 13547 | tcaaaagccct | 228 | 129 |
| CRN-51 | 13501 | 13512 | | | tgtgccgcacgg | 237 | 130 |
| | | | 13537 | 13547 | tcaaaagccct | 228 | 130 |
| CRN-21 | 13503 | 13514 | | | cctgtgccgcac | 226 | 131 |
| | | | 13520 | 13531 | cgacatcagtac | 227 | 131 |
| CRN-22 | 13503 | 13514 | | | cctgtgccgcac | 226 | 132 |
| | | | 13537 | 13547 | tcaaaagccct | 228 | 132 |
| CRN-23 | 13520 | 13531 | | | cgacatcagtac | 227 | 133 |
| | | | 13537 | 13547 | tcaaaagccct | 228 | 133 |
| CRN-150 | 29671 | 29682 | | | aaagattgctat | 238 | 134 |
| | | | 61 | 72 | cgtttagagaac | 178 | 134 |
| CRN-119 | 29689 | 29699 | | | aatgttacaca | 239 | 135 |
| | | | 61 | 72 | cgtttagagaac | 178 | 135 |
| CRN-176 | 29689 | 29699 | | | aatgttacaca | 239 | 136 |
| | | | 64 | 75 | gttcgtttagag | 175 | 136 |
| CRN-120 | 29712 | 29723 | | | tggtggctcttt | 240 | 137 |
| | | | 61 | 72 | cgtttagagaac | 178 | 137 |
| CRN-151 | 29720 | 29731 | | | tgaaaatgtggt | 212 | 138 |
| | | | 61 | 72 | cgtttagagaac | 178 | 138 |
| CRN-174 | 29744 | 29755 | | | cgatcgtactcc | 241 | 139 |
| | | | 61 | 72 | cgtttagagaac | 178 | 139 |
| CRN-121 | 29744 | 29757 | | | ctcgatcgtactcc | 213 | 140 |
| | | | 61 | 72 | cgtttagagaac | 178 | 140 |
| CRN-66 | 29744 | 29757 | | | ctcgatcgtactcc | 213 | 141 |
| | | | 64 | 75 | gttcgtttagag | 175 | 141 |
| CRN-130 | 29744 | 29757 | | | ctcgatcgtactcc | 213 | 142 |
| | | | 99 | 110 | gcatgcagccga | 196 | 142 |
| CRN-175 | 29745 | 29756 | | | tcgatcgtactc | 242 | 143 |
| | | | 61 | 72 | cgtttagagaac | 178 | 143 |
| CRN-152 | 29746 | 29757 | | | ctcgatcgtact | 243 | 144 |
| | | | 61 | 72 | cgtttagagaac | 178 | 144 |
| CRN-177 | 29746 | 29757 | | | ctcgatcgtact | 243 | 145 |
| | | | 64 | 75 | gttcgtttagag | 175 | 145 |
| CRN-71 | 29757 | 29744 | | | ggagtacgatcgag | 216 | 146 |
| | | | 75 | 64 | ctctaaacgaac | 199 | 146 |
| CRN-122 | 29789 | 29800 | | | ctcttccatata | 244 | 147 |
| | | | 61 | 72 | cgtttagagaac | 178 | 147 |
| CRN-158 | 29803 | 29814 | | | tttacacattag | 245 | 148 |
| | | | 61 | 72 | cgtttagagaac | 178 | 148 |
| CRN-159 | 29804 | 29815 | | | ttttacacatta | 246 | 149 |
| | | | 61 | 72 | cgtttagagaac | 178 | 149 |
| CRN-160 | 29805 | 29816 | | | attttacacatt | 247 | 150 |
| | | | 61 | 72 | cgtttagagaac | 178 | 150 |
| CRN-161 | 29806 | 29817 | | | aattttacacat | 248 | 151 |
| | | | 61 | 72 | cgtttagagaac | 178 | 151 |
| CRN-162 | 29807 | 29818 | | | taattttacaca | 249 | 152 |
| | | | 61 | 72 | cgtttagagaac | 178 | 152 |
| CRN-155 | 29808 | 29819 | | | ttaattttacac | 250 | 153 |
| | | | 59 | 70 | tttagagaacag | 182 | 153 |
| CRN-154 | 29808 | 29819 | | | ttaattttacac | 250 | 154 |
| | | | 60 | 71 | gtttagagaaca | 183 | 154 |
| CRN-123 | 29808 | 29819 | | | ttaattttacac | 250 | 155 |
| | | | 61 | 72 | cgtttagagaac | 178 | 155 |
| CRN-163 | 29809 | 29820 | | | attaattttaca | 251 | 156 |
| | | | 61 | 72 | cgtttagagaac | 178 | 156 |
| CRN-153 | 29810 | 29821 | | | aattaattttac | 252 | 157 |
| | | | 61 | 72 | cgtttagagaac | 178 | 157 |
| CRN-65 | 29822 | 29833 | | | tagcactactaa | 208 | 158 |
| | | | 64 | 75 | gttcgtttagag | 175 | 158 |
| CRN-129 | 29822 | 29833 | | | tagcactactaa | 208 | 159 |
| | | | 99 | 110 | gcatgcagccga | 196 | 159 |
| CRN-124 | 29823 | 29834 | | | atagcactacta | 253 | 160 |
| | | | 61 | 72 | cgtttagagaac | 178 | 160 |
| CRN-69 | 29833 | 29822 | | | ttagtagtgcta | 217 | 161 |
| | | | 75 | 64 | ctctaaacgaac | 199 | 161 |
| CRN-35 | 29843 | 29854 | | | gctattaaaatc | 209 | 162 |
| | | | 64 | 75 | gttcgtttagag | 175 | 162 |
| CRN-67 | 29854 | 29843 | | | gattttaatagc | 218 | 163 |
| | | | 75 | 64 | ctctaaacgaac | 199 | 163 |
| CRN-125 | 29856 | 29867 | | | attctcctaaga | 210 | 164 |
| | | | 78 | 89 | acacagatttta | 195 | 164 |
| CRN-126 | 29856 | 29867 | | | attctcctaaga | 210 | 165 |
| | | | 99 | 110 | gcatgcagccga | 196 | 165 |
| CRN-127 | 29856 | 29867 | | | attctcctaaga | 210 | 166 |
| | | | 122 | 132 | ttatactgcgt | 192 | 166 |
| P26 | 29638 | 29648 | | | ctacttgtgct | 254 | 167 |
| | | | 29652 | 29665 | attaaagttaacta | 255 | 167 |
| P27 | 29667 | 29682 | | | aaagattgctatgtga | 256 | 168 |
| | | | 29689 | 29699 | aatgttacaca | 239 | 168 |
| CRN-31 | 29712 | 29723 | | | tggtggctcttt | 240 | 169 |
| | | | 29744 | 29757 | ctcgatcgtactcc | 213 | 169 |
| CRN-32 | 29720 | 29731 | | | tgaaaatgtggt | 212 | 170 |
| | | | 29744 | 29757 | ctcgatcgtactcc | 213 | 170 |
| CRN-30 | 29744 | 29757 | | | ctcgatcgtactcc | 213 | 171 |
| | | | 29822 | 29833 | tagcactactaa | 208 | 171 |
| CRN-28 | 29744 | 29757 | | | ctcgatcgtactcc | 213 | 172 |
| | | | 29843 | 29854 | gctattaaaatc | 209 | 172 |
| CRN-29 | 29744 | 29757 | | | ctcgatcgtactcc | 213 | 173 |
| | | | 29856 | 29867 | attctcctaaga | 210 | 173 |

### <Example 2: Antiviral Activity Measurement of PMO Using SARS-CoV-2 Virus>

A mixture of the test substance PMO and Endo-Porter (Gene Tools, LLC, 0.9 µL/well) at a final concentration of 3 to 50 µM was added to a 96-well plate, and seeded at 3.0 × 10⁴ cells/well with human ACE2 stable expressing cell (293T-ACE2) prepared by infecting 293T cells with a human ACE2-expressing lentiviral vector to treat with the test substance PMO. The next day, SARS-CoV-2 virus solution was added thereto at MOI = 0.01 to infect the cells with the virus. The SARS-CoV-2 virus was prepared by obtaining the WK521 strain from The National Institute of Infectious Diseases as a base, and inserting the Nano-luciferase gene into a coding region for the accessory protein, ORF8, using an approach similar to that described in the literature (Terada et al., 2019. J Virol 93:e01208-19. https://doi.org/10.1128/JVI.01208-19.) to form recombinant SARS-CoV-2 virus (rSARS-CoV-2-ORF8-Nluc) for use. The virus in the medium was removed by medium exchange 1 hour after the addition of the virus solution. The culture supernatant was collected 24 hours after the viral infection, and cells were obtained by preparing a cell lysate using Passive Lysis Buffer (Promega Corporation). The luciferase activity in the cell lysate was measured using Luciferase Assay System (Promega Corporation) according to the attached protocol to evaluate the amount of SARS-CoV-2 viruses in the cells. Also, 5 µL of the collected culture supernatant was separately added to the 96-well plate in which 293T-ACE2 cells were seeded at 3.0 × 10⁴ cells/well to infect the cells with the virus in the culture supernatant. 24 hours after the infection with the culture supernatant, the amount of SARS-CoV-2 viruses in the cells was evaluated in a manner similar to that of the initial viral infection to evaluate the amount of infectious virus particles in the culture supernatant. The rate of suppressing viruses was calculated as follows. (Luciferase activity value of samples derived from test substance PMO-treated cells) / (average value of luciferase activity value of samples derived from untreated cells (n=3))

Formula I was calculated for each sample derived from treated cells (n=3), and the suppression rate was calculated by the following formula. Suppression rate = (1 - average value of calculated values in Formula I (n=3)) × 100

The results are shown in Table 4.

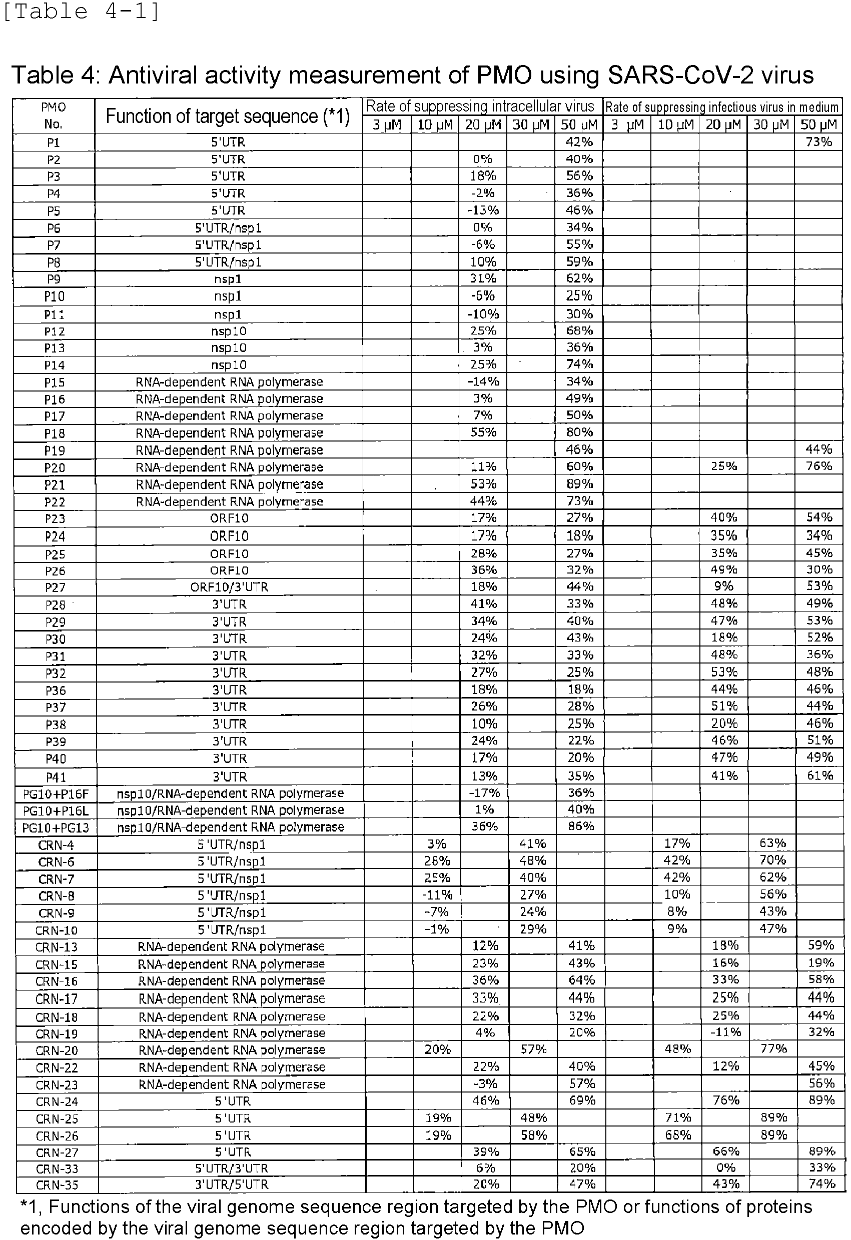

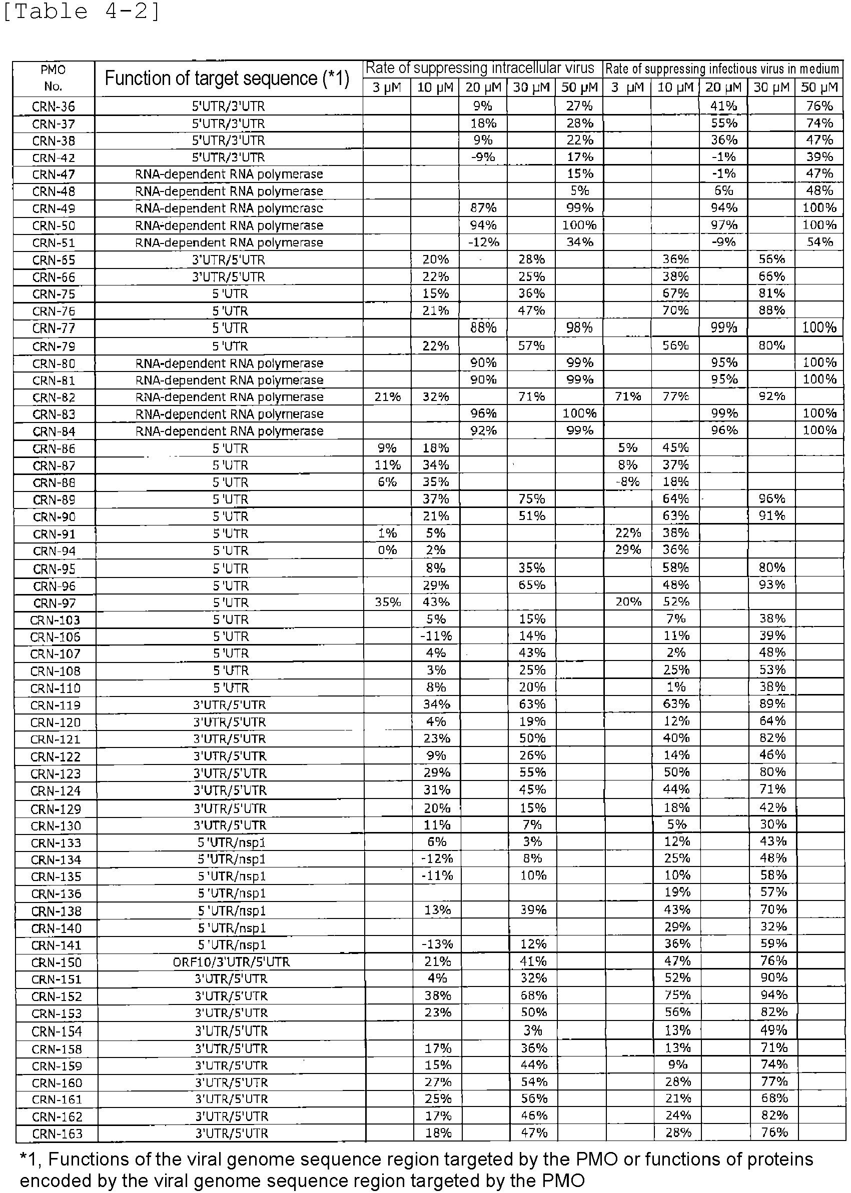

## Claims

1. An antisense oligomer, or a pharmaceutically acceptable salt thereof, or a hydrate of the antisense oligomer or the salt having a length of 15 to 30 bases, comprising a base sequence complementary to a base sequence in a target region,
wherein the target region comprises a sequence of at least 10 consecutive bases in at least one region selected from the group consisting of a 5' UTR region, a nsp1 region, a nsp10 region, an RNA-dependent RNA polymerase region, an ORF10 region, and a 3' UTR region in the genome RNA of SARS-CoV-2, or a complementary sequence thereof,
wherein the antisense oligomer, or the pharmaceutically acceptable salt thereof, or the hydrate of the antisense oligomer or the salt has an antiviral effect on a virus selected from the group consisting of SARS-CoV-2, SARS-CoV-1, and MERS-CoV.

2. The antisense oligomer, or the pharmaceutically acceptable salt thereof, or the hydrate of the antisense oligomer or the salt according to claim 1, wherein the target region is a base sequence selected from the group consisting of positions 43 to 116, 122 to 132, 185 to 208, 242 to 279, 290 to 312, 402 to 425, 455 to 477, 13363 to 13407, 13412 to 13435, 13458 to 13547, 13578 to 13601, 29554 to 29580, 29598 to 29634, 29638 to 29648, 29652 to 29665, 29667 to 29682, 29689 to 29699, 29708 to 29731, 29744 to 29768, and 29787 to 29867 of a base sequence of SEQ ID NO: 1, or a complementary sequence thereof.

3. The antisense oligomer, or the pharmaceutically acceptable salt thereof, or the hydrate of the antisense oligomer or the salt according to claim 2, wherein the target region is a base sequence selected from the group consisting of positions 44 to 67, 52 to 75, 55 to 75, 71 to 94, 93 to 116, 185 to 208, 242 to 265, 246 to 269, 250 to 273, 255 to 278, 290 to 312, 402 to 425, 455 to 477, 13363 to 13386, 13384 to 13407, 13412 to 13435, 13461 to 13484, 13466 to 13489, 13470 to 13493, 13475 to 13498, 13479 to 13502, 13488 to 13513, 13502 to 13525, 13515 to 13538, 13578 to 13601, 29554 to 29580, 29598 to 29621, 29611 to 29634, 29708 to 29731, 29744 to 29768, 29787 to 29810, 29792 to 29815, 29797 to 29820, 29817 to 29840, 29822 to 29845, 29827 to 29850, 29832 to 29855, 29837 to 29860, and 29844 to 29867 of the base sequence of SEQ ID NO: 1, or the complementary sequence thereof.

4. The antisense oligomer, or the pharmaceutically acceptable salt thereof, or the hydrate of the antisense oligomer or the salt according to any one of claims 1 to 3, wherein the target region comprises a sequence of at least 15 consecutive bases in at least one region selected from the group consisting of the 5' UTR region, the nsp1 region, the nsp10 region, the RNA-dependent RNA polymerase region, the ORF10 region, and the 3' UTR region in the genome RNA of SARS-CoV-2, or the complementary sequence thereof.

5. The antisense oligomer, or the pharmaceutically acceptable salt thereof, or the hydrate of the antisense oligomer or the salt according to any one of claims 1 to 4, comprising:
(a) a base sequence selected from the group consisting of SEQ ID NOs: 2 to 40;
(b) a base sequence obtained by adding, deleting, or substituting one or several bases in the base sequence selected from the group consisting of SEQ ID NOs: 2 to 40; or
(c) a base sequence having 80% or more sequence identity with the base sequence selected from the group consisting of SEQ ID NOs: 2 to 40,
wherein the antisense oligomer, or the pharmaceutically acceptable salt thereof, or the hydrate of the antisense oligomer or the salt inhibits a function of the target region.

6. An antisense oligomer, or a pharmaceutically acceptable salt thereof, or a hydrate of the antisense oligomer or the salt, comprising:
a first antisense oligomer unit having a length of 8 to 20 bases, comprising a base sequence complementary to a base sequence in a first target region, wherein the first target region comprises a sequence of at least 10 consecutive bases in a first region selected from the group consisting of a 5' UTR region, a nsp1 region, a nsp10 region, an RNA-dependent RNA polymerase region, an ORF10 region, and a 3' UTR region in a genome RNA of SARS-CoV-2, or a complementary sequence thereof; and
a second antisense oligomer unit having a length of 8 to 20 bases, comprising a base sequence complementary to a base sequence in a second target region, wherein the second target region comprises a sequence of at least 10 consecutive bases in a second region selected from the group consisting of the 5' UTR region, the nsp1 region, the nsp10 region, the RNA-dependent RNA polymerase region, the ORF10 region, and the 3' UTR region in the genome RNA of SARS-CoV-2, or a complementary sequence thereof, wherein
(i) the difference between a position of a base sequence of SEQ ID NO: 1 at an end of the sequence of at least 10 consecutive bases in the first region, or a complementary sequence thereof, and a position of the base sequence of SEQ ID NO: 1 at an end of the sequence of at least 10 consecutive bases in the second region, or a complementary sequence thereof is 500 bases or less,
(ii) the first and second regions are the 5' UTR and the 3' UTR regions, respectively, or the 3' UTR and the 5' UTR regions, respectively, or
(iii) a surrounding sequence of the first region and a surrounding sequence of the second region are complementary to each other, and the surrounding sequences base-pair with each other when replicating, transcribing or translating a virus,
wherein the antisense oligomer, or the pharmaceutically acceptable salt thereof, or the hydrate of the antisense oligomer or the salt has an antiviral effect on a virus selected from the group consisting of SARS-CoV-2, SARS-CoV-1, and MERS-CoV.

7. The antisense oligomer, or the pharmaceutically acceptable salt thereof, or the hydrate of the antisense oligomer or the salt according to claim 6, wherein the sequence of at least 10 consecutive bases in the first region and the sequence of at least 10 consecutive bases in the second region are not consecutive or overlapping with each other.

8. The antisense oligomer, or the pharmaceutically acceptable salt thereof, or the hydrate of the antisense oligomer or the salt according to claim 7, wherein the first and second target regions are each base sequences selected from the group consisting of positions 43 to 89, 98 to 110, 122 to 132, 190 to 202, 242 to 279, 290 to 312, 408 to 420, 455 to 477, 13363 to 13386, 13388 to 13401, 13418 to 13432, 13458 to 13516, 13518 to 13532, 13537 to 13547, 13582 to 13598, 29554 to 29566, 29568 to 29580, 29599 to 29613, 29615 to 29634, 29638 to 29648, 29652 to 29665, 29667 to 29682, 29689 to 29699, 29712 to 29731, 29744 to 29757, 29759 to 29768, and 29787 to 29867 of a base sequence of SEQ ID NO: 1, or complementary sequences thereof.

9. The antisense oligomer, or the pharmaceutically acceptable salt thereof, or the hydrate of the antisense oligomer or the salt according to claim 8, comprising:
(a) a base sequence selected from the group consisting of SEQ ID NOs: 41 to 173;
(b) a base sequence obtained by adding, deleting, or substituting one or several bases in the base sequence selected from the group consisting of SEQ ID NOs: 41 to 173; or
(c) a base sequence having 80% or more sequence identity with the base sequence selected from the group consisting of SEQ ID NOs: 41 to 173,
wherein the antisense oligomer, or the pharmaceutically acceptable salt thereof, or the hydrate of the antisense oligomer or the salt inhibits a function of the first region and/or the second region.

10. The antisense oligomer, or the pharmaceutically acceptable salt thereof, or the hydrate of the antisense oligomer or the salt according to claim 9, comprising:
(a) a base sequence selected from the group consisting of SEQ ID NO: 41, SEQ ID NO: 44, SEQ ID NO: 45, SEQ ID NO: 48, SEQ ID NO: 51, SEQ ID NO: 52, SEQ ID NO: 53, SEQ ID NO: 55, SEQ ID NO: 57, SEQ ID NO: 58, SEQ ID NO: 80, SEQ ID NO: 83, SEQ ID NO: 89, SEQ ID NO: 123, SEQ ID NO: 125, SEQ ID NO: 126, SEQ ID NO: 135, SEQ ID NO: 140, and SEQ ID NO: 155;
(b) a base sequence obtained by adding, deleting, or substituting one or several bases in the base sequence selected from the group consisting of SEQ ID NO: 41, SEQ ID NO: 44, SEQ ID NO: 45, SEQ ID NO: 48, SEQ ID NO: 51, SEQ ID NO: 52, SEQ ID NO: 53, SEQ ID NO: 55, SEQ ID NO: 57, SEQ ID NO: 58, SEQ ID NO: 80, SEQ ID NO: 83, SEQ ID NO: 89, SEQ ID NO: 123, SEQ ID NO: 125, SEQ ID NO: 126, SEQ ID NO: 135, SEQ ID NO: 140, and SEQ ID NO: 155; or
(c) a base sequence having 80% or more sequence identity with the base sequence selected from the group consisting of SEQ ID NO: 41, SEQ ID NO: 44, SEQ ID NO: 45, SEQ ID NO: 48, SEQ ID NO: 51, SEQ ID NO: 52, SEQ ID NO: 53, SEQ ID NO: 55, SEQ ID NO: 57, SEQ ID NO: 58, SEQ ID NO: 80, SEQ ID NO: 83, SEQ ID NO: 89, SEQ ID NO: 123, SEQ ID NO: 125, SEQ ID NO: 126, SEQ ID NO: 135, SEQ ID NO: 140, and SEQ ID NO: 155.

11. The antisense oligomer, or the pharmaceutically acceptable salt thereof, or the hydrate of the antisense oligomer or the salt according to any one of claims 1 to 10, wherein the virus is SARS-CoV-2 or SARS-CoV-1.

12. The antisense oligomer, or the pharmaceutically acceptable salt thereof, or the hydrate of the antisense oligomer or the salt according to any one of claims 1 to 11, wherein the antisense oligomer is a morpholino oligomer.

13. The antisense oligomer, or the pharmaceutically acceptable salt thereof, or the hydrate of the antisense oligomer or the salt according to claim 12, wherein the antisense oligomer is a phosphorodiamidate morpholino oligomer.

14. The antisense oligomer, or the pharmaceutically acceptable salt thereof, or the hydrate of the antisense oligomer or the salt according to any one of claims 1 to 13, wherein the antisense oligomer has any group represented by the following chemical formulas (1) and (2) at the 5' end:

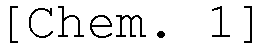

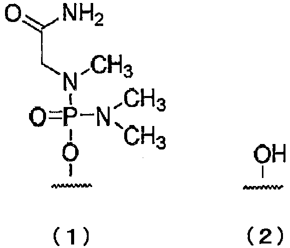

15. A pharmaceutical composition comprising the antisense oligomer, or the pharmaceutically acceptable salt thereof, or the hydrate of the antisense oligomer or the salt according to any one of claims 1 to 14.

16. The pharmaceutical composition according to claim 15, for treating and/or preventing a viral infectious disease selected from the group consisting of SARS-CoV-2, SARS-CoV-1, and MERS-CoV.

17. A method for treating and/or preventing a viral infectious disease selected from the group consisting of SARS-CoV-2, SARS-CoV-1, and MERS-CoV, comprising a step of administering, to a subject, an effective amount of the antisense oligomer, or the pharmaceutically acceptable salt thereof, or the hydrate of the antisense oligomer or the salt according to any one of claims 1 to 14, or the pharmaceutical composition according to claim 15 or 16.
